# EUROPEAN PATENT APPLICATION

(11) **EP 3 293 203 A1**
(43) Date of publication of application: **14.03.2018**
(21) Application number: 16306149.2
(22) Date of filing: 12.09.2016
(51) Int. Cl.: C07K 16/44

(54) **ACID-ALPHA GLUCOSIDASE VARIANTS AND USES THEREOF**

(71) Applicant: GENETHON, 91000 Evry (FR); Université Pierre et Marie Curie (Paris 6), 75005 Paris (FR)
(72) Inventor: MINGOZZI, Federico, 75011 Paris (FR); RONZITTI, Giuseppe, 75011 Paris (FR); COLELLA, Pasqualina, 75019 Paris (FR); PUZZO, Francesco, 75019 Paris (FR)
(74) Representative: Cabinet Becker et Associés

(57) **Abstract**

The present invention relates to variants of acid-alpha glucosidase and uses thereof.

## Description

The present invention relates to variants of acid-alpha glucosidase (GAA) and uses thereof. Said variants are linked to heterogenous signal peptides.

Pompe disease, also known as glycogen storage disease (GSD) type II and acid maltase deficiency, is an autosomal recessive metabolic myopathy caused by a deficiency of the lysosomal enzyme acid alpha-glucosidase (GAA). GAA is an exo-1,4 and 1,6-α-glucosidase that hydrolyzes glycogen to glucose in the lysosome. Deficiency of GAA leads to glycogen accumulation in lysosomes and causes progressive damage to respiratory, cardiac, and skeletal muscle. The disease ranges from a rapidly progressive infantile course that is usually fatal by 1-2 years of age to a more slowly progressive and heterogeneous course that causes significant morbidity and early mortality in children and adults. Hirschhorn RR, The Metabolic and Molecular Bases of Inherited Disease, 3: 3389-3420 (2001, McGraw-Hill); Van der Ploeg and Reuser, Lancet 372: 1342-1351 (2008).

Current human therapy for treating Pompe disease involves administration of recombinant human GAA, otherwise termed enzyme-replacement therapy (ERT). ERT has demonstrated efficacy for severe, infantile GSD II. However the benefit of enzyme therapy is limited by the need for frequent infusions and the development of inhibitor antibodies against recombinant hGAA (Amalfitano, A., et al. (2001) Genet. In Med. 3:132-138). Furthermore, ERT does not correct efficiently the entire body, probably because of a combination of poor biodistribution of the protein following peripheral vein delivery, lack of uptake from several tissues, and high immunogenicity.

As an alternative or adjunct to ERT, the feasibility of gene therapy approaches to treat GSD-II have been investigated (Amalfitano, A., et al. (1999) Proc. Natl. Acad. Sci. USA 96:8861-8866, Ding, E., et al. (2002) Mol. Ther. 5:436-446, Fraites, T. J., et al. (2002) Mol. Ther. 5:571-578, Tsujino, S., et al. (1998) Hum. Gene Ther. 9:1609-1616). However, muscle-directed gene transfer to correct the genetic defect has to face the limitation of the systemic nature of the disease and the fact that muscle expression of a transgene tends to be more immunogenic compared with other tissues.

Doerfler et al., 2016 describe the combined administration of two constructs encoding a human codon-optimized GAA, one under the control of a liver specific promoter and the other one under the control of a muscle-specific promoter. Liver-specific promoter driven expression of GAA is employed to promote immune tolerance to GAA in a Gaa^{-/-} mouse model, while muscle-specific promoter driven expression of GAA provides expression of the therapeutic protein in part of the tissues targeted for therapy. However, this strategy is not entirely satisfactory in that it requires the use of multiple constructs and it does not result in body wide expression of GAA.

Modified GAA proteins have been proposed in the past to improve lysosomal storage disease treatment. In particular, application WO2004064750 and Sun et al. 2006, disclose a chimeric GAA polypeptide comprising a signal peptide operably linked to GAA as a way to enhance targeting of the protein to the secretory pathway.

However, therapies available to the patient are not entirely satisfactory and improved GAA polypeptides and GAA production is still a need in the art. In particular, a need still exists of a long term efficacy of the treatment with GAA, of high level GAA production, of improved immunological tolerance to the produced GAA polypeptide, and of improved uptake of GAA by the cells and tissues in need thereof. In addition, in WO2004064750 and Sun et al., 2006, tissue distribution of the chimeric GAA polypeptide disclosed therein is not entirely satisfactory. Therefore, a need still exists for a GAA polypeptide that would be fully therapeutic, by allowing a correction of glycogen accumulation in most if not all tissues of interest.

### SUMMARY OF THE INVENTION

The present invention relates to GAA variants that are expressed and secreted at higher levels compared to the wild type GAA protein and that elicit improved correction of the pathological accumulation of glycogen body-wide and results in the induction of immunological tolerance to GAA.

According to one aspect, the invention provides a nucleic acid molecule encoding a functional chimeric GAA polypeptide, comprising a signal peptide moiety and a functional GAA moiety. In the encoded chimeric GAA polypeptide, the endogenous (or natural) signal peptide of a GAA polypeptide is replaced with the signal peptide of another protein. The nucleic acid molecule therefore encodes a chimeric GAA polypeptide comprising a signal peptide from another protein than a GAA, operably linked to a GAA polypeptide. The encoded chimeric polypeptide is a functional GAA protein wherein the amino acid sequence corresponding to the natural signal peptide of GAA (such as that corresponding to nucleotides 1 to 81 of SEQ ID NO: 1 which is a wild-type nucleic acid encoding human GAA) is replaced by the amino acid sequence of a different protein. In a preferred embodiment, the encoded signal peptide has an amino acid sequence selected in the group consisting of SEQ ID NO:2 to 4. In a particular embodiment, the GAA moiety is a N-terminally truncated form of a parent GAA polypeptide.

In a particular embodiment, the GAA moiety has 1 to 75 consecutive amino acids deleted at its N-terminal end as compared to a parent GAA polypeptide, wherein the parent polypeptide corresponds to a precursor form of a GAA polypeptide devoid of its signal peptide. In a particular embodiment, the truncated GAA polypeptide has 6, 7, 8, 9, 10, 40, 41, 42, 43 or 44 consecutive amino acids deleted at its N-terminal end as compared to a parent GAA polypeptide, in particular 8 or 42 consecutive amino acids deleted at its N-terminal end as compared to a parent GAA polypeptide. In a further particular embodiment, the GAA moiety has 8 consecutive amino acid deleted at its N-terminal end as compared to a parent GAA. An illustrative parent GAA polypeptide is represented by the human GAA polypeptide shown in SEQ ID NO:5 or SEQ ID NO:36.

In another particular embodiment, the nucleic acid molecule of the invention is a nucleotide sequence optimized to improve the expression of and/or improve immune tolerance to the chimeric GAA *in vivo.*

In a particular embodiment, the nucleic acid molecule of the invention encodes a chimeric GAA polypeptide comprising the moieties shown in the following table 1:

| Signal peptide moiety | GAA moiety |
|---|---|
| SEQ ID NO:2 | wild-type hGAA devoid of its natural signal |
| SEQ ID NO:3 | peptide; e.g. SEQ ID NO:5 or SEQ ID NO:36, in |
| SEQ ID NO:4 | particular SEQ ID NO:5 |
| SEQ ID NO:2 | truncated hGAA deleted for 8 consecutive N-terminal amino acids; e.g. SEQ ID NO:29 |
| SEQ ID NO:3 | |
| SEQ ID NO:4 | |
| SEQ ID NO:2 | Truncated hGAA deleted for 42 consecutive N-terminal amino acids; e.g. SEQ ID NO:30 |
| SEQ ID NO:3 | |
| SEQ ID NO:4 | |

For example, such nucleic acid molecules may be the result of the following combinations shown in table 2:

| Signal peptide moiety coding sequence | GAA moiety coding sequence |
|---|---|
| SEQ ID NO:26 | |
| SEQ ID NO:27 | SEQ ID NO:31 |
| SEQ ID NO:28 | |
| SEQ ID NO:26 | |
| SEQ ID NO:27 | SEQ ID NO:13 |
| SEQ ID NO:28 | |
| SEQ ID NO:26 | |
| SEQ ID NO:27 | SEQ ID NO:14 |
| SEQ ID NO:28 | |
| SEQ ID NO:26 | |
| SEQ ID NO:27 | SEQ ID NO:32 |
| SEQ ID NO:28 | |
| SEQ ID NO:26 | |
| SEQ ID NO:27 | SEQ ID NO:33 |
| SEQ ID NO:28 | |
| SEQ ID NO:26 | |
| SEQ ID NO:27 | SEQ ID NO:34 |
| SEQ ID NO:28 | |
| SEQ ID NO:26 | |
| SEQ ID NO:27 | SEQ ID NO:35 |
| SEQ ID NO:28 | |

In yet another aspect, the invention relates to a nucleic acid construct, comprising the nucleic acid molecule of the invention operably linked to one or more regulatory sequences such as a promoter, an intron, a polyadenylation signal and/or an enhancer (for example a cis-regulatory module, or CRM). In a particular embodiment, the promoter is a liver-specific promoter preferably selected in the group consisting of the alpha-1 antitrypsin promoter (hAAT), the transthyretin promoter, the albumin promoter and the thyroxine-binding globulin (TBG) promoter. In another particular embodiment, the promoter is a muscle-specific promoter, such as the Spc5-12, MCK and desmin promoters. In another embodiment, the promoter is an ubiquitous promoter such as the CMV, CAG and PGK promoters. The nucleic acid construct may further optionally comprises an intron, in particular an intron selected in the group consisting of a human beta globin b2 (or HBB2) intron, a FIX intron, a chicken beta-globin intron and a SV40 intron, wherein said intron is optionally a modified intron such as a modified HBB2 intron of SEQ ID NO:7, a modified FIX intron of SEQ ID NO:9, or a modified chicken beta-globin intron of SEQ ID NO: 11.

In another particular embodiment, the nucleic acid construct comprises, preferably in this order,: an enhancer; an intron; a promoter, in particular a liver-specific promoter; the nucleic acid sequence encoding the chimeric GAA polypeptide; and a polyadenylation signal, the construct comprising preferably, in this order: an ApoE control region; a HBB2 intron, in particular a modified HBB2 intron; a hAAT promoter; the nucleic acid sequence encoding the chimeric GAA polypeptide; and a bovine growth hormone polyadenylation signal. In specific embodiment, the nucleic acid construct comprises a nucleotide sequence selected in the group consisting of the combinations of sequences shown in table 2, more particularly the nucleotide sequence of SEQ ID NO:17 (corresponding to the fusion of SEQ ID NO:26 and SEQ ID NO:32), 18 (corresponding to the fusion of SEQ ID NO:27 and SEQ ID NO:32) or 19 (corresponding to the fusion of SEQ ID NO:28 and SEQ ID NO:32).

According to another aspect, the invention relates to a vector comprising the nucleic acid molecule or the nucleic acid construct according to the invention. In a particular embodiment, the vector is a viral vector, preferably a retroviral vector, such as a lentiviral vector, or an AAV vector.

According to another embodiment, the viral vector is a single-stranded or double-stranded self-complementary AAV vector, preferably an AAV vector with an AAV-derived capsid, such as an AAV1, AAV2, variant AAV2, AAV3, variant AAV3, AAV3B, variant AAV3B, AAV4, AAV5, AAV6, variant AAV6, AAV7, AAV8, AAV9, AAV10 such as AAVcy10 and AAVrh10, AAVrh74, AAVdj, AAV-Anc80, AAV-LK03, AAV2i8, and porcine AAV, such as AAVpo4 and AAVpo6 capsid or with a chimeric capsid.

According to a further particular embodiment, the AAV vector has an AAV8, AAV9, AAVrh74 or AAV2i8 capsid, in particular an AAV8, AAV9 or AAVrh74 capsid, more particularly an AAV8 capsid.

In another aspect, the invention relates to a cell transformed with the nucleic acid molecule, the nucleic acid construct or the vector of the invention. In a particular embodiment, the cell is a liver cell or a muscle cell.

According to another aspect, the invention relates to a chimeric GAA polypeptide, comprising a signal peptide moiety and a functional GAA moiety. The signal peptide moiety is selected in the group consisting of SEQ ID NO:2 to 4, preferably SEQ ID NO:2. Furthermore, the GAA moiety may be a truncated form of a parent GAA polypeptide, such as a GAA moiety having 1 to 75 consecutive amino acids truncated at its N-terminal end as compared to a parent GAA polypeptide, in particular 6, 7, 8, 9, 10, 20, 41, 42, 43 or 44 consecutive amino acids truncated at its N-terminal end as compared to a parent GAA polypeptide, such as 8 or 42 consecutive amino acids truncated at its N-terminal end as compared to a parent GAA polypeptide, wherein the GAA moiety is in particular a truncated form of the human GAA protein of SEQ ID NO:5 or SEQ ID NO:36, in particular of SEQ ID NO:5. In a particular embodiment, the GAA moiety has 8 consecutive amino acids truncated at its N-terminal end as compared to a parent GAA polypeptide (more particularly the parent GAA polypeptide of SEQ ID NO:5 or SEQ ID NO:36, in particular of SEQ ID NO:5). In a particular embodiment of the invention, the chimeric GAA polypeptide of the invention is selected in the group consisting of the combinations of amino acid sequences shown in table 1.

In another aspect, the invention relates to a pharmaceutical composition, comprising, in a pharmaceutically acceptable carrier, the nucleic acid sequence, the nucleic acid construct, the vector, the cell or the chimeric polypeptide disclosed herein.

Another aspect of the invention relates to the nucleic acid sequence, the nucleic acid construct, the vector, the cell, or the chimeric polypeptide of the invention, for use as a medicament.

In yet another aspect, the invention relates to the nucleic acid sequence, the nucleic acid construct, the vector, the cell, or the chimeric polypeptide of the invention, for use in a method for treating a glycogen storage disease. In a particular embodiment, the glycogen storage disease is GSDI, GSDII, GSDIII, GSDIV, GSDV, GSDVI, GSDVII, GSDVIII or lethal congenital glycogen storage disease of the heart. In a more particular embodiment, the glycogen storage disease is selected in the group consisting of GSDI, GSDII and GSDIII, more particularly in the group consisting of GSDII and GSDIII. In an even more particular embodiment, the glycogen storage disease is GSDII.

### LEGENDS TO THE FIGURES

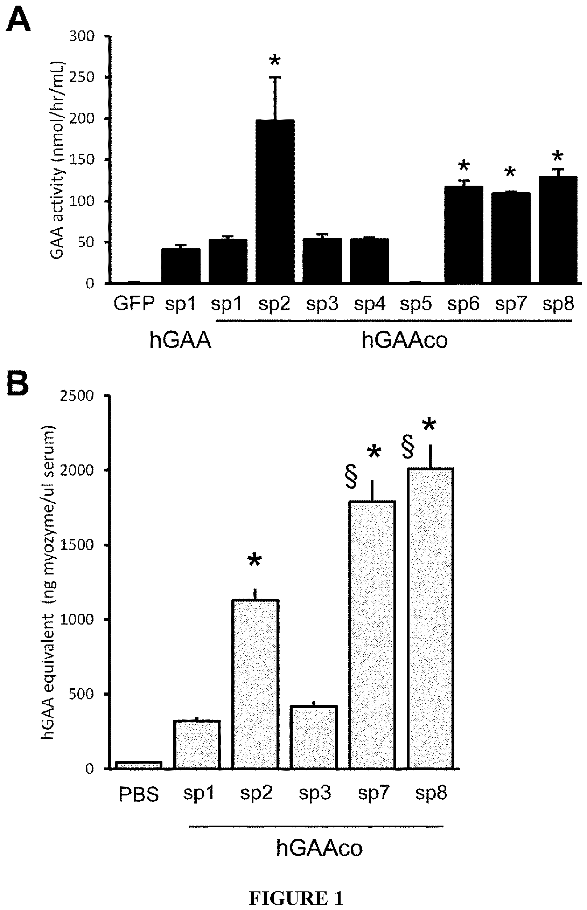

**Figure 1****.** Signal peptides enhance secretion of hGAA to a variable extent in vitro and in vivo. **Panel A.** Human hepatoma cells (Huh7) were transfected by Lipofectamine™ with a control plasmid (GFP), a plasmid expressing wild-type hGAA under the transcriptional control of a liver specific promoter (noted as sp1), or plasmids expressing sequence optimized hGAA (hGAAco) fused with signal peptides 1-8 (sp2 (sp1-8) of synthetic origin or derived from other highly-secreted proteins. 48 hours after transfection the activity of hGAA in the culture media was measured by a fluorogenic enzymatic assay and GAA activity evaluated against a standard curve of 4-methylumbelliferone. The histogram plot shows the average ± SE of the levels of secreted hGAA deriving from three different experiments. Statistical analysis has been performed by ANOVA (* = p<0.05 vs mock transfected cells). **Panel B.** The histogram plot shows the average ± SE of the activity of hGAA in serum of 3-month-old C57B16J mice (n=5 mice/group) 1 month after the injection of PBS (PBS) or 1E12 vg/kg of AAV8 vectors expressing sequence optimized hGAA (hGAAco) under the transcriptional control of human alpha-1-antytripsin promoter and fused with signal peptides 1 to 3 and 7-8 (sp1-3, 7-8). The activity of hGAA in serum has been quantified by a fluorogenic enzymatic assay and GAA activity evaluated against a standard curve of recombinant hGAA protein. Statistical analysis has been performed by ANOVA (* = p<0.05 vs PBS injected, § = p<0.05 vs sp2).

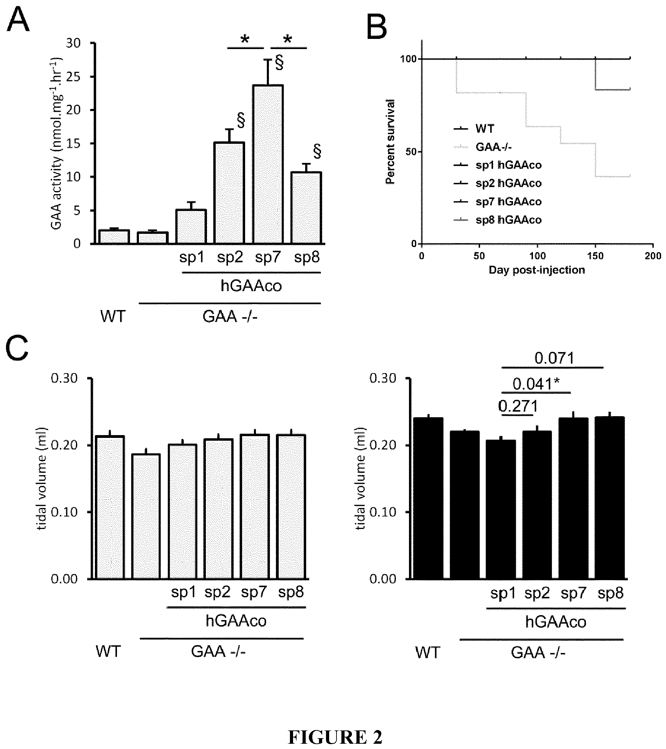

**Figure 2****.** sp7 signal peptide increases levels of circulating hGAA and rescue the respiratory impairment in a Pompe disease mouse model. 4 months-old wild type (WT) and GAA^{-/-} mice (n=6-9 mice/group) were intravenously injected with PBS or 2E12 vg/kg of AAV8 vectors expressing sequence optimized hGAA (hGAAco) under the transcriptional control of human alpha-1-antytripsin promoter and fused with signal peptides 1, 2, 7 and 8 (sp1, 2, 7, 8). **Panel A.** The histogram plot shows the hGAA activity measured by fluorogenic assay in blood three months after vectors injection. Statistical analysis has been performed by ANOVA (* = p<0.05 as indicated, § = p<0.05 vs sp1 treated mice). **Panel B.** Kaplan-Mayer survival curve measured on mice treated as described above and followed for 6 months. Statistical analysis has been performed by log-rank test (* = p<0.05). **Panel C.** Respiratory function assessment. Histograms show the tidal volume, in milliliters (ml) measured three (gray bars) and six (black bars) months after the treatment with indicated vectors. Statistical analysis has been performed by ANOVA, in the histogram are reported the p-values obtained vs sp1 treated GAA -/- animals (* = p<0.05).

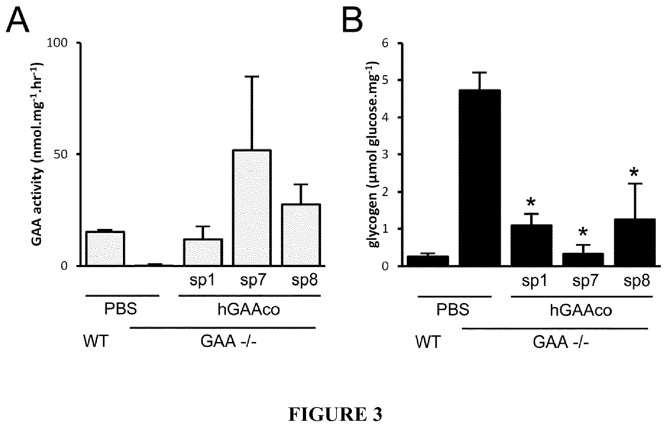

**Figure 3****.** Biochemical correction of glycogen content in quadriceps. 4 months-old GAA^{-/-} mice were intravenously injected with PBS or 2E12 vg/kg of AAV8 vectors expressing sequence optimized hGAA (hGAAco) under the transcriptional control of human alpha-1-antytripsin promoter and fused with signal peptides 1, 7 and 8 (sp1, 7, 8). **Panel A.** hGAA activity measured by fluorogenic assay in quadriceps. **Panel B.** In the histogram is shown the glycogen content expressed as glucose released after enzymatic digestion of glycogen, measured in the quadriceps. Statistical analysis has been performed by ANOVA (*=p<0.05 vs PBS injected GAA -/- mice).

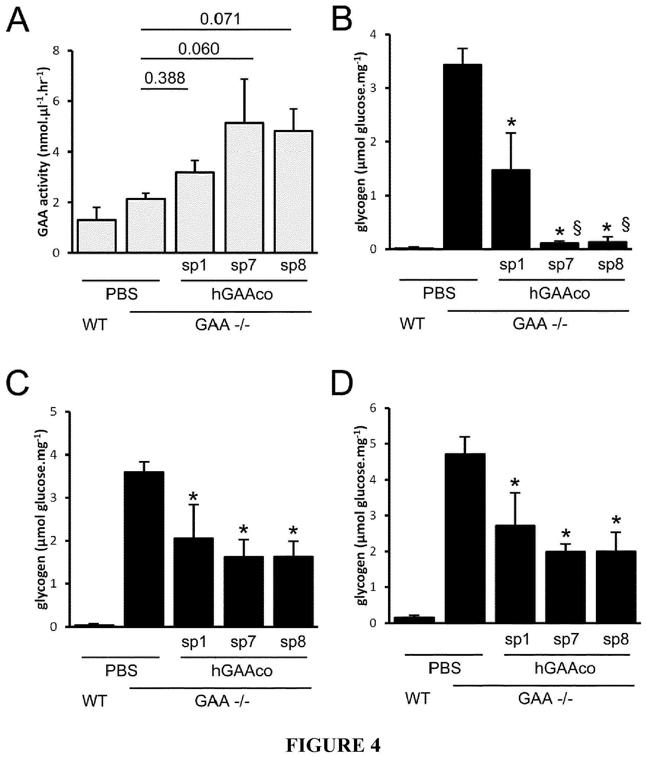

**Figure 4****.** Biochemical correction of glycogen content in heart, diaphragm and quadriceps. 4 months-old wild type (WT) and GAA^{-/-} mice (n=4-5 mice/group) were intravenously injected with PBS 6E11 vg/kg of AAV8 vectors expressing sequence optimized hGAA (hGAAco) under the transcriptional control of human alpha-1-antytripsin promoter and fused with signal peptides 1, 7 and 8 (sp1, 7, 8). **Panel A.** The histogram plot shows the hGAA activity measured by fluorogenic assay in blood three months after vector injection. Statistical analysis has been performed by ANOVA, in the histogram are reported the p-values obtained vs PBS treated GAA -/- animals (* = p<0.05). **Panel B-D.** The histogram plots show the glycogen content expressed as glucose released after enzymatic digestion of glycogen, measured in the heart **(panel B),** diaphragm **(panel C)** and quadriceps **(panel D).** Statistical analysis has been performed by ANOVA (* = p<0.05 vs PBS injected GAA -/- mice, § = p<0,05 vs. sp1-treated mice).

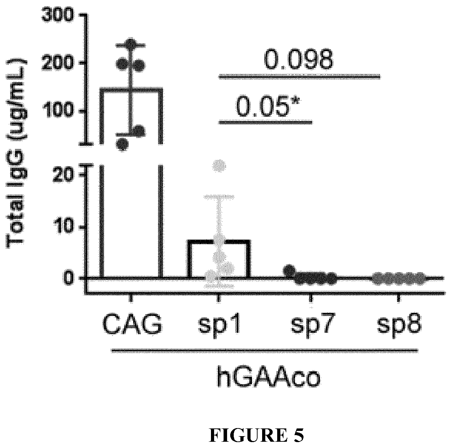

**Figure 5****.** Highly secreted hGAA reduces humoral responses directed against the transgene in a Pompe disease mouse model. 4 months-old GAA-/- mice were intravenously injected with PBS or 6E11 vg/kg of AAV8 vectors expressing sequence optimized hGAA (hGAAco) under the transcriptional control of human alpha-1-antytripsin promoter and fused with signal peptide 1, 7 and 8 (sp1, 7, 8). 1 month after the injections, sera were analyzed for the presence of anti-hGAA antibodies by ELISA. The quantification of circulating IgG levels has been performed against a standard curve of purified mouse IgG. The sera of mice injected intramuscularly with a AAV9 vector expressing sp1 hGAAco under the transcriptional control of a constitutive promoter (CAG) were loaded as positive control and not considered in the statistical analysis. Statistical analysis has been performed by ANOVA (* = p≤0.05 vs sp1)

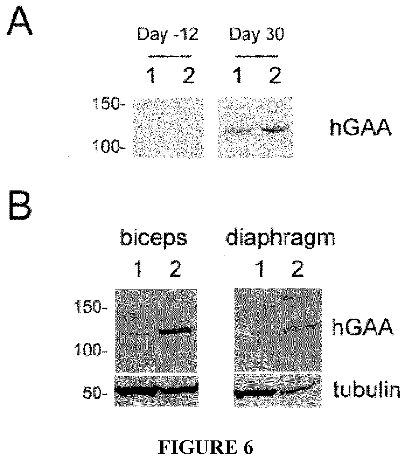

**Figure 6****.** AAV8-hAAT-sp7-Δ8-hGAAco1 injection leads to efficacious secretion of hGAA in the blood and uptake in muscle in NHP. Two *Macaca Fascicularis* monkeys were injected at day 0 with 2E12 vg/kg of AAV8-hAAT-sp7-A8-hGAAco1.**Panel A** hGAA western blot performed on serum from the two monkeys obtained twelve days before and 30 days after vector administration. On the left are indicated the positions of the bands of the molecular weight marker (st) running in parallel with the samples. **Panel B** Three months after vector injection the monkeys were sacrificed and tissues harvested for biochemical evaluation of hGAA uptake. A hGAA Western blot was performed on tissue extracts obtained from biceps and diaphragm. An anti-tubulin antibody was used as loading control. On the left are indicated the positions of the bands of the molecular weight marker running in parallel with the samples.

### DETAILED DESCRIPTION OF THE INVENTION

The present invention relates to a nucleic acid molecule encoding a chimeric GAA polypeptide. This chimeric GAA polypeptide comprises a signal peptide moiety and a functional GAA moiety, wherein the signal peptide moiety is selected in the group consisting of SEQ ID NO:2 to 4. The inventors have surprisingly shown that fusion of one of these signal peptides to a GAA protein greatly improves GAA secretion while reducing its immunogenicity.

Lysosomal acid α-glucosidase or "GAA" (E.C. 3.2. 1.20) (1,4-α-D-glucan glucohydrolase), is an exo-1,4-α-D-glucosidase that hydrolyses both α-1,4 and α-1,6 linkages of oligosaccharides to liberate glucose. A deficiency in GAA results in glycogen storage disease type II (GSDII), also referred to as Pompe disease (although this term formally refers to the infantile onset form of the disease). It catalyzes the complete degradation of glycogen with slowing at branching points. The 28 kb human acid α-glucosidase gene on chromosome 17 encodes a 3.6 kb mRNA which produces a 952 amino acid polypeptide (Hoefsloot et al., (1988) EMBO J. 7: 1697; Martiniuk et al., (1990) DNA and Cell Biology 9: 85). The enzyme receives co-translational N-linked glycosylation in the endoplasmic reticulum. It is synthesized as a 110-kDa precursor form, which matures by extensive glycosylation modification, phosphorylation and by proteolytic processing through an approximately 90-kDa endosomal intermediate into the final lysosomal 76 and 67 kDa forms (Hoefsloot, (1988) EMBO J. 7: 1697; Hoefsloot et al., (1990) Biochem. J. 272: 485; Wisselaar et al., (1993) J. Biol. Chem. 268: 2223; Hermans et al., (1993) Biochem. J. 289: 681).

In patients with GSD II, a deficiency of acid α-glucosidase causes massive accumulation of glycogen in lysosomes, disrupting cellular function (Hirschhorn, R. and Reuser, A. J. (2001), in The Metabolic and Molecular Basis for Inherited Disease, (eds, Scriver, C. R. et al.) pages 3389-3419 (McGraw-Hill, New York). In the most common infantile form, patients exhibit progressive muscle degeneration and cardiomyopathy and die before two years of age. Severe debilitation is present in the juvenile and adult onset forms.

Furthermore, patients having other GSDs may benefit from the administration of an optimized form of GAA. For example, it has been shown (Sun et al. (2013) Mol Genet Metab 108(2): 145; WO2010/005565) that administration of GAA reduces glycogen in primary myoblasts from glycogen storage disease type III (GSD III) patients.

The term "GAA" or "GAA polypeptide", as used herein, encompasses mature (∼76 or ∼67 kDa) and precursor (e.g., ∼110 kDa) GAA, in particular the precursor form, as well as modified or mutated by insertion(s), deletion (s) and/or substitution(s)) GAA proteins or fragments thereof that are functional derivatives of GAA, i.e. that retain biological function of GAA (i.e., have at least one biological activity of the native GAA protein, e. g., can hydrolyze glycogen, as defined above) and GAA variants (e.g., GAA II as described by Kunita et al., (1997) Biochemica et Biophysica Acta 1362: 269; GAA polymorphisms and SNPs are described by Hirschhorn, R. and Reuser, A. J. (2001) In The Metabolic and Molecular Basis for Inherited Disease (Scriver, C. R. , Beaudet, A. L., Sly, W. S. & Valle, D. Eds. ), pp. 3389- 3419. McGraw-Hill, New York, see pages 3403-3405). Any GAA coding sequence known in the art may be used, for example, see SEQ ID NO:1; GenBank Accession number NM_00152 and Hoefsloot et al., (1988) EMBO J. 7: 1697 and Van Hove et al., (1996) Proc. Natl. Acad. Sci. USA 93: 65 (human), GenBank Accession number NM_008064 (mouse), and Kunita et al., (1997) Biochemica et Biophysica Acta 1362: 269 (quail).

In the context of the present invention, a "precursor form of GAA" is a form of the GAA polypeptide that comprises its natural signal peptide. For example, the sequence of SEQ ID NO:12 and SEQ ID NO:37 are the precursor forms of human GAA (hGAA) variants. Within SEQ ID NO:12 and SEQ ID NO:37, amino acid residues 1-27 correspond to the signal peptide of the hGAA polypeptide.

In the context of the present invention, a truncated GAA polypeptide of the invention is derived from a parent GAA polypeptide. According to the present invention a "parent GAA polypeptide" may be a functional, precursor GAA sequence as defined above, but devoid of its signal peptide. For example, with reference to wild-type human GAA polypeptide, a complete wild-type GAA polypeptide (i.e. the precursor form of GAA) is represented in SEQ ID NO:12 or SEQ ID NO:37 and has a signal peptide (corresponding to amino acids 1-27 of SEQ ID NO:12 or SEQ ID NO:37), whereas the parent GAA polypeptide serving as basis for the truncated GAA forms of these wild-type human GAA polypeptides are represented in SEQ ID NO:5 and SEQ ID NO:36 and have no signal peptide. In this example, the latter, corresponding to amino acids 28-952 of SEQ ID NO:12 and to amino acids 28-952 of SEQ ID NO37, is referred to as a parent GAA polypeptide.

The coding sequence of the GAA polypeptide can be derived from any source, including avian and mammalian species. The term "avian" as used herein includes, but is not limited to, chickens, ducks, geese, quail, turkeys and pheasants. The term "mammal" as used herein includes, but is not limited to, humans, simians and other non-human primates, bovines, ovines, caprines, equines, felines, canines, lagomorphs, etc. In embodiments of the invention, the nucleic acids of the invention encode a human, mouse or quail, in particular a human, GAA polypeptide. In a further particular embodiment, the GAA polypeptide encoded by the nucleic acid molecule of the invention comprises the amino acid sequence shown in SEQ ID NO:5 or in SEQ ID NO:36, which corresponds to hGAA without its signal peptide (of note, the natural signal peptide of hGAA corresponds to amino acid 1-27 in SEQ ID NO:12 or in SEQ ID NO:37, which corresponds to hGAA including its natural signal peptide).

In another embodiment of the invention, the nucleic acid molecule of the invention has at least 75 percent (such as at least 77%), at least 80 percent or at least 82 percent (such as at least 83%) identify to nucleotides 82-2859 of the sequence shown in SEQ ID NO:1, which is the sequence coding the wild-type hGAA of SEQ ID NO:37 (nucleotides 1-81 of SEQ ID NO:1 being the part encoding for the natural signal peptide of hGAA).

The GAA moiety of the nucleic acid molecule of the invention preferably has at least 85 percent, more preferably at least 90 percent, and even more preferably at least 92 percent identity, in particular at least 95 percent identity, for example at least 98, 99 or 100 percent identity to the nucleotide sequence of SEQ ID NO: 13 or 14, which are sequences optimized for transgene expression *in vivo.*

In addition, the signal peptide moiety of the chimeric GAA protein encoded by the nucleic acid molecule of the invention may comprise from 1 to 5, in particular from 1 to 4, in particular from 1 to 3, more particularly from 1 to 2, in particular 1 amino acid deletion(s), insertion(s) or substitution(s) as compared to the sequences shown in SEQ ID NO:2 to 4, as long as the resulting sequence corresponds to a functional signal peptide, i.e. a signal peptide to that allows secretion of a GAA protein. In a particular embodiment, the signal peptide moiety sequence consists of a sequence selected in the group consisting of SEQ ID NO:2 to 4.

The term "identical" and declinations thereof refers to the sequence identity between two nucleic acid molecules. When a position in both of the two compared sequences is occupied by the same base e.g., if a position in each of two DNA molecules is occupied by adenine, then the molecules are identical at that position. The percent of identity between two sequences is a function of the number of matching positions shared by the two sequences divided by the number of positions compared X 100. For example, if 6 of 10 of the positions in two sequences are matched then the two sequences are 60% identical. Generally, a comparison is made when two sequences are aligned to give maximum identity. Various bioinformatic tools known to the one skilled in the art might be used to align nucleic acid sequences such as BLAST or FASTA.

In a particular embodiment, the GAA moiety of the nucleic acid molecule of the invention comprises the sequence shown in SEQ ID NO:13 or SEQ ID NO: 14.

The nucleic acid molecule of the invention encodes a functional GAA polypeptide, i.e. it encodes for a human GAA polypeptide that, when expressed, has the functionality of wild-type GAA protein. As defined above, the functionality of wild-type GAA is to hydrolyse both α-1,4 and α-1,6 linkages of oligosaccharides and polysaccharides, more particularly of glycogen, to liberate glucose. The functional GAA polypeptide encoded by the nucleic acid of the invention may have a hydrolysing activity on glycogen of at least 50 %, 60 %, 70 %, 80 %, 90 %, 95 %, 99 %, or at least 100 % as compared to the wild-type GAA polypeptide encoded by the nucleic acid sequence of SEQ ID NO:1, SEQ ID NO:13 or SEQ ID NO:14 (i.e. the GAA polypeptide having the amino acid sequence of SEQ ID NO:5). The activity of the GAA protein encoded by the nucleic acid of the invention may even be of more than 100 %, such as of more than 110 %, 120 %, 130 %, 140 %, or even more than 150 % of the activity of the wild-type GAA polypeptide encoded by the nucleic acid sequence of SEQ ID SEQ ID NO:1, NO:13 or SEQ ID NO:14 (i.e. the GAA polypeptide having the amino acid sequence of SEQ ID NO:5).

A skilled person is readily able to determine whether a nucleic acid according to the invention expresses a functional GAA protein. Suitable methods would be apparent to those skilled in the art. For example, one suitable in vitro method involves inserting the nucleic acid into a vector, such as a plasmid or viral vector, transfecting or transducing host cells, such as 293T or HeLa cells, or other cells such as Huh7, with the vector, and assaying for GAA activity. Alternatively, a suitable *in vivo* method involves transducing a vector containing the nucleic acid into a mouse model of Pompe disease or another glycogen storage disorder and assaying for functional GAA in the plasma of the mouse and presence of GAA in tissues. Suitable methods are described in more details in the experimental part below.

The inventors have found that the above described nucleic acid molecule causes surprisingly high levels of expression of functional GAA protein both *in vitro* and *in vivo* compared to the wild-type GAA cDNA. Furthermore, as also shown by the inventors, the chimeric GAA polypeptide produced from liver and muscle cells expressing the nucleic acid molecule of the invention induces no humoral immune response against the transgene. This means that this nucleic acid molecule may be used to produce high levels of GAA polypeptide, and provides therapeutic benefits such as avoiding to resort to immunosuppressive treatments, allowing low dose immunosuppressive treatment, and allowing repeated administration of the nucleic acid molecule of the invention to a subject in need thereof. Therefore, the nucleic acid molecule of the invention is of special interest in contexts where GAA expression and/or activity is deficient or where high levels of expression of GAA can ameliorate a disease, such as for a glycogen storage disease. In a particular, the glycogen storage disease may be GSDI (von Gierke's disease), GSDII (Pompe disease), GSDIII (Cori disease), GSDIV, GSDV, GSDVI, GSDVII, GSDVIII or lethal congenital glycogen storage disease of the heart. More particularly, the glycogen storage disease is selected in the group consisting of GSDI, GSDII and GSDIII, even more particularly in the group consisting of GSDII and GSDIII. In an even more particular embodiment, the glycogen storage disease is GSDII. In particular, the nucleic acid molecules of the invention may be useful in gene therapy to treat GAA-deficient conditions, or other conditions associated by accumulation of glycogen such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII. In an even more particular embodiment, the nucleic acid molecules of the invention may be useful in gene therapy to treat GSDII.

The sequence of the nucleic acid molecule of the invention, encoding a functional GAA, is optimized for expression of the GAA polypeptide *in vivo.* Sequence optimization may include a number of changes in a nucleic acid sequence, including codon optimization, increase of GC content, decrease of the number of CpG islands, decrease of the number of alternative open reading frames (ARFs) and decrease of the number of splice donor and splice acceptor sites. Because of the degeneracy of the genetic code, different nucleic acid molecules may encode the same protein. It is also well known that the genetic codes of different organisms are often biased towards using one of the several codons that encode the same amino acid over the others. Through codon optimization, changes are introduced in a nucleotide sequence that take advantage of the codon bias existing in a given cellular context so that the resulting codon optimized nucleotide sequence is more likely to be expressed in such given cellular context at a relatively high level compared to the non-codon optimised sequence. In a preferred embodiment of the invention, such sequence optimized nucleotide sequence encoding a truncated GAA is codon-optimized to improve its expression in human cells compared to non-codon optimized nucleotide sequences coding for the same truncated GAA protein, for example by taking advantage of the human specific codon usage bias.

Table 3 provides a description of relevant parameters with respect to sequence optimization conducted by the inventors:

| sequence | WT | co1 | co2 |
|---|---|---|---|
| CAI^{a} | 0.84 | 0.94 | 0.77 |
| GC content^{b} | 64.7 | 61.9 | 54.4 |
| aORF 5'→3'^{c} | 2 | 3 | 0 |
| aORF 3'→5'^{d} | 5 | 4 | 0 |
| SA^{e} | 3 | 0 | 1 |
| SD^{f} | 3 | 0 | 0 |
| % identity vs wt^{g} | | 83.1 | 77.7 |
| % identity vs co1^{h} | | | 80.8 |
| CpG islandsⁱ | 4 | 5 | 1 |

| | | | |
|---|---|---|---|
| **Table 3.** Description of the optimized sequences. Table illustrating the characteristics of the two hGAA optimized sequences compared to the wild-type one. **a)** codon adaptation index and **b)** GC content calculated using a rare codon analysis tool (http://www.genscript.com). **c)** and **d)** are respectively the alternative open reading frames calculated on the 5' to 3' (aORF 5'→3')and 3' to 5' (aORF 3'→5')strands. **e)** and **f)** are respectively the acceptor (SA) and donor (SD) splicing sites calculated using a splicing site online prediction tool (http://www.fruitfly.org/se_tools/splice.html). **g)** and **h)** are respectively the percentual identity calculated versus wild-type (wt) and optimized co1 sequence. i) CpG islands calculated using MethDB online tool (http://www.methdb.de/links.html). CpG islands are sequences longer than 100 bp, with GC content>60% and an observed/expected ratio>0.6. | | | |

In a particular embodiment, the optimized GAA coding sequence is codon optimized, and/or has an increased GC content and/or has a decreased number of alternative open reading frames, and/or has a decreased number of splice donor and/or splice acceptor sites, as compared to nucleotides 82-2859 of the wild-type hGAA coding sequence of SEQ ID NO:1. For example, nucleic acid sequence of the invention results in an at least 2, 3, 4, 5 or 10 % increase of GC content in the GAA sequence as compared to the sequence of the wild-type GAA sequence. In a particular embodiment, the nucleic acid sequence of the invention results in a 2, 3, 4 or, more particularly, 5% or 10% (particularly 5%) increase of GC content in the GAA sequence as compared to the sequence of the wild-type GAA nucleotide sequence. In a particular embodiment, the nucleic acid sequence of the invention encoding a functional GAA polypeptide is "substantially identical", that is, about 70% identical, more preferably about 80% identical, even more preferably about 90% identical, even more preferably about 95% identical, even more preferably about 97%, 98% or even 99% identical to nucleotides 82-2859 of the sequence shown in SEQ ID NO: 1. As mentioned above, in addition to the GC content and/or number of ARFs, sequence optimization may also comprise a decrease in the number of CpG islands in the sequence and/or a decrease in the number of splice donor and acceptor sites. Of course, as is well known to those skilled in the art, sequence optimization is a balance between all these parameters, meaning that a sequence may be considered optimized if at least one of the above parameters is improved while one or more of the other parameters is not, as long as the optimized sequence leads to an improvement of the transgene, such as an improved expression and/or a decreased immune response to the transgene *in vivo.*

In addition, the adaptiveness of a nucleotide sequence encoding a functional GAA to the codon usage of human cells may be expressed as codon adaptation index (CAI). A codon adaptation index is herein defined as a measurement of the relative adaptiveness of the codon usage of a gene towards the codon usage of highly expressed human genes. The relative adaptiveness (w) of each codon is the ratio of the usage of each codon, to that of the most abundant codon for the same amino acid. The CAI is defined as the geometric mean of these relative adaptiveness values. Non-synonymous codons and termination codons (dependent on genetic code) are excluded. CAI values range from 0 to 1, with higher values indicating a higher proportion of the most abundant codons (see Sharp and Li, 1987, Nucleic Acids Research 15: 1281-1295; also see: Kim et al, Gene. 1997, 199:293-301; zur Megede et al, Journal of Virology, 2000, 74: 2628-2635). Preferably, a nucleic acid molecule encoding a GAA has a CAI of at least 0.75 (in particular 0.77), 0.8, 0.85, 0.90, 0.92 or 0.94.

In one embodiment, the nucleic acid molecule of the invention encodes a protein having between 0 and 50, between 0 and 30, between 0 and 20, between 0 and 15, between 0 and 10, or between 0 and 5 amino acid changes to the protein encoded by the nucleotide sequence of SEQ ID NO: 13 or SEQ ID NO:14. Furthermore, the GAA protein encoded by the nucleic acid of the invention may be a variant of a functional GAA protein known in the art, wherein the nucleic acid molecule of the invention encodes a protein having between 0 and 50, between 0 and 30, between 0 and 20, between 0 and 15, between 0 and 10, or between 0 and 5 amino acid changes to GAA protein known in the art. Such GAA protein known in the art that may serve as the basis for designing functional variant may be found in particular in the Uniprot entry of GAA (accession number P10253; corresponding to BenBank CAA68763.1; SEQ ID NO:37). In a further particular embodiment, the GAA moiety of the nucleic acid sequence of the invention encodes variants GAA polypeptides, or functional variants of such peptides as defined herein, such as those selected in the group consisting of the polypeptides identified as Genbank Accession Numbers AAA52506.1 (SEQ ID NO:38), EAW89583.1 (SEQ ID NO:39) and ABI53718.1 (SEQ ID NO:40). Other variant GAA polypeptides include those described in WO2012/145644, WO00/34451 and US6,858,425. In a particular embodiment, the parent GAA polypeptide is derived from the amino acid sequence shown in SEQ ID NO: 12 or SEQ ID NO:37.

In a particular embodiment, the GAA polypeptide encoded by the nucleic acid molecule of the invention is a functional GAA and has a sequence identity to hGAA protein shown in SEQ ID NO:5 or SEQ ID NO:36, in particular in SEQ ID NO:5, optionally taking into account the truncation carried out if a truncated form is considered as a reference to sequence identity, of at least 80 %, in particular at least 85 %, 90 %, 95 %, more particularly at least 96%, 97%, 98%, or 99%. In a particular embodiment, the GAA protein encoded by the nucleic acid molecule of the invention has the sequence shown in SEQ ID NO:5 or SEQ ID NO:36, in particular in SEQ ID NO:5.

The term "nucleic acid sequence" (or nucleic acid molecule) refers to a DNA or RNA molecule in single or double stranded form, particularly a DNA encoding a GAA protein according to the invention.

The invention also relates to a nucleic acid molecule encoding a chimeric functional GAA polypeptide comprising a signal peptide selected in the group consisting of SEQ ID NO:2 to 4.

In particular, the inventors have further surprisingly shown that signal peptide replacement results in the production of higher expression levels and higher secretion of functional GAA polypeptide as compared to a previously reported other chimeric GAA polypeptide comprising GAA fused to the signal peptide of human alpha-1-antitrypsin (hAAT, chimeric GAA protein described in WO2004064750 and Sun et al. 2006). In the nucleic acid molecule of the invention, the signal peptide moiety corresponds to a sequence encoding a signal peptide having an amino acid sequence selected in the group consisting of SEQ ID NO:2 to 4 (otherwise referred to herein as an "alternative signal peptide"). The nucleic acid molecule of the invention may further be an optimized sequence coding for a chimeric GAA polypeptide comprising an alternative signal peptide operably linked to a functional GAA polypeptide.

As compared to a wild-type GAA polypeptide, the endogenous signal peptide of wild-type GAA is replaced with an exogenous signal peptide, i.e. a signal peptide derived from a protein different from GAA. The exogenous signal peptide fused to the remainder of the GAA protein increases the secretion of the resulting chimeric GAA polypeptide as compared to the corresponding GAA polypeptide comprising its natural signal peptide. Furthermore, according to a particular embodiment of the invention, the nucleotide sequence corresponding to the alternative signal peptide may be an optimized sequence as provided above.

The signal peptides workable in the present invention include amino acids 1-25 from iduronate-2-sulphatase (SEQ ID NO:3), amino acids 1-20 from chymotrypsinogen B2 (SEQ ID NO:2) and amino acids 1-23 from protease C1 inhibitor (SEQ ID NO:4). The signal peptides of SEQ ID NO:2 to SEQ ID NO:4, allow higher secretion of the chimeric GAA protein both *in vitro* and *in vivo* when compared to the GAA comprising its natural signal peptide, or to a chimeric GAA protein comprising the signal peptide of hAAT.

The relative proportion of newly-synthesized GAA that is secreted from the cell can be routinely determined by methods known in the art and described in the examples. Secreted proteins can be detected by directly measuring the protein itself (e.g., by Western blot) or by protein activity assays (e.g. , enzyme assays) in cell culture medium, serum, milk, etc.

Those skilled in the art will further understand that the chimeric GAA polypeptide can contain additional amino acids, e. g., as a result of manipulations of the nucleic acid construct such as the addition of a restriction site, as long as these additional amino acids do not render the signal peptide or the GAA polypeptide non-functional. The additional amino acids can be cleaved or can be retained by the mature polypeptide as long as retention does not result in a non-functional polypeptide.

Furthermore, the chimeric GAA polypeptide encoded by the nucleic acid molecule as herein described may comprise a GAA moiety that is a functional, truncated form of GAA. By "truncated form", it is meant a GAA polypeptide that comprises one or several consecutive amino acids deleted from the N-terminal part of a parent GAA polypeptide. Therefore, the GAA moiety in the chimeric GAA polypeptide of the invention may be a N-terminally truncated form of a parent GAA polypeptide. According to the present invention, a "parent GAA polypeptide" is a GAA polypeptide devoid of a signal peptide, such as a precursor form of a GAA devoid of a signal peptide, in particular the hGAA polypeptide shown in SEQ ID NO:5, or SEQ ID NO:36, in particular in SEQ ID NO5, and may be any of the variants as disclosed above. For example, with reference to typical wild-type human GAA polypeptides, the complete wild-type GAA polypeptide is represented in SEQ ID NO:12 or in SEQ ID NO:37, and have a signal peptide, whereas the parent GAA polypeptide serving as basis for the truncated GAA form of this wild-type human GAA polypeptide is represented in SEQ ID NO:5 or SEQ ID NO:36, respectively, and have no signal peptide. In this example, the latter are referred to as a parent GAA polypeptide. In a variant of this particular embodiment, at least one amino acid is deleted from the N-terminal end of the parent GAA protein. For example, the GAA moiety may have 1 to 75 consecutive amino acids or more than 75 consecutive amino acids deleted from its N-terminal end as compared to the parent GAA polypeptide. Specifically, the truncated GAA moiety may have 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34,35,36,37,38,39,40,41,42,43,44,45,46,47,48,49,50,51,52,53,54,55,56,57,58,59,60, 61, 62, 63, 64, 65, 66, 67, 68, 69, 70, 71, 72, 73, 74 or 75 consecutive amino acids deleted from its N-terminal end as compared to the parent GAA protein (in particular a truncated form of the parent hGAA polypeptide shown in SEQ ID NO:5 or SEQ ID NO:36, in particular in SEQ ID NO:5). Using an alternative nomenclature, the GAA polypeptide resulting from the truncation of 1 amino acid in the parent GAA polypeptide is referred to as Δ1 GAA truncated form, the GAA polypeptide resulting from the truncation of 2 consecutive amino acids from the N-terminal end is referred to as Δ2 GAA truncated form, the GAA polypeptide resulting from the truncation of 3 consecutive amino acids in the parent GAA polypeptide is referred to as Δ3 GAA truncated form), etc. In a particular embodiment, the chimeric GAA protein of the invention comprises a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46, Δ47, Δ48, Δ49, Δ50, Δ51, Δ52, Δ53, Δ54, Δ55, Δ56, Δ57, Δ58, Δ59, Δ60, Δ61, Δ62, Δ63, Δ64, Δ65, Δ66, Δ67, Δ68, Δ69, Δ70, Δ71, Δ72, Δ73, Δ74 or Δ75 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4. In a particular embodiment, the GAA moiety of the chimeric GAA protein is a Δ6, Δ7, Δ8, Δ9 or Δ10 truncated form of GAA (in particular of the parent hGAA protein shown in SEQ ID NO: 5 or SEQ ID NO:36, in particular in SEQ ID NO:5), in particular a Δ8 truncated form of GAA (in particular of the parent hGAA protein shown in SEQ ID NO: 5 or SEQ ID NO:36, in particular in SEQ ID NO:5). In another particular embodiment, the GAA moiety of the chimeric GAA protein is a Δ40, Δ41, Δ42, Δ43 or Δ44 truncated form of GAA (in particular of the parent hGAA protein shown in SEQ ID NO: 5 or SEQ ID NO:36, in particular in SEQ ID NO:5), in particular a Δ42 truncated form of GAA (in particular of the parent hGAA protein shown in SEQ ID NO: 5 or SEQ ID NO:36, in particular in SEQ ID NO:5).

In a particular embodiment, of the invention, the chimeric GAA polypeptide of the invention comprises a truncated GAA moiety derived from a functional parent human GAA polypeptide. In a further particular embodiment, the parent hGAA polypeptide is the hGAA polypeptide shown in SEQ ID NO:5 or SEQ ID NO:36, in particular in SEQ ID NO:5. In a variant of this embodiment, the GAA moiety in the chimeric GAA polypeptide of the invention is a Δ1, Δ2, Δ3, Δ4, Δ5, Δ6, Δ7, Δ8, Δ9, Δ10, 411, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46, Δ47, Δ48, Δ49, Δ50, Δ51, Δ52, Δ53, Δ54, Δ55, Δ56, Δ57, Δ58, Δ59, Δ60, Δ61, Δ62, Δ63, Δ64, Δ65, Δ66, Δ67, Δ68, Δ69, Δ70, Δ71, Δ72, Δ73, Δ74 or Δ75 GAA truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 75, 80, 85, 90, 91, 92, 93, 94, 95, 96, 97, 98 or 99 percent identity to SEQ ID NO:5 or SEQ ID NO:36, in particular to SEQ ID NO:5. In a further particular embodiment, the GAA moiety of the chimeric GAA polypeptide of the invention is a Δ6, Δ7, Δ8, Δ9, Δ10, Δ40, Δ41, Δ42, Δ43 or Δ44, in particular a Δ8 or Δ42 truncated form of a hGAA polypeptide, and more particularly of the hGAA polypeptide shown in SEQ ID NO:5 or SEQ ID NO:36, in particular in SEQ ID NO:5, or of a functional variant thereof comprising amino acid substitutions in the sequence shown in SEQ ID NO:5 or SEQ ID NO:36, in particular in SEQ ID NO:5, and having at least 80, 85, 90, 95, 96, 97, 98 or 99 percent identity to SEQ ID NO:5 or SEQ ID NO:36, in particular to SEQ ID NO:5. In a specific embodiment, the GAA moiety in the chimeric GAA polypeptide of the invention has an amino acid sequence consisting of the sequence shown in SEQ ID NO:29 or SEQ ID NO:30.

The invention also relates to a nucleic acid construct comprising a nucleic acid molecule of the invention. The nucleic acid construct may correspond to an expression cassette comprising the nucleic acid sequence of the invention, operably linked to one or more expression control sequences and/or other sequences improving the expression of a transgene and/or sequences enhancing the secretion of the encoded protein and/or sequences enhancing the uptake of the encoded protein. As used herein, the term "operably linked" refers to a linkage of polynucleotide elements in a functional relationship. A nucleic acid is "operably linked" when it is placed into a functional relationship with another nucleic acid sequence. For instance, a promoter, or another transcription regulatory sequence, is operably linked to a coding sequence if it affects the transcription of the coding sequence. Such expression control sequences are known in the art, such as promoters, enhancers (such as cis-regulatory modules (CRMs)), introns, polyA signals, etc.

In particular, the expression cassette may include a promoter. The promoter may be an ubiquitous or tissue-specific promoter, in particular a promoter able to promote expression in cells or tissues in which expression of GAA is desirable such as in cells or tissues in which GAA expression is desirable in GAA-deficient patients. In a particular embodiment, the promoter is a liver-specific promoter such as the alpha-1 antitrypsin promoter (hAAT) (SEQ ID NO:15), the transthyretin promoter, the albumin promoter, the thyroxine-binding globulin (TBG) promoter, the LSP promoter (comprising a thyroid hormone-binding globulin promoter sequence, two copies of an alphal-microglobulin/bikunin enhancer sequence, and a leader sequence - 34.Ill, C. R., et al. (1997). Optimization of the human factor VIII complementary DNA expression plasmid for gene therapy of hemophilia A. Blood Coag. Fibrinol. 8: S23-S30.), etc. Other useful liver-specific promoters are known in the art, for example those listed in the Liver Specific Gene Promoter Database compiled the Cold Spring Harbor Laboratory (http://rulai.cshl.edu/LSPD/). A preferred promoter in the context of the invention is the hAAT promoter. In another embodiment, the promoter is a promoter directing expression in one tissue or cell of interest (such as in muscle cells), and in liver cells. For example, to some extent, promoters specific of muscle cells such as the desmin, Spc5-12 and MCK promoters may present some leakage of expression into liver cells, which can be advantageous to induce immune tolerance of the subject to the GAA protein expressed from the nucleic acid of the invention.

Other tissue-specific or non-tissue-specific promoters may be useful in the practice of the invention. For example, the expression cassette may include a tissue-specific promoter which is a promoter different from a liver specific promoter. For example the promoter may be muscle-specific, such as the desmin promoter (and a desmin promoter variant such as a desmin promoter including natural or artificial enhancers), the SPc5-12 promoter or the MCK promoter. In another embodiment, the promoter is a promoter specific of other cell lineage, such as the erythropoietin promoter, for the expression of the GAA polypeptide from cells of the erythroid lineage.

In another embodiment, the promoter is an ubiquitous promoter. Representative ubiquitous promoters include the cytomegalovirus enhancer/chicken beta actin (CAG) promoter, the cytomegalovirus enhancer/promoter (CMV), the PGK promoter, the SV40 early promoter, etc.

In addition, the promoter may also be an endogenous promoter such as the albumin promoter or the GAA promoter.

In a particular embodiment, the promoter is associated to an enhancer sequence, such as cis-regulatory modules (CRMs) or an artificial enhancer sequence. For example, the promoter may be associated to an enhancer sequence such as the human ApoE control region (or Human apolipoprotein E/C-I gene locus, hepatic control region HCR-1 - Genbank accession No. U32510, shown in SEQ ID NO:16). In a particular embodiment, an enhancer sequence such as the ApoE sequence is associated to a liver-specific promoter such as those listed above, and in particular such as the hAAT promoter. Other CRMs useful in the practice of the present invention include those described in Rincon et al., Mol Ther. 2015 Jan;23(1):43-52, Chuah et al., Mol Ther. 2014 Sep;22(9):1605-13 or Nair et al., Blood. 2014 May 15;123(20):3195-9.

In another particular embodiment, the nucleic acid construct comprises an intron, in particular an intron placed between the promoter and the GAA coding sequence. An intron may be introduced to increase mRNA stability and the production of the protein. In a further embodiment, the nucleic acid construct comprises a human beta globin b2 (or HBB2) intron, a coagulation factor IX (FIX) intron, a SV40 intron or a chicken beta-globin intron. In another further embodiment, the nucleic acid construct of the invention contains a modified intron (in particular a modified HBB2 or FIX intron) designed to decrease the number of, or even totally remove, alternative open reading frames (ARFs) found in said intron. Preferably, ARFs are removed whose length spans over 50 bp and have a stop codon in frame with a start codon. ARFs may be removed by modifying the sequence of the intron. For example, modification may be carried out by way of nucleotide substitution, insertion or deletion, preferably by nucleotide substitution. As an illustration, one or more nucleotides, in particular one nucleotide, in an ATG or GTG start codon present in the sequence of the intron of interest may be replaced resulting in a non-start codon. For example, an ATG or a GTG may be replaced by a CTG, which is not a start codon, within the sequence of the intron of interest.

The classical HBB2 intron used in nucleic acid constructs is shown in SEQ ID NO:6. For example, this HBB2 intron may be modified by eliminating start codons (ATG and GTG codons) within said intron. In a particular embodiment, the modified HBB2 intron comprised in the construct has the sequence shown in SEQ ID NO:7. The classical FIX intron used in nucleic acid constructs is derived from the first intron of human FIX and is shown in SEQ ID NO:8. FIX intron may be modified by eliminating start codons (ATG and GTG codons) within said intron. In a particular embodiment, the modified FIX intron comprised in the construct of the invention has the sequence shown in SEQ ID NO:9. The classical chicken-beta globin intron used in nucleic acid constructs is shown in SEQ ID NO:10. Chicken-beta globin intron may be modified by eliminating start codons (ATG and GTG codons) within said intron. In a particular embodiment, the modified chicken-beta globin intron comprised in the construct of the invention has the sequence shown in SEQ ID NO:11.

The inventors have previously shown in WO2015/162302 that such a modified intron, in particular a modified HBB2 or FIX intron, has advantageous properties and can significantly improve the expression of a transgene.

In a particular embodiment, the nucleic acid construct of the invention is an expression cassette comprising, in the 5' to 3' orientation, a promoter optionally preceded by an enhancer, the coding sequence of the invention (i.e. the optimized GAA coding sequence of the invention, the chimeric GAA coding sequence of the invention, or the chimeric and optimized GAA coding sequence of the invention), and a polyadenylation signal (such as the bovine growth hormone polyadenylation signal, the SV40 polyadenylation signal, or another naturally occurring or artificial polyadenylation signal). In a particular embodiment, the nucleic acid construct of the invention is an expression cassette comprising, in the 5' to 3' orientation, a promoter optionally preceded by an enhancer, (such as the ApoE control region), an intron (in particular an intron as defined above), the coding sequence of the invention, and a polyadenylation signal. In a further particular embodiment, the nucleic acid construct of the invention is an expression cassette comprising, in the 5' to 3' orientation, an enhancer such as the ApoE control region, a promoter, an intron (in particular an intron as defined above), the coding sequence of the invention, and a polyadenylation signal. In a further particular embodiment of the invention the expression cassette comprising, in the 5' to 3' orientation, an ApoE control region, the hAAT-liver specific promoter, a HBB2 intron (in particular a modified HBB2 intron as defined above), the coding sequence of the invention, and the bovine growth hormone polyadenylation signal, such as the nucleic acid construct shown in any one of SEQ ID NO:20 to SEQ ID NO:22, which includes the sequence-optimized GAA nucleic acid molecule of SEQ ID NO:13 combined to each of the signal peptide-encoding sequences shown in SEQ ID NO:2 to 4. In other embodiments, the expression cassette contains the coding sequence resulting from one of the combinations of sequences shown in table 2 above.

In a particular embodiment, the expression cassette comprises the ApoE control region, the hAAT-liver specific promoter, a codon-optimized HBB2 intron, the coding sequence of the invention and the bovine growth hormone polyadenylation signal.

In designing the nucleic acid construct of the invention, one skilled in the art will take care of respecting the size limit of the vector used for delivering said construct to a cell or organ. In particular, one skilled in the art knows that a major limitation of AAV vector is its cargo capacity which may vary from one AAV serotype to another but is thought to be limited to around the size of parental viral genome. For example, 5 kb, is the maximum size usually thought to be packaged into an AAV8 capsid (Wu Z. et al., Mol Ther., 2010, 18(1): 80-86; Lai Y. et al., Mol Ther., 2010, 18(1): 75-79; Wang Y. et al., Hum Gene Ther Methods, 2012, 23(4): 225-33). Accordingly, those skilled in the art will take care in practicing the present invention to select the components of the nucleic acid construct of the invention so that the resulting nucleic acid sequence, including sequences coding AAV 5'- and 3'-ITRs to preferably not exceed 110 % of the cargo capacity of the AAV vector implemented, in particular to preferably not exceed 5.5 kb.

The invention also relates to a vector comprising a nucleic acid molecule or construct as disclosed herein. In particular, the vector of the invention is a vector suitable for protein expression, preferably for use in gene therapy. In one embodiment, the vector is a plasmid vector. In another embodiment, the vector is a nanoparticle containing a nucleic acid molecule of the invention, in particular a messenger RNA encoding the GAA polypeptide of the invention. In another embodiment, the vector is a system based on transposons, allowing integration of the nucleic acid molecule or construct of the invention in the genome of the target cell, such as the hyperactive Sleeping Beauty (SB100X) transposon system (Mates et al. 2009). In another embodiment, the vector is a viral vector suitable for gene therapy, targeting any cell of interest such as liver tissue or cells, muscle cell, CNS cells (such as brain cells), or hematopoietic stem cells such as cells of the erythroid lineage (such as erythrocytes). In this case, the nucleic acid construct of the invention also contains sequences suitable for producing an efficient viral vector, as is well known in the art. In a particular embodiment, the viral vector is derived from an integrating virus. In particular, the viral vector may be derived from a retrovirus or a lentivirus. In a further particular embodiment, the viral vector is an AAV vector, such as an AAV vector suitable for transducing liver tissues or cells, more particularly an AAV-1, -2 and AAV-2 variants (such as the quadruple-mutant capsid optimized AAV-2 comprising an engineered capsid with Y44+500+730F+T491V changes, disclosed in Ling et al., 2016 Jul 18, Hum Gene Ther Methods. [Epub ahead of print]), -3 and AAV-3 variants (such as the AAV3-ST variant comprising an engineered AAV3 capsid with two amino acid changes, S663V+T492V, disclosed in Vercauteren et al., 2016, Mol. Ther. Vol. 24(6), p. 1042), -3B and AAV-3B variants, -4, -5, -6 and AAV-6 variants (such as the AAV6 variant comprising the triply mutated AAV6 capsid Y731F/Y705F/T492V form disclosed in Rosario et al., 2016, Mol Ther Methods Clin Dev. 3, p.16026), -7, -8, -9, -10 such as - cy10 and -rh10, -rh74, -dj, Anc80, LK03, AAV2i8, porcine AAV serotypes such as AAVpo4 and AAVpo6, etc., vector or a retroviral vector such as a lentiviral vector and an alpha-retrovirus. As is known in the art, depending on the specific viral vector considered for use, additional suitable sequences will be introduced in the nucleic acid construct of the invention for obtaining a functional viral vector. Suitable sequences include AAV ITRs for an AAV vector, or LTRs for lentiviral vectors. As such, the invention also relates to an expression cassette as described above, flanked by an ITR or an LTR on each side.

Advantages of viral vectors are discussed in the following part of this disclosure. Viral vectors are preferred for delivering the nucleic acid molecule or construct of the invention, such as a retroviral vector, for example a lentiviral vector, or a non-pathogenic parvovirus, more preferably an AAV vector. The human parvovirus Adeno-Associated Virus (AAV) is a dependovirus that is naturally defective for replication which is able to integrate into the genome of the infected cell to establish a latent infection. The last property appears to be unique among mammalian viruses because the integration occurs at a specific site in the human genome, called AAVS1, located on chromosome 19 (19q13.3-qter).

Therefore, AAV vectors have arisen considerable interest as a potential vectors for human gene therapy. Among the favorable properties of the virus are its lack of association with any human disease, its ability to infect both dividing and non-dividing cells, and the wide range of cell lines derived from different tissues that can be infected.

Among the serotypes of AAVs isolated from human or non-human primates (NHP) and well characterized, human serotype 2 is the first AAV that was developed as a gene transfer vector. Other currently used AAV serotypes include AAV-1, AAV-2 variants (such as the quadruple-mutant capsid optimized AAV-2 comprising an engineered capsid with Y44+500+730F+T491V changes, disclosed in Ling et al., 2016 Jul 18, Hum Gene Ther Methods. [Epub ahead of print]), -3 and AAV-3 variants (such as the AAV3-ST variant comprising an engineered AAV3 capsid with two amino acid changes, S663V+T492V, disclosed in Vercauteren et al., 2016, Mol. Ther. Vol. 24(6), p. 1042), -3B and AAV-3B variants, -4, -5, -6 and AAV-6 variants (such as the AAV6 variant comprising the triply mutated AAV6 capsid Y731F/Y705F/T492V form disclosed in Rosario et al., 2016, Mol Ther Methods Clin Dev. 3, p.16026), -7, -8, -9, -10 such as cy10 and -rh10, -rh74, -dj, Anc80, LK03, AAV2i8, porcine AAV serotypes such as AAVpo4 and AAVpo6, and tyrosine, lysine and serine capsid mutants of the AAV serotypes, etc.. In addition, other non-natural engineered variants and chimeric AAV can also be useful.

AAV viruses may be engineered using conventional molecular biology techniques, making it possible to optimize these particles for cell specific delivery of nucleic acid sequences, for minimizing immunogenicity, for tuning stability and particle lifetime, for efficient degradation, for accurate delivery to the nucleus.

Desirable AAV fragments for assembly into vectors include the cap proteins, including the vp1, vp2, vp3 and hypervariable regions, the rep proteins, including rep 78, rep 68, rep 52, and rep 40, and the sequences encoding these proteins. These fragments may be readily utilized in a variety of vector systems and host cells.

AAV-based recombinant vectors lacking the Rep protein integrate with low efficacy into the host's genome and are mainly present as stable circular episomes that can persist for years in the target cells. Alternatively to using AAV natural serotypes, artificial AAV serotypes may be used in the context of the present invention, including, without limitation, AAV with a non-naturally occurring capsid protein. Such an artificial capsid may be generated by any suitable technique, using a selected AAV sequence (e.g., a fragment of a vp1 capsid protein) in combination with heterologous sequences which may be obtained from a different selected AAV serotype, non-contiguous portions of the same AAV serotype, from a non-AAV viral source, or from a non-viral source. An artificial AAV serotype may be, without limitation, a chimeric AAV capsid, a recombinant AAV capsid, or a "humanized" AAV capsid.

Accordingly, the present invention relates to an AAV vector comprising the nucleic acid molecule or construct of the invention. In the context of the present invention, the AAV vector comprises an AAV capsid able to transduce the target cells of interest, in particular hepatocytes. According to a particular embodiment, the AAV vector is of the AAV-1, -2, AAV-2 variants (such as the quadruple-mutant capsid optimized AAV-2 comprising an engineered capsid with Y44+500+730F+T491V changes, disclosed in Ling et al., 2016 Jul 18, Hum Gene Ther Methods. [Epub ahead of print]), -3 and AAV-3 variants (such as the AAV3-ST variant comprising an engineered AAV3 capsid with two amino acid changes, S663V+T492V, disclosed in Vercauteren et al., 2016, Mol. Ther. Vol. 24(6), p. 1042), -3B and AAV-3B variants, -4, -5, -6 and AAV-6 variants (such as the AAV6 variant comprising the triply mutated AAV6 capsid Y731F/Y705F/T492V form disclosed in Rosario et al., 2016, Mol Ther Methods Clin Dev. 3, p.16026), -7, -8, -9, -10 such as -cy10 and -rh10, -rh74, -dj, Anc80, LK03, AAV2i8, porcine AAV such as AAVpo4 and AAVpo6, and tyrosine, lysine and serine capsid mutants of a AAV serotypes, etc., serotype. In a particular embodiment, the AAV vector is of the AAV8, AAV9, AAVrh74 or AAV2i8 serotype (i.e. the AAV vector has a capsid of the AAV8, AAV9, AAVrh74 or AAV2i8 serotype). In a further particular embodiment, the AAV vector is a pseudotyped vector, i.e. its genome and capsid are derived from AAVs of different serotypes. For example, the pseudotyped AAV vector may be a vector whose genome is derived from one of the above mentioned AAV serotypes, and whose capsid is derived from another serotype. For example, the genome of the pseudotyped vector may have a capsid derived from the AAV8, AAV9, AAVrh74 or AAV2i8 serotype, and its genome may be derived from and different serotype. In a particular embodiment, the AAV vector has a capsid of the AAV8, AAV9 or AAVrh74 serotype, in particular of the AAV8 or AAV9 serotype, more particularly of the AAV8 serotype.

In a specific embodiment, wherein the vector is for use in delivering the transgene to muscle cells, the AAV vector may be selected, among others, in the group consisting of AAV8, AAV9 and AAVrh74. In another specific embodiment, wherein the vector is for use in delivering the transgene to liver cells, the AAV vector may be selected, among others, in the group consisting of AAV5, AAV8, AAV9, AAV-LK03, AAV-Anc80 and AAV3B.

In another embodiment, the capsid is a modified capsid. In the context of the present invention, a "modified capsid" may be a chimeric capsid or capsid comprising one or more variant VP capsid proteins derived from one or more wild-type AAV VP capsid proteins.

In a particular embodiment, the AAV vector is a chimeric vector, i.e. its capsid comprises VP capsid proteins derived from at least two different AAV serotypes, or comprises at least one chimeric VP protein combining VP protein regions or domains derived from at least two AAV serotypes. Examples of such chimeric AAV vectors useful to transduce liver cells are described in Shen et al., Molecular Therapy, 2007 and in Tenney et al., Virology, 2014. For example a chimeric AAV vector can derive from the combination of an AAV8 capsid sequence with a sequence of an AAV serotype different from the AAV8 serotype, such as any of those specifically mentioned above. In another embodiment, the capsid of the AAV vector comprises one or more variant VP capsid proteins such as those described in WO2015013313, in particular the RHM4-1, RHM15-1, RHM15-2, RHM15-3/RHM15-5, RHM15-4 and RHM15-6 capsid variants, which present a high liver tropism.

In another embodiment, the modified capsid can be derived also from capsid modifications inserted by error prone PCR and/or peptide insertion (e.g. as described in Bartel et al., 2011). In addition, capsid variants may include single amino acid changes such as tyrosine mutants (e.g. as described in Zhong et al., 2008)

In addition, the genome of the AAV vector may either be a single stranded or self-complementary double-stranded genome (McCarty et al., Gene Therapy, 2003). Self-complementary double-stranded AAV vectors are generated by deleting the terminal resolution site (trs) from one of the AAV terminal repeats. These modified vectors, whose replicating genome is half the length of the wild type AAV genome have the tendency to package DNA dimers. In a preferred embodiment, the AAV vector implemented in the practice of the present invention has a single stranded genome, and further preferably comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid.

In a particularly preferred embodiment, the invention relates to an AAV vector comprising, in a single-stranded or double-stranded, self-complementary genome (e.g. a single-stranded genome), the nucleic acid acid construct of the invention. In one embodiment, the AAV vector comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid. In a further particular embodiment, said nucleic acid is operably linked to a promoter, especially an ubiquitous or liver-specific promoter. According to a specific variant embodiment, the promoter is an ubiquitous promoter such as the cytomegalovirus enhancer/chicken beta actin (CAG) promoter, the cytomegalovirus enhancer/promoter (CMV), the PGK promoter and the SV40 early promoter. In a specific variant, the ubiquitous promoter is the CAG promoter. According to another variant, the promoter is a liver-specific promoter such as the alpha-1 antitrypsin promoter (hAAT), the transthyretin promoter, the albumin promoter and the thyroxine-binding globulin (TBG) promoter. In a specific variant, the liver-specific promoter is the hAAT liver-specific promoter of SEQ ID NO:15. In a further particular embodiment, the nucleic acid construct comprised into the genome of the AAV vector of the invention further comprises an intron as described above, such as an intron placed between the promoter and the nucleic acid sequence encoding the GAA coding sequence (i.e. the optimized GAA coding sequence of the invention, the chimeric GAA coding sequence of the invention, or the chimeric and optimized GAA coding sequence of the invention). Representative introns that may be included within the nucleic acid construct introduced within the AAV vector genome include, without limitation, the human beta globin b2 (or HBB2) intron, the FIX intron and the chicken beta-globin intron. Said intron within the genome of the AAV vector may be a classical (or unmodified) intron or a modified intron designed to decrease the number of, or even totally remove, alternative open reading frames (ARFs) within said intron. Modified and unmodified introns that may be used in the practice of this embodiment where the nucleic acid of the invention is introduced within an AAV vector are thoroughly described above. In a particular embodiment, the AAV vector, in particular an AAV vector comprising an AAV8, AAV9, AAVrh74 or AAV2i8 capsid, in particular an AAV8, AAV9 or AAVrh74 capsid, such as an AAV8 or AAV9 capsid, more particularly an AAV8 capsid, of the invention includes within its genome a modified (or optimized) intron such as the modified HBB2 intron of SEQ ID NO:7, the modified FIX intron of SEQ ID NO:9 and the modified chicken beta-globin intron of SEQ ID NO:11. In a further particular embodiment, the vector of the invention is an AAV vector comprising comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid, comprising a genome containing, in the 5' to 3' orientation: an AAV 5'-ITR (such as an AAV2 5'-ITR); an ApoE control region; the hAAT-liver specific promoter; a HBB2 intron (in particular a modified HBB2 intron as defined above); the GAA coding sequence of the invention; the bovine growth hormone polyadenylation signal; and an AAV 3'-ITR (such as an AAV2 3'-ITR), such as a genome comprising a the nucleic acid shown in SEQ ID NO:20, 21 or 22 (including the nucleic acid sequence shown in SEQ ID NO:17, 18 and 19, respectively, corresponding to an optimized sequence encoding a Δ8 truncated form of GAA derived from the parent hGAA of SEQ ID NO:5) flanked by an AAV 5'-ITR (such as an AAV2 5'-ITR) and an AAV 3'-ITR (such as an AAV2 3'-ITR). Other expression cassette useful in the practice of the present invention comprise those signal peptide moiety and GAA moiety in any one of the sequence combinations shown in table 2 above.

In a particular embodiment of the invention, the nucleic acid construct of the invention comprises a liver-specific promoter as described above, and the vector is a viral vector capable of transducing liver tissue or cells as described above. The inventors present below data showing that the protolerogenic and metabolic properties of the liver are advantageously implemented thanks to this embodiment to develop highly efficient and optimized vectors to express secretable forms of GAA in hepatocytes and to induce immune tolerance to the protein.

In addition, in a further particular embodiment, the invention provides the combination of two vectors, such as two viral vectors, in particular two AAV vectors, for improving gene delivery and treatment efficacy in the cells of interest. For example, the two vectors may carry the nucleic acid molecule of the invention coding for the GAA protein of the invention, under the control of one different promoter in each of these two vectors. In a particular embodiment, one vector comprises a promoter which is a liver-specific promoter (as one of those described above), and the other vector comprises a promoter which is specific of another tissue of interest for the treatment of a glycogen storage disorder, such as a muscle-specific promoter, for example the desmin promoter. In a particular variant of this embodiment, this combination of vectors corresponds to multiple co-packaged AAV vectors produced as described in WO2015196179.

In another aspect, the invention provides a chimeric GAA polypeptide, comprising a signal peptide moiety and a GAA moiety, wherein the naturally occurring GAA signal peptide is replaced with a signal peptide selected in the group consisting of SEQ ID NO:2 to 4. In a particular embodiment, the chimeric GAA polypeptide of the invention may be a polypeptide derived from a truncated form of GAA, as described above. For example, the chimeric GAA protein of the invention may a Δ1, Δ2, Δ3, Δ4, Δ5, Δ6, Δ7, Δ8, Δ9, Δ10, Δ11, Δ12, Δ13, Δ14, Δ15, Δ16, Δ17, Δ18, Δ19, Δ20, Δ21, Δ22, Δ23, Δ24, Δ25, Δ26, Δ27, Δ28, Δ29, Δ30, Δ31, Δ32, Δ33, Δ34, Δ35, Δ36, Δ37, Δ38, Δ39, Δ40, Δ41, Δ42, Δ43, Δ44, Δ45, Δ46, Δ47, Δ48, Δ49, Δ50, Δ51, Δ52, Δ53, Δ54, Δ55, Δ56, Δ57, Δ58, Δ59, Δ60, Δ61, Δ62, Δ63, Δ64, Δ65, Δ66, Δ67, Δ68, Δ69, Δ70, Δ71, Δ72, Δ73, Δ74 or Δ75 GAA truncated form moiety (in particular a truncated form of the parent hGAA protein shown in SEQ ID NO: 5 or SEQ ID NO:36, in particular in SEQ ID NO:5), fused at its N-terminal end to a signal peptide selected in the group consisting of SEQ ID NO:2 to 4. In a particular embodiment, the GAA moiety of the chimeric GAA protein is a Δ6, Δ7, Δ8, A9 or Δ10 truncated form of GAA (in particular of the parent hGAA protein shown in SEQ ID NO: 5 or SEQ ID NO:36, in particular in SEQ ID NO:5), in particular a Δ8 truncated form of GAA (in particular of the parent hGAA protein shown in SEQ ID NO: 5 or SEQ ID NO:36, in particular in SEQ ID NO:5). In another particular embodiment, the GAA moiety of the chimeric GAA protein is a Δ40, Δ41, Δ42, Δ43 or Δ44 truncated form of GAA (in particular of the parent hGAA protein shown in SEQ ID NO: 5 or SEQ ID NO:36, in particular in SEQ ID NO:5), in particular a Δ42 truncated form of GAA (in particular of the parent hGAA protein shown in SEQ ID NO: 5 or SEQ ID NO:36, in particular in SEQ ID NO:5). In a particular embodiment, the chimeric GAA polypeptide has the sequence resulting from one of the combination shown in table 1 above, or is a functional derivative thereof having at least 90% identity, in particular at least 95%, at least 96%, at least 97%, at least 98%, or at least 99% identity to the resulting sequence combination.

The invention also relates to a cell, for example a liver cell, that is transformed with a nucleic acid molecule or construct of the invention as is the case for *ex vivo* gene therapy. Cells of the invention may be delivered to the subject in need thereof, such as GAA-deficient patient, by any appropriate administration route such as via injection in the liver or in the bloodstream of said subject. In a particular embodiment, the invention involves introducing the nucleic acid of the invention into liver cells, in particular into liver cells of the subject to be treated, and administering said transformed liver cells into which the nucleic acid has been introduced to the subject. Advantageously, this embodiment is useful for secreting GAA from said cells. In a particular embodiment, the liver cells are liver cells from the patient to be treated, or are liver stem cells that are further transformed, and differentiated *in vitro* into liver cells, for subsequent administration to the patient.

The present invention further relates to a transgenic, nonhuman animal comprising in its genome the nucleic acid molecule or construct encoding a GAA protein according to the invention. In a particular embodiment, the animal is a mouse.

Apart from the specific delivery systems embodied below in the examples, various delivery systems are known and can be used to administer the nucleic acid molecule or construct of the invention, e.g., encapsulation in liposomes, microparticles, microcapsules, recombinant cells capable of expressing the coding sequence of the invention, receptor-mediated endocytosis, construction of a therapeutic nucleic acid as part of a retroviral or other vector, etc.

According to an embodiment, it may be desirable to introduce the chimeric GAA polypeptide, nucleic acid molecule, nucleic acid construct or cell of the invention into the liver of the subject by any suitable route. In addition naked DNA such as minicircles and transposons can be used for delivery or lentiviral vectors. Additionally, gene editing technologies such as zinc finger nucleases, meganucleases, TALENs, and CRISPR can also be used to deliver the coding sequence of the invention.

The present invention also provides pharmaceutical compositions comprising the nucleic acid molecule, the nucleic acid construct, the vector, the chimeric GAA polypeptide, or the cell of the invention. Such compositions comprise a therapeutically effective amount of the therapeutic (the nucleic acid molecule, the nucleic acid construct, the vector, the chimeric GAA polypeptide or the cell of the invention), and a pharmaceutically acceptable carrier. In a specific embodiment, the term "pharmaceutically acceptable" means approved by a regulatory agency of the Federal or a state government or listed in the U.S. or European Pharmacopeia or other generally recognized pharmacopeia for use in animals, and humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsions, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the subject. In a particular embodiment, the nucleic acid, vector or cell of the invention is formulated in a composition comprising phosphate-buffered saline and supplemented with 0.25% human serum albumin. In another particular embodiment, the nucleic acid, vector or cell of the invention is formulated in a composition comprising ringer lactate and a non-ionic surfactant, such as pluronic F68 at a final concentration of 0.01-0.0001%, such as at a concentration of 0.001%, by weight of the total composition. The formulation may further comprise serum albumin, in particular human serum albumin, such as human serum albumin at 0.25%. Other appropriate formulations for either storage or administration are known in the art, in particular from WO 2005/118792 or Allay et al., 2011.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection.

In an embodiment, the nucleic acid molecule, the nucleic acid construct, the vector, the chimeric GAA polypeptide or the cell of the invention can be delivered in a vesicle, in particular a liposome. In yet another embodiment, the nucleic acid molecule, the nucleic acid construct, the vector, the chimeric GAA polypeptide or the cell of the invention can be delivered in a controlled release system.

Methods of administration of the nucleic acid molecule, the nucleic acid construct, the vector, the chimeric GAA polypeptide or the cell of the invention include but are not limited to intradermal, intramuscular, intraperitoneal, intravenous, subcutaneous, intranasal, epidural, and oral routes. In a particular embodiment, the administration is via the intravenous or intramuscular route. The nucleic acid molecule, the nucleic acid construct, the vector, the chimeric GAA polypeptide or the cell of the invention, whether vectorized or not, may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local.

In a specific embodiment, it may be desirable to administer the pharmaceutical compositions of the invention locally to the area in need of treatment, e.g. the liver. This may be achieved, for example, by means of an implant, said implant being of a porous, nonporous, or gelatinous material, including membranes, such as sialastic membranes, or fibers.

The amount of the therapeutic (i.e. the nucleic acid molecule, the nucleic acid construct, the vector, the chimeric GAA polypeptide or the cell of the invention) of the invention which will be effective in the treatment of a glycogen storage disease can be determined by standard clinical techniques. In addition, in vivo and/or in vitro assays may optionally be employed to help predict optimal dosage ranges. The precise dose to be employed in the formulation will also depend on the route of administration, and the seriousness of the disease, and should be decided according to the judgment of the practitioner and each patient's circumstances. The dosage of the nucleic acid molecule, the nucleic acid construct, the vector, the chimeric GAA polypeptide or the cell of the invention administered to the subject in need thereof will vary based on several factors including, without limitation, the route of administration, the specific disease treated, the subject's age or the level of expression necessary to obtain the therapeutic effect. One skilled in the art can readily determine, based on its knowledge in this field, the dosage range required based on these factors and others. In case of a treatment comprising administering a viral vector, such as an AAV vector, to the subject, typical doses of the vector are of at least 1x10⁸ vector genomes per kilogram body weight (vg/kg), such as at least 1x10⁹ vg/kg, at least 1x10⁹ vg/kg, at least 1x10¹¹ vg/kg, at least 1x10⁹ vg/kg at least 1x10¹³ vg/kg, or at least 1x10¹⁴ vg/kg.

The invention also relates to a method for treating a glycogen storage disease, which comprises a step of delivering a therapeutic effective amount of the nucleic acid, the vector, the chimeric polypeptide, the pharmaceutical composition or the cell of the invention to a subject in need thereof.

The invention also relates to a method for treating a glycogen storage disease, said method inducing no immune response to the transgene (i.e. to the chimeric GAA polypeptide of the invention), or inducing a reduced immune response to the transgene, comprising a step of delivering a therapeutic effective amount of the nucleic acid molecule, nucleic acid construct, vector, pharmaceutical composition or cell of the invention to a subject in need thereof. The invention also relates to a method for treating a glycogen storage disease, said method comprising repeated administration of a therapeutic effective amount of the nucleic acid molecule, nucleic acid construct, vector, pharmaceutical composition or cell of the invention to a subject in need thereof. In this aspect, the nucleic acid molecule or the nucleic acid construct of the invention comprises a promoter which is functional in liver cells, thereby allowing immune tolerance to the expressed chimeric GAA polypeptide produced therefrom. As well, in this aspect, the pharmaceutical composition used in this aspect comprises a nucleic acid molecule or nucleic acid construct comprising a promoter which is functional in liver cells. In case of delivery of liver cells, said cells may be cells previously collected from the subject in need of the treatment and that were engineered by introducing therein the nucleic acid molecule or the nucleic acid construct of the invention to thereby make them able to produce the chimeric GAA polypeptide of the invention. According to an embodiment, in the aspect comprising a repeated administration, said administration may be repeated at least once or more, and may even be considered to be done according to a periodic schedule, such as once per week, per month or per year. The periodic schedule may also comprise an administration once every 2, 3, 4, 5, 6, 7, 8, 9 or 10 year, or more than 10 years. In another particular embodiment, administration of each administration of a viral vector of the invention is done using a different virus for each successive administration, thereby avoiding a reduction of efficacy because of a possible immune response against a previously administered viral vector. For example, a first administration of a viral vector comprising an AAV8 capsid may be done, followed by the administration of a vector comprising an AAV9 capsid, or even by the administration of a virus unrelated to AAVs, such as a retroviral or lentiviral vector.

According to the present invention, a treatment may include curative, alleviation or prophylactic effects. Accordingly, therapeutic and prophylactic treatment includes amelioration of the symptoms of a particular glycogen storage disease or preventing or otherwise reducing the risk of developing a particular glycogen storage disease. The term "prophylactic" may be considered as reducing the severity or the onset of a particular condition. "Prophylactic" also includes preventing reoccurrence of a particular condition in a patient previously diagnosed with the condition. "Therapeutic" may also reduce the severity of an existing condition. The term 'treatment' is used herein to refer to any regimen that can benefit an animal, in particular a mammal, more particularly a human subject.

The invention also relates to an *ex vivo* gene therapy method for the treatment of a glycogen storage disease, comprising introducing the nucleic acid molecule or the nucleic acid construct of the invention into an isolated cell of a patient in need thereof, for example an isolated hematopoietic stem cell, and introducing said cell into said patient in need thereof. In a particular embodiment of this aspect, the nucleic acid molecule or construct is introduced into the cell with a vector as defined above. In a particular embodiment, the vector is an integrative viral vector. In a further particular embodiment, the viral vector is a retroviral vector, such as a lenviral vector. For example, a lentiviral vector as disclosed in van Til et al., 2010, Blood, 115(26), p. 5329, may be used in the practice in the method of the present invention.

The invention also relates to the nucleic acid molecule, the nucleic acid construct, the vector, the chimeric GAA polypeptide or the cell of the invention for use as a medicament.

The invention also relates to the nucleic acid molecule, the nucleic acid construct, the vector, the chimeric GAA polypeptide or the cell of the invention, for use in a method for treating a disease caused by a mutation in the GAA gene, in particular in a method for treating Pompe disease. The invention further relates to the nucleic acid molecule, the nucleic acid construct, the vector, the chimeric GAA polypeptide or the cell of the invention, for use in a method for treating a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII. The chimeric GAA polypeptide of the invention may be administered to a patient in need thereof, for use in enzyme replacement therapy (ERT), such as for use in enzyme replacement therapy of one of a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII.

The invention further relates to the use of the nucleic acid molecule, the nucleic acid construct, the vector, the chimeric GAA polypeptide or the cell of the invention, in the manufacture of a medicament useful for treating a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII.

### EXAMPLES

The invention is further described in detail by reference to the following experimental examples and the attached figures. These examples are provided for purposes of illustration only, and are not intended to be limiting.

### MATERIAL AND METHODS

### GAA activity

GAA activity was measured following homogenization of frozen tissue samples in distilled water. 50-100 mg of tissue were weighed and homogenized, then centrifuged for 20 minutes at 10000 x g. The reaction was set up with 10 µl of supernatant and 20 µl of substrate - 4MUα-D-glucoside, in a 96 wells plate. The reaction mixture was incubated at 37°C for one hour, and then stopped by adding 150 µl of Sodium Carbonate buffer pH 10.5. A standard curve (0-2500 pmol/µl of 4MU) was used to measure released fluorescent 4MU from individual reaction mixture, using the EnSpire alpha plate reader (Perkin-Elmer) at 449 nm (Emission) and 360 nm (Excitation). The protein concentration of the clarified supernatant was quantified by BCA (Thermo Fisher Scientific). To calculate the GAA activity, released 4MU concentration was divided by the sample protein concentration and activity was reported as nmol/hour/mg protein.

### Glycogen content

Glycogen content was measured indirectly as the glucose released after total digestion by *Aspergillus Niger* amyloglucosidase of the tissue homogenates obtained as described above. The reaction was set in a 96-well plate up with 20 µl of tissue homogenate and 55 µl of distilled water. Samples were incubated for 5 min at 95°C and then cooled at 4°C. 25 µl of amyloglucosidase (diluted 1:50 in 0.1M potassium acetate pH5.5) were added to each sample. A control reaction without amyloglucosidase was also set up for each sample. Both sample and control reaction were incubated at 37°C for 90 minutes. The reaction was stopped by incubating samples for 5 min at 95°C. The glucose released was determined using the Glucose assay kit (Sigma-Aldrich) by measuring the absorbance using the EnSpire alpha plate reader (Perkin-Elmer) at 540 nm

### Plethysmography

A flow-through (0.5 L/min) plethysmograph (EMKA technologies) was used to measure the pattern of breathing in control and Gaa-/- mice. A clear Plexiglas chamber was calibrated with known airflow and pressure signals before data collection. Signals were analyzed by using the IOX2 software (EMKA technologies). The following variables were measured: breathing frequency, tidal volume and minute ventilation. Ventilation data were collected in 5-min bins. Five minutes were allowed for acclimation to the chamber. During both acclimation and data acquirement, mice were breathing normoxic air (21% 02, 79% N2).

### Mouse studies

Gaa -/- mouse was generated by targeted disruption of exon 6 and is maintained on the C57BL/6J/129X1/SvJ background (Raben N. et al 1998). Vectors were delivered via the tail vein in a volume of 0.2 ml. Serum samples were collected monthly to monitor levels of secreted hGAA. PBS-injected affected animals and wild type littermates were used as controls.

### Anti-hGAA antibody determination

Maxisorp 96 wells plates (Thermo Fisher Scientific) were coated with Myozime® protein in carbonate buffer at 4°C overnight. A standard curve of rat recombinant IgG (Sigma Aldrich) was coated to the wells in seven 2-fold dilution starting from 1 µg/ml. After blocking, plasma samples were added to plates and incubated 1 hr at 37C. Detection was performed by adding to the wells 3,3',5,5'-tetramethylbenzidine substrate (BD Biosciences), and color development was measured at 450 and 570 nm (for background subtraction) on an Enspire plate reader (Perkin Elmer) after blocking the reaction with H2SO4.

### NHP study

Male Cynomolgus macaques were housed in stainless steel cages and maintained on a 12-hour light/dark cycle. All macaques had neutralizing antibody titers of <1:5 before the start of the study. A dose of 2E12 vg/kg of AAV8-hAAT-sp7-Δ8-hGAAco1 was infused via the saphenous vein. Blood samples were taken 12 days before and 30 days after the injection via the femoral vein. Whole blood was collected in EDTA containing tubes and centrifuged to separate serum. Three months after vector administration all macaques were euthanized. The animals were first anesthetized with a mixture of ketamine/dexmedetomidine and then euthanized using sodium pentobarbital injected IV. Tissues were immediately collected and frozen in liquid nitrogen.

### Western blot analysis

Total homogenates were obtained from frozen muscles. Protein concentration was determined in the extracts by Pierce BCA Protein Assay (Thermo Fisher Scientific), following manufacturer's instructions. Western blot was performed with an anti hGAA antibody (Abcam). Anti-tubulin antibody (Sigma Aldrich) was used as loading controls.

### RESULTS

In an effort to improve current gene replacement therapies for Pompe disease, we engineered the hGAA sequence to increase its secretion by exchanging wild-type signal peptide (indicated here as sp1) with different signal peptides (sp2 to 8, described in table 4) in the sequence optimized sequence of hGAA (SEQ ID NO:13).

**Table 4**

| Signal peptide | DNA sequence | Aminoacid sequence | Sequence optimized |
|---|---|---|---|
| sp1 | atgggagtgcggcaccctccatgtagccacagactgctggcc gtgtgtgccctggtgtctctggctacagctgccctgctg | mgvrhppcshrllavcalvslataall | YES |
| sp2 | atgcctagctctgtgtcctggggcattctgctgctggccggcc tgtgttgtctggtgcctgtgtctctggcc | mpssvswgilllaglcclvpvsla | YES |
| sp3 | atgctgctgctgtctgcactgctgctgggcctggcctttggcta ctct | mlllsalllglafgys | YES |
| sp4 | atgctgctgagctttgccctgctgctgggactggccctgggct actct | mllsfalllglalgys | YES |
| sp5 | atgctgctggaacatgccctgctgctgggactggcccacggc tattct | mllehalllglahgys | YES |
| sp6 | atgcctccacctagaacaggcagaggcctgctgtggctgggc ctggtgctgtctagtgtgtgtgtggccctgggc | mppprtgrgllwlglvlssvcvalg | NO |
| sp7 | atggcctttctgtggctgctgagctgttgggccctgctgggca ccaccttcggc | maflwllscwallgttfg | YES |
| sp8 | atggccagcagactgaccctgctgacactccttctgctgctgc tggccggcgatagagccagcagc | masrltlltllllllagdrass | YES |

We transfected hepatoma cells (Huh-7) with plasmids expressing GFP or wild-type hGAA (hGAA; SEQ ID NO:37) in parallel with plasmids expressing codon optimized hGAA (hGAAco) fused with signal peptides 1 to 8. 48 hours after transfection the growth medium has been analyzed for the presence of hGAA. Notably only four of the constructs bearing efficient signal peptide led to the secretion of hGAA levels significantly higher than what observed in the negative control represented by GFP-transfected cells (Figure 1A). Constructs expressing the hGAA chimeric protein carrying the signal peptides sp2, sp6, sp7, and sp8 secreted higher levels of hGAA in medium (p<0.05 vs. GFP). We then packaged these constructs in AAV8 vectors produced by triple transfection and cesium chloride purification and we injected them in wild-type C57BL/6J mice. We then compared in vivo GAA serum levels across constructs in which the signal peptides sp1, 2, 3, 7 and 8 (figure 1B) were used. One month after the injection of 1E12 vg/kg of AAV8 vectors expressing hGAAco we observed a significantly higher level of circulating hGAA compared to PBS injected mice. Interestingly, the level of circulating hGAA was significantly higher in mice treated with vectors expressing hGAAco fused with sp2, 7, and 8. Surprisingly, secretion levels achieved with sp2 construct in vivo were significantly lower than those measured with sp7- and sp8- engineered hGAA (Figure 1B). Taken together these data indicate that the substitution of wild-type signal peptide with signal peptides deriving from a protein efficiently secreted in the liver is an effective strategy to increase circulating level of hGAA in vivo. Moreover, the unexpected results obtained in vivo with sp7 and 8 signal peptides indicate that not all signal peptides are equally efficient in vivo, and that signal peptides sp7 and sp8 drive superior efficacy of secretion in vivo compared with sp1 and sp2.

Those findings were then confirmed in an animal model of the disease, GAA -/- mice. This mouse model presents no residual activity of the enzyme in muscle, together with glycogen accumulation in different organs, resulting in muscular strength impairment and reduced lifespan.

To compare the effectiveness of the different vectors in the rescue of the Pompe disease phenotype in GAA-/- mice, we followed long-term the effects of the injection of 2E12 vg/kg of vectors expressing hGAAco and engineered version fused with signal peptides sp2, 7, and 8. Three months after the injections, we observed significantly increased circulating hGAA after the injection with AAV8 expressing hGAAco bearing the highly efficient signal peptides sp2, 7, and 8 (Figure 2A). Notably, hGAAco fused with sp7 signal peptide leaded to levels of hGAA in circulation significantly higher than those observed for the other two constructs. The long term follow-up in this experiment permitted us to estimate the survival of GAA -/- mice. Mice were injected at 4 months of age and then followed for six months. During this period 8/10 GAA-/- mice died in the PBS injected group whereas just 1/45 death was reported in GAA-/- animals treated with hGAAco expressing constructs and in wild-type animals. The statistical significance of this finding (figure 2B) indicates that the treatment with all hGAAco expressing vectors, independently of the secretion level, rescues the lethal phenotype observed in GAA -/- mice. Another phenotype reported for this mouse model is a decreased respiratory function. In particular, a decreased tidal volume has been reported (DeRuisseau et al PNAS 2008) and it has been demonstrated that the decrease is due to the accumulation of glycogen in the nervous system. The rescue of glycogen level in the nervous system depends on the ability of the hGAA to cross the blood-brain barrier and it has been demonstrated in other lysosomal storage disorders (Polito et al Hum. Mol. Genet. 2010, Cho et al Orph. J. of Rare Dis. 2015) that this is directly dependent from the circulating levels of the protein. We therefore evaluated the effect of long-term, high circulating level of hGAA on the tidal volume of GAA -/- mice. Three months after the injections, GAA -/- mice shown a decreased, although not significantly (p = 0.104), tidal volume whereas mice treated with sp7 shown a tidal volume very similar to those observed in WT mice (p = 0.974) (figure 2C, left). Six months after the injections, only two GAA -/- mice survived and they appear to have a less severe respiratory phenotype. Again, mice treated with sp7 hGAAco had a tidal volume similar to that observed in WT animals (p= 0.969) (figure 2C, right). Importantly, a statistically significant difference between the tidal volume measured in mice treated with sp1 and sp7 hGAAco (p = 0.041) was noted, showing a more marked improvement in sp7-GAA treated mice. Taken together these data indicate that liver transduction with an AAV8 expressing hGAAco fused with sp7 signal peptide results in superior level of hGAA in the blood with a concomitant complete phenotypical correction of respiratory function in GAA-/- mice.

We then verified if the high level of circulating hGAA rescued the glycogen accumulation in skeletal muscle. We measured hGAA activity in the quadriceps of mice injected as described above. Injection of hGAA expressing vectors leaded to an increase in hGAA activity in quadriceps to levels comparable to those observed in WT animals (figure 3A). Measurement of glycogen in quadriceps indicate that GAA -/- mice accumulate ∼ 20-fold more glycogen than WT animals (p = 3.5E-6). This accumulation is reversed by the treatment with hGAA expressing vector (p<0.05 vs GAA-/-), with the sp7 that shown the lowest glycogen levels, undistinguishable from the levels of wild type animals (p = 0.898 vs. WT) (Figure 3B).

To verify that the fusion of hGAA with an efficient signal peptide improve its secretion and increase the phenotypical correction of the disease in vivo, we injected GAA -/- mice a low vector dose, and we evaluated the biochemical correction of the phenotype. Three months after the injection of 6E11 vg/kg of vectors expressing hGAAco fused with signal peptide 1, 7, and 8, we measured circulating hGAA. Notably sp 7 and 8 leaded to a three-fold increase in the secreted hGAA detectable in serum compared to PBS-treated mice (Figure 4A). We further investigated the therapeutic effects of AAV8 vectors expressing hGAAco by performing biochemical analysis of tissues from treated animals and controls. We evaluated the glycogen content in heart, diaphragm, and quadriceps of GAA -/- mice treated as described above. Notably, we observed high levels of hGAA in tissues after treatment with hGAAco expressing vectors (data not shown) that correlated with a significant reduction in glycogen content in all the tissues considered (figure 4B-D). In particular, in the heart (figure 4B) the level of glycogen measured after treatment with vectors bearing the highly efficient signal peptides sp7 and 8 were undistinguishable from those observed in non-affected wild-type animals (p = 0.983 and 0.996 vs. WT respectively). Importantly, the level observed after treatment with both the sp7 and sp8 vectors were significantly reduced compared with GAA -/- animals PBS-injected or treated with wild-type hGAAco expressing vector (noted as sp1). We also tested if the liver transduction with our vectors induced a humoral response against the transgene. Mice were injected intravenously with AAV8 vectors expressing hGAAco1 or hGAAco2 fused with sp7 or sp8, under the transcriptional control of a liver specific promoter. Mice injected intramuscularly with an AAV9 expressing hGAAco under the transcriptional control of a constitutive promoter (CAG, chicken beta actin promoter and cytomegalovirus enhancer) showed very high levels of total IgG specific to the hGAA transgene, whereas vectors expressing the same protein in the liver showed lower levels of humoral responses to the hGAA transgene. Interestingly, 3/5 mice injected with sp1 hGAAco (wild type signal peptide) expressing vector developed low but detectable levels of antibodies. Mice injected with the highly secreted vector sp7 had significant decreased levels of anti-hGAA IgG levels. These data indicate that the expression of a transgene in the liver is fundamental for the induction of peripheral tolerance, also they provide indications that highly secretable hGAA transgenes are less immunogenic than their wild type counterpart.

The best performing vector selected in the mouse study was injected in two non-human primates (NHP, Macaca Fascicularis sp.) to verify the efficacy of secretion of our vector and the uptake in muscles. We injected two monkeys with 2E12 vg/kg of AAV8-hAAT-sp7-Δ8-hGAAco1. One month after the injection we measured the levels of hGAA in the serum of the two animals by western blot using a specific anti-hGAA antibody. We observed a clear band with a size compatible with that of hGAA in the two monkeys. This band was not present in serum samples obtained 12 days before vector injection, thus confirming the specificity of our detection method (Figure 6A). Three months after the injection we sacrificed the animals and we obtained tissues to verify if hGAA secreted from the liver in the bloodstream were efficiently taken up by muscle. We performed a western blot using an antibody specific for hGAA on total lysates obtained from biceps and diaphragm of the two monkeys. Interestingly we were able to observe a clear band in animal number 2 which also showed the highest levels of hGAA in the bloodstream (Figure 6B). Also, in animal number 1 we could observe a fainter band with a molecular weight consistent with that of hGAA in both muscles analyzed. These data indicate that the AAV8-hAAT-sp7-Δ8-hGAAco1 vector efficiently transduces liver in NHP. They also demonstrate that the protein secreted in the bloodstream is efficiently taken up in muscle and that this uptake is correlated with the level of hGAA measured in blood.

## Claims

1. A nucleic acid molecule encoding a functional chimeric GAA protein, comprising a signal peptide moiety and a functional GAA moiety, in particular a GAA moiety corresponding to a truncated form of a parent GAA, wherein the signal peptide moiety has an amino acid sequence selected in the group consisting of SEQ ID NO:2 to 4, preferably SEQ ID NO:2.

2. The nucleic acid molecule according to claim 1, wherein said GAA moiety has 1 to 75 consecutive amino acids truncated at its N-terminal end as compared to a parent GAA polypeptide, wherein said GAA moiety has in particular 6, 7, 8, 9, 10, 40, 41, 42, 43 or 44 consecutive amino acids truncated at its N-terminal end as compared to a parent GAA polypeptide, in particular 8 or 42 consecutive amino acids truncated at its N-terminal end as compared to a parent GAA polypeptide.

3. The nucleic acid molecule according to any one of claims 1 to 2, wherein said GAA moiety has 8 consecutive amino acids truncated at its N-terminal end as compared to a parent GAA.

4. The nucleic acid molecule according to any one of claims 1 to 3, wherein said parent GAA is the human GAA polypeptide shown in SEQ ID NO:5 or SEQ ID NO:36, in particular SEQ ID NO:5.

5. The nucleic acid molecule according to any one of claims 1 to 4, which is a nucleotide sequence optimized to improve the expression of and/or improve immune tolerance to the chimeric GAA *in vivo,* in particular the nucleotide sequence shown in SEQ ID NO:13 or 14.

6. The nucleic acid molecule according to any one of claims 1 to 5, comprising a nucleotide sequence resulting from the combination of the following sequences:
| Signal peptide moiety coding sequence | GAA moiety coding sequence |
|---|---|
| SEQ ID NO:26 | |
| SEQ ID NO:27 | SEQ ID NO:31 |
| SEQ ID NO:28 | |
| SEQ ID NO:26 | |
| SEQ ID NO:27 | SEQ ID NO:13 |
| SEQ ID NO:28 | |
| SEQ ID NO:26 | |
| SEQ ID NO:27 | SEQ ID NO:14 |
| SEQ ID NO:28 | |
| SEQ ID NO:26 | |
| SEQ ID NO:27 | SEQ ID NO:32 |
| SEQ ID NO:28 | |
| SEQ ID NO:26 | |
| SEQ ID NO:27 | SEQ ID NO:33 |
| SEQ ID NO:28 | |
| SEQ ID NO:26 | |
| SEQ ID NO:27 | SEQ ID NO:34 |
| SEQ ID NO:28 | |
| SEQ ID NO:26 | |
| SEQ ID NO:27 | SEQ ID NO:35 |
| SEQ ID NO:28 | |

7. A nucleic acid construct, comprising the nucleic acid molecule according to any one of claims 1 to 6, which is an expression cassette comprising said nucleic acid molecule operably linked to a promoter, such as a liver-specific promoter preferably selected in the group consisting of the alpha-1 antitrypsin promoter (hAAT), the transthyretin promoter, the albumin promoter and the thyroxine-binding globulin (TBG) promoter, wherein said nucleic acid construct optionally further comprises an intron, in particular an intron selected in the group consisting of a human beta globin b2 (or HBB2) intron, a FIX intron, a chicken beta-globin intron and a SV40 intron, wherein said intron is optionally a modified intron such as a modified HBB2 intron of SEQ ID NO:7, a modified FIX intron of SEQ ID NO:9, or a modified chicken beta-globin intron of SEQ ID NO:11.

8. The nucleic acid construct according to claim 7, comprising, preferably in this order,: an enhancer; an intron; a promoter, in particular a liver-specific promoter; the nucleic acid sequence encoding the chimeric GAA polypeptide; and a polyadenylation signal, the construct comprising preferably, in this order: an ApoE control region; a HBB2 intron, in particular a modified HBB2 intron; a hAAT promoter; the nucleic acid sequence encoding the chimeric GAA polypeptide; and a bovine growth hormone polyadenylation signal, said nucleic acid construct more particularly comprising the nucleotide sequence of SEQ ID NO:20, 21 or 22.

9. A vector comprising the nucleic acid molecule or the nucleic acid construct according to any one of claims 1 to 8, which is a viral vector, preferably a retroviral vector, such as a lentiviral vector, or an AAV vector, such as a single-stranded or double-stranded self-complementary AAV vector, preferably an AAV vector with an AAV-derived capsid, such as an AAV1, AAV2, variant AAV2, AAV3, variant AAV3, AAV3B, variant AAV3B, AAV4, AAV5, AAV6, variant AAV6, AAV7, AAV8, AAV9, AAV10 such as AAVcy10 and AAVrh10, AAVrh74, AAVdj, AAV-Anc80, AAV-LK03, AAV2i8, and porcine AAV, such as AAVpo4 and AAVpo6 capsid or with a chimeric capsid, wherein the AAV vector has more particularly an AAV8, AAV9, AAVrh74 or AAV2i8 capsid, in particular an AAV8, AAV9 or AAVrh74 capsid, more particularly an AAV8 capsid.

10. A cell transformed with the nucleic acid molecule of any one of claims 1 to 6, the nucleic acid construct of any one of claims 7 to 8 or the vector of claim 9, wherein the cell is in particular a liver cell or a muscle cell.

11. A chimeric GAA polypeptide, comprising a signal peptide moiety and a functional GAA moiety, wherein the signal peptide moiety is selected in the group consisting of SEQ ID NO:2 to 4, wherein in particular the GAA moiety is a truncated form of a parent GAA polypeptide, such as a GAA moiety having 1 to 75 consecutive amino acids deleted at its N-terminal end as compared to a parent GAA polypeptide, in particular 6, 7, 8, 9, 10, 20, 41, 42, 43 or 44 consecutive amino acids deleted at its N-terminal end as compared to a parent GAA polypeptide, such as 8 or 42 consecutive amino acids deleted at its N-terminal end as compared to a parent GAA polypeptide, wherein the GAA moiety is in particular a truncated form of the human GAA protein of SEQ ID NO:5 or SEQ ID NO:36, in particular of SEQ ID NO:5.

12. The chimeric GAA polypeptide according to claim 11, wherein the GAA moiety has 8 consecutive amino acids truncated at its N-terminal end as compared to a parent GAA polypeptide, in particular as compared to the human GAA protein of SEQ ID NO:5 or SEQ ID NO:36, in particular of SEQ ID NO:5.

13. The chimeric GAA polypeptide according to claim 11 or 12, comprising an amino acid sequence resulting from the combination of the following sequences:
| Signal peptide moiety | GAA moiety |
|---|---|
| SEQ ID NO:2 | SEQ ID NO:5 or SEQ ID NO:36, in |
| SEQ ID NO:3 | particular SEQ ID NO:5 |
| SEQ ID NO:4 | |
| SEQ ID NO:2 | SEQ ID NO:29 |
| SEQ ID NO:3 | |
| SEQ ID NO:4 | |
| SEQ ID NO:2 | SEQ ID NO:30 |
| SEQ ID NO:3 | |
| SEQ ID NO:4 | |

14. A pharmaceutical composition, comprising, in a pharmaceutically acceptable carrier, the nucleic acid sequence of any one of claims 1 to 6, the nucleic acid construct of any one of claims 7 to 8, the vector of claim 9, the cell according to claim 10, or the chimeric polypeptide according to any one of claims 11 to 13.

15. The nucleic acid sequence of any one of claims 1 to 6, the nucleic acid construct of any one of claims 7 to 8, the vector of claim 9, the cell according to claim 10, or the chimeric polypeptide according to any one of claims 11 to 13, for use as a medicament.

16. The nucleic acid sequence of any one of claims 1 to 6, the nucleic acid construct of any one of claims 7 to 8, the vector of claim 9, the cell according to claim 10, or the chimeric polypeptide according to any one of claims 11 to 13, for use in a method for treating a 1 a glycogen storage disease, such as GSDI (von Gierke's disease), GSDII (Pompe disease), GSDIII (Cori disease), GSDIV, GSDV, GSDVI, GSDVII, GSDVIII and lethal congenital glycogen storage disease of the heart, more particularly GSDI, GSDII or GSDIII, even more particularly GSDII and GSDIII, and most particularly GSDII.


SEQUENCE LISTING

<110>  GENETHON et al.

<120>  ACID-ALPHA GLUCOSIDASE VARIANTS AND USES THEREOF

<130>  B2298EP00

<160>  40

<170>  PatentIn version 3.3

<210>  1
<211>  2859
<212>  DNA
<213>  homo sapiens

<400>  1
atgggagtga ggcacccgcc ctgctcccac cggctcctgg ccgtctgcgc cctcgtgtcc      60

ttggcaaccg cagcgctcct ggggcacatc ctactccatg atttcctgct ggttccccga     120

gagctgagtg gctcctcccc agtcctggag gagactcacc cagctcacca gcagggagcc     180

agcagaccag ggccccggga tgcccaggca caccccgggc ggccgcgagc agtgcccaca     240

cagtgcgacg tcccccccaa cagccgcttc gattgcgccc ctgacaaggc catcacccag     300

gaacagtgcg aggcccgcgg ctgttgctac atccctgcaa agcaggggct gcagggagcc     360

cagatggggc agccctggtg cttcttccca cccagctacc ccagctacaa gctggagaac     420

ctgagctcct ctgaaatggg ctacacggcc accctgaccc gtaccacccc caccttcttc     480

cccaaggaca tcctgaccct gcggctggac gtgatgatgg agactgagaa ccgcctccac     540

ttcacgatca aagatccagc taacaggcgc tacgaggtgc ccttggagac cccgcatgtc     600

cacagccggg caccgtcccc actctacagc gtggagttct ccgaggagcc cttcggggtg     660

atcgtgcgcc ggcagctgga cggccgcgtg ctgctgaaca cgacggtggc gcccctgttc     720

tttgcggacc agttccttca gctgtccacc tcgctgccct cgcagtatat cacaggcctc     780

gccgagcacc tcagtcccct gatgctcagc accagctgga ccaggatcac cctgtggaac     840

cgggaccttg cgcccacgcc cggtgcgaac ctctacgggt ctcacccttt ctacctggcg     900

ctggaggacg gcgggtcggc acacggggtg ttcctgctaa acagcaatgc catggatgtg     960

gtcctgcagc cgagccctgc ccttagctgg aggtcgacag gtgggatcct ggatgtctac    1020

atcttcctgg gcccagagcc caagagcgtg gtgcagcagt acctggacgt tgtgggatac    1080

ccgttcatgc cgccatactg gggcctgggc ttccacctgt gccgctgggg ctactcctcc    1140

accgctatca cccgccaggt ggtggagaac atgaccaggg cccacttccc cctggacgtc    1200

cagtggaacg acctggacta catggactcc cggagggact tcacgttcaa caaggatggc    1260

ttccgggact tcccggccat ggtgcaggag ctgcaccagg gcggccggcg ctacatgatg    1320

atcgtggatc ctgccatcag cagctcgggc cctgccggga gctacaggcc ctacgacgag    1380

ggtctgcgga ggggggtttt catcaccaac gagaccggcc agccgctgat tgggaaggta 1440

tggcccgggt ccactgcctt ccccgacttc accaacccca cagccctggc ctggtgggag 1500

gacatggtgg ctgagttcca tgaccaggtg cccttcgacg gcatgtggat tgacatgaac 1560

gagccttcca acttcatcag gggctctgag gacggctgcc ccaacaatga gctggagaac 1620

ccaccctacg tgcctggggt ggttgggggg accctccagg cggccaccat ctgtgcctcc 1680

agccaccagt ttctctccac acactacaac ctgcacaacc tctacggcct gaccgaagcc 1740

atcgcctccc acagggcgct ggtgaaggct cgggggacac gcccatttgt gatctcccgc 1800

tcgacctttg ctggccacgg ccgatacgcc ggccactgga cgggggacgt gtggagctcc 1860

tgggagcagc tcgcctcctc cgtgccagaa atcctgcagt ttaacctgct gggggtgcct 1920

ctggtcgggg ccgacgtctg cggcttcctg ggcaacacct cagaggagct gtgtgtgcgc 1980

tggacccagc tgggggcctt ctaccccttc atgcggaacc acaacagcct gctcagtctg 2040

ccccaggagc cgtacagctt cagcgagccg gcccagcagg ccatgaggaa ggccctcacc 2100

ctgcgctacg cactcctccc ccacctctac acactgttcc accaggccca cgtcgcgggg 2160

gagaccgtgg cccggcccct cttcctggag ttccccaagg actctagcac ctggactgtg 2220

gaccaccagc tcctgtgggg ggaggccctg ctcatcaccc cagtgctcca ggccgggaag 2280

gccgaagtga ctggctactt ccccttgggc acatggtacg acctgcagac ggtgccagta 2340

gaggcccttg gcagcctccc acccccacct gcagctcccc gtgagccagc catccacagc 2400

gaggggcagt gggtgacgct gccggccccc ctggacacca tcaacgtcca cctccgggct 2460

gggtacatca tccccctgca gggccctggc ctcacaacca cagagtcccg ccagcagccc 2520

atggccctgg ctgtggccct gaccaagggt ggggaggccc gaggggagct gttctgggac 2580

gatggagaga gcctggaagt gctggagcga ggggcctaca cacaggtcat cttcctggcc 2640

aggaataaca cgatcgtgaa tgagctggta cgtgtgacca gtgagggagc tggcctgcag 2700

ctgcagaagg tgactgtcct gggcgtggcc acggcgcccc agcaggtcct ctccaacggt 2760

gtccctgtct ccaacttcac ctacagcccc gacaccaagg tcctggacat ctgtgtctcg 2820

ctgttgatgg gagagcagtt tctcgtcagc tggtgttag 2859

<210> 2
<211> 18
<212> PRT
<213> artificial

<220>
<223> sp7

<400> 2

Met Ala Phe Leu Trp Leu Leu Ser Cys Trp Ala Leu Leu Gly Thr Thr

1               5                   10                  15

Phe Gly

<210>  3
<211>  25
<212>  PRT
<213>  artificial

<220>
<223>  sp6

<400>  3

Met Pro Pro Pro Arg Thr Gly Arg Gly Leu Leu Trp Leu Gly Leu Val
1               5                   10                  15

Leu Ser Ser Val Cys Val Ala Leu Gly
            20                  25

<210>  4
<211>  22
<212>  PRT
<213>  artificial

<220>
<223>  sp8

<400>  4

Met Ala Ser Arg Leu Thr Leu Leu Thr Leu Leu Leu Leu Leu Leu Ala
1               5                   10                  15

Gly Asp Arg Ala Ser Ser
            20

<210>  5
<211>  925
<212>  PRT
<213>  artificial

<220>
<223>  hGAAwt w/o sp

<400>  5

Gly His Ile Leu Leu His Asp Phe Leu Leu Val Pro Arg Glu Leu Ser
1               5                   10                  15

Gly Ser Ser Pro Val Leu Glu Glu Thr His Pro Ala His Gln Gln Gly
            20                  25                  30

Ala Ser Arg Pro Gly Pro Arg Asp Ala Gln Ala His Pro Gly Arg Pro
        35                  40                  45

Arg Ala Val Pro Thr Gln Cys Asp Val Pro Pro Asn Ser Arg Phe Asp
    50                  55                  60

Cys Ala Pro Asp Lys Ala Ile Thr Gln Glu Gln Cys Glu Ala Arg Gly
65                  70                  75                  80

Cys Cys Tyr Ile Pro Ala Lys Gln Gly Leu Gln Gly Ala Gln Met Gly
                85                  90                  95

Gln Pro Trp Cys Phe Phe Pro Pro Ser Tyr Pro Ser Tyr Lys Leu Glu
            100                 105                 110

Asn Leu Ser Ser Ser Glu Met Gly Tyr Thr Ala Thr Leu Thr Arg Thr
        115                 120                 125

Thr Pro Thr Phe Phe Pro Lys Asp Ile Leu Thr Leu Arg Leu Asp Val
    130                 135                 140

Met Met Glu Thr Glu Asn Arg Leu His Phe Thr Ile Lys Asp Pro Ala
145                 150                 155                 160

Asn Arg Arg Tyr Glu Val Pro Leu Glu Thr Pro His Val His Ser Arg
                165                 170                 175

Ala Pro Ser Pro Leu Tyr Ser Val Glu Phe Ser Glu Glu Pro Phe Gly
            180                 185                 190

Val Ile Val Arg Arg Gln Leu Asp Gly Arg Val Leu Leu Asn Thr Thr
        195                 200                 205

Val Ala Pro Leu Phe Phe Ala Asp Gln Phe Leu Gln Leu Ser Thr Ser
    210                 215                 220

Leu Pro Ser Gln Tyr Ile Thr Gly Leu Ala Glu His Leu Ser Pro Leu
225                 230                 235                 240

Met Leu Ser Thr Ser Trp Thr Arg Ile Thr Leu Trp Asn Arg Asp Leu
                245                 250                 255

Ala Pro Thr Pro Gly Ala Asn Leu Tyr Gly Ser His Pro Phe Tyr Leu
            260                 265                 270

Ala Leu Glu Asp Gly Gly Ser Ala His Gly Val Phe Leu Leu Asn Ser
        275                 280                 285

Asn Ala Met Asp Val Val Leu Gln Pro Ser Pro Ala Leu Ser Trp Arg

290                 295                 300

Ser Thr Gly Gly Ile Leu Asp Val Tyr Ile Phe Leu Gly Pro Glu Pro
305                 310                 315                 320

Lys Ser Val Val Gln Gln Tyr Leu Asp Val Val Gly Tyr Pro Phe Met
                325                 330                 335

Pro Pro Tyr Trp Gly Leu Gly Phe His Leu Cys Arg Trp Gly Tyr Ser
            340                 345                 350

Ser Thr Ala Ile Thr Arg Gln Val Val Glu Asn Met Thr Arg Ala His
        355                 360                 365

Phe Pro Leu Asp Val Gln Trp Asn Asp Leu Asp Tyr Met Asp Ser Arg
    370                 375                 380

Arg Asp Phe Thr Phe Asn Lys Asp Gly Phe Arg Asp Phe Pro Ala Met
385                 390                 395                 400

Val Gln Glu Leu His Gln Gly Gly Arg Arg Tyr Met Met Ile Val Asp
                405                 410                 415

Pro Ala Ile Ser Ser Ser Gly Pro Ala Gly Ser Tyr Arg Pro Tyr Asp
            420                 425                 430

Glu Gly Leu Arg Arg Gly Val Phe Ile Thr Asn Glu Thr Gly Gln Pro
        435                 440                 445

Leu Ile Gly Lys Val Trp Pro Gly Ser Thr Ala Phe Pro Asp Phe Thr
    450                 455                 460

Asn Pro Thr Ala Leu Ala Trp Trp Glu Asp Met Val Ala Glu Phe His
465                 470                 475                 480

Asp Gln Val Pro Phe Asp Gly Met Trp Ile Asp Met Asn Glu Pro Ser
                485                 490                 495

Asn Phe Ile Arg Gly Ser Glu Asp Gly Cys Pro Asn Asn Glu Leu Glu
            500                 505                 510

Asn Pro Pro Tyr Val Pro Gly Val Val Gly Gly Thr Leu Gln Ala Ala
        515                 520                 525

Thr Ile Cys Ala Ser Ser His Gln Phe Leu Ser Thr His Tyr Asn Leu
    530                 535                 540

His Asn Leu Tyr Gly Leu Thr Glu Ala Ile Ala Ser His Arg Ala Leu
545                 550                 555                 560

Val Lys Ala Arg Gly Thr Arg Pro Phe Val Ile Ser Arg Ser Thr Phe
                565                 570                 575

Ala Gly His Gly Arg Tyr Ala Gly His Trp Thr Gly Asp Val Trp Ser
            580                 585                 590

Ser Trp Glu Gln Leu Ala Ser Ser Val Pro Glu Ile Leu Gln Phe Asn
        595                 600                 605

Leu Leu Gly Val Pro Leu Val Gly Ala Asp Val Cys Gly Phe Leu Gly
    610                 615                 620

Asn Thr Ser Glu Glu Leu Cys Val Arg Trp Thr Gln Leu Gly Ala Phe
625                 630                 635                 640

Tyr Pro Phe Met Arg Asn His Asn Ser Leu Leu Ser Leu Pro Gln Glu
                645                 650                 655

Pro Tyr Ser Phe Ser Glu Pro Ala Gln Gln Ala Met Arg Lys Ala Leu
            660                 665                 670

Thr Leu Arg Tyr Ala Leu Leu Pro His Leu Tyr Thr Leu Phe His Gln
        675                 680                 685

Ala His Val Ala Gly Glu Thr Val Ala Arg Pro Leu Phe Leu Glu Phe
    690                 695                 700

Pro Lys Asp Ser Ser Thr Trp Thr Val Asp His Gln Leu Leu Trp Gly
705                 710                 715                 720

Glu Ala Leu Leu Ile Thr Pro Val Leu Gln Ala Gly Lys Ala Glu Val
                725                 730                 735

Thr Gly Tyr Phe Pro Leu Gly Thr Trp Tyr Asp Leu Gln Thr Val Pro
            740                 745                 750

Val Glu Ala Leu Gly Ser Leu Pro Pro Pro Pro Ala Ala Pro Arg Glu
        755                 760                 765

Pro Ala Ile His Ser Glu Gly Gln Trp Val Thr Leu Pro Ala Pro Leu
    770                 775                 780

Asp Thr Ile Asn Val His Leu Arg Ala Gly Tyr Ile Ile Pro Leu Gln
785                 790                 795                 800

Gly Pro Gly Leu Thr Thr Thr Glu Ser Arg Gln Gln Pro Met Ala Leu
                805                 810                 815

Ala Val Ala Leu Thr Lys Gly Gly Glu Ala Arg Gly Glu Leu Phe Trp
            820                 825                 830

Asp Asp Gly Glu Ser Leu Glu Val Leu Glu Arg Gly Ala Tyr Thr Gln
        835                 840                 845

Val Ile Phe Leu Ala Arg Asn Asn Thr Ile Val Asn Glu Leu Val Arg
    850                 855                 860

Val Thr Ser Glu Gly Ala Gly Leu Gln Leu Gln Lys Val Thr Val Leu
865                 870                 875                 880

Gly Val Ala Thr Ala Pro Gln Gln Val Leu Ser Asn Gly Val Pro Val
                885                 890                 895

Ser Asn Phe Thr Tyr Ser Pro Asp Thr Lys Val Leu Asp Ile Cys Val
            900                 905                 910

Ser Leu Leu Met Gly Glu Gln Phe Leu Val Ser Trp Cys
        915                 920                 925

<210>  6
<211>  441
<212>  DNA
<213>  artificial

<220>
<223>  HBB2 intron

<400>  6
gtacacatat tgaccaaatc agggtaattt tgcatttgta attttaaaaa atgctttctt      60

cttttaatat acttttttgt ttatcttatt tctaatactt tccctaatct ctttctttca     120

gggcaataat gatacaatgt atcatgcctc tttgcaccat tctaaagaat aacagtgata     180

atttctgggt taaggcaata gcaatatttc tgcatataaa tatttctgca tataaattgt     240

aactgatgta agaggtttca tattgctaat agcagctaca atccagctac cattctgctt     300

ttattttatg gttgggataa ggctggatta ttctgagtcc aagctaggcc cttttgctaa     360

tcatgttcat acctcttatc ttcctcccac agctcctggg caacgtgctg gtctgtgtgc     420

tggcccatca ctttggcaaa g                                               441

<210>  7
<211>  441
<212>  DNA
<213>  modified HBB2 intron

<400>  7
gtacacatat tgaccaaatc agggtaattt tgcatttgta attttaaaaa atgctttctt      60

cttttaatat acttttttgt ttatcttatt tctaatactt tccctaatct ctttctttca     120

gggcaataat gatacaatgt atcatgcctc tttgcaccat tctaaagaat aacagtgata     180

atttctgggt taaggcaata gcaatatttc tgcatataaa tatttctgca tataaattgt     240

aactgatgta agaggtttca tattgctaat agcagctaca atccagctac cattctgctt     300

ttattttctg gttgggataa ggctggatta ttctgagtcc aagctaggcc cttttgctaa     360

tcttgttcat acctcttatc ttcctcccac agctcctggg caacctgctg gtctctctgc     420

tggcccatca ctttggcaaa g                                               441

<210>  8
<211>  1438
<212>  DNA
<213>  artificial

<220>
<223>  FIX intron

<400>  8
ggtttgtttc cttttttaaa atacattgag tatgcttgcc ttttagatat agaaatatct      60

gatgctgtct tcttcactaa attttgatta catgatttga cagcaatatt gaagagtcta     120

acagccagca cgcaggttgg taagtactgg ttctttgtta gctaggtttt cttcttcttc     180

atttttaaaa ctaaatagat cgacaatgct tatgatgcat ttatgtttaa taaacactgt     240

tcagttcatg atttggtcat gtaattcctg ttagaaaaca ttcatctcct tggtttaaaa     300

aaattaaaag tgggaaaaca aagaaatagc agaatatagt gaaaaaaaat aaccacatta     360

tttttgtttg gacttaccac tttgaaatca aaatgggaaa caaaagcaca aacaatggcc     420

ttatttacac aaaaagtctg attttaagat atatgacatt tcaaggtttc agaagtatgt     480

aatgaggtgt gtctctaatt ttttaaatta tatatcttca atttaaagtt ttagttaaaa     540

cataaagatt aacctttcat tagcaagctg ttagttatca ccaacgcttt tcatggatta     600

ggaaaaaatc attttgtctc tatgtcaaac atcttggagt tgatatttgg ggaaacacaa     660

tactcagttg agttccctag gggagaaaag cacgcttaag aattgacata aagagtagga     720

agttagctaa tgcaacatat atcactttgt tttttcacaa ctacagtgac tttatgtatt     780

tcccagagga aggcatacag ggaagaaatt atcccatttg gacaaacagc atgttctcac     840

aggaagcatt tatcacactt acttgtcaac tttctagaat caaatctagt agctgacagt     900

accaggatca ggggtgccaa ccctaagcac ccccagaaag ctgactggcc ctgtggttcc     960

cactccagac atgatgtcag ctgtgaaatc gacgtcgctg gaccataatt aggcttctgt    1020

tcttcaggag acatttgttc aaagtcattt gggcaaccat attctgaaaa cagcccagcc    1080

agggtgatgg atcactttgc aaagatcctc aatgagctat tttcaagtga tgacaaagtg 1140

tgaagttaac cgctcatttg agaactttct ttttcatcca aagtaaattc aaatatgatt 1200

agaaatctga ccttttatta ctggaattct cttgactaaa agtaaaattg aattttaatt 1260

cctaaatctc catgtgtata cagtactgtg ggaacatcac agattttggc tccatgccct 1320

aaagagaaat tggctttcag attatttgga ttaaaaacaa agactttctt aagagatgta 1380

aaattttcat gatgttttct tttttgctaa aactaaagaa ttattctttt acatttca 1438

<210> 9
<211> 1438
<212> DNA
<213> artificial

<220>
<223> modified FIX intron

<400> 9
   ggtttgtttc cttttttaaa atacattgag tatgcttgcc ttttagatat agaaatatct 60
   gatgctgtct tcttcactaa attttgatta catgatttga cagcaatatt gaagagtcta 120
   acagccagca cgcaggttgg taagtactgg ttctttgtta gctaggtttt cttcttcttc 180
   atttttaaaa ctaaatagat cgacattgct tttgttgcat ttatgtttaa taaacactgt 240
   tcagttcatg atttggtcat gtaattcctg ttagaaaaca ttcatctcct tggtttaaaa 300
   aaattaaaag tgggaaaaca aagaaatagc agaatatagt gaaaaaaaat aaccacatta 360
   tttttgtttg gacttaccac tttgaaatca aattgggaaa caaaagcaca aacaatggcc 420
   ttatttacac aaaaagtctg attttaagat atatgacatt tcaaggtttc agaagtatgt 480
   aatgaggtgt gtctctaatt ttttaaatta tatatcttca atttaaagtt ttagttaaaa 540
   cataaagatt aacctttcat tagcaagctg ttagttatca ccaacgcttt tcatggatta 600
   ggaaaaaatc attttgtctc tttgtcaaac atcttggagt tgatatttgg ggaaacacaa 660
   tactcagttg agttccctag gggagaaaag cacgcttaag aattgacata aagagtagga 720
   agttagctat tgcaacatat atcactttgt tttttcacaa ctacagtgac tttttgtatt 780
   tcccagagga aggcatacag ggaagaaatt atcccatttg gacaaacagc ttgttctcac 840
   aggaagcatt tatcacactt acttgtcaac tttctagaat caaatctagt agctgacagt 900
   accaggatca ggggtgccaa ccctaagcac ccccagaaag ctgactggcc ctgtggttcc 960
   cactccagac atgatgtcag ctgtgaaatc gacgtcgctg gaccataatt aggcttctgt 1020
   tcttcaggag acatttgttc aaagtcattt gggcaaccat attctgaaaa cagcccagcc 1080
   agggtgttgg atcactttgc aaagatcctc attgagctat tttcaagtgt tgacaaagtg 1140
   tgaagttaac cgctcatttg agaactttct ttttcatcca aagtaaattc aaatatgatt 1200
   agaaatctga ccttttatta ctggaattct cttgactaaa agtaaaattg aattttaatt 1260
   cctaaatctc catgtgtata cagtactgtg ggaacatcac agattttggc tccatgccct 1320
   aaagagaaat tggctttcag attatttgga ttaaaaacaa agactttctt aagagatgta 1380
   aaattttctt gttgttttct tttttgctaa aactaaagaa ttattctttt acatttca 1438

<210> 10
<211> 881
<212> DNA
<213> artificial

<220>
<223> chicken beta-globin intron

<400> 10
gcgggagtcg ctgcgttgcc ttcgccccgt gccccgctcc gccgccgcct cgcgccgccc 60

gccccggctc tgactgaccg cgttactccc acaggtgagc gggcgggacg gcccttctcc 120

tccgggctgt aattagcgct tggtttaatg acggcttgtt tcttttctgt ggctgcgtga 180

aagccttgag gggctccggg agggcccttt gtgcgggggg agcggctcgg ggggtgcgtg 240

cgtgtgtgtg tgcgtgggga gcgccgcgtg cggctccgcg ctgcccggcg gctgtgagcg 300

ctgcgggcgc ggcgcggggc tttgtgcgct ccgcagtgtg cgcgagggga gcgcggccgg 360

gggcggtgcc ccgcggtgcg gggggggctg cgaggggaac aaaggctgcg tgcggggtgt 420

gtgcgtgggg gggtgagcag ggggtgtggg cgcgtcggtc gggctgcaac cccccctgca 480

cccccctccc cgagttgctg agcacggccc ggcttcgggt gcggggctcc gtacggggcg 540

tggcgcgggg ctcgccgtgc cgggcggggg gtggcggcag gtgggggtgc cgggcggggc 600

ggggccgcct cgggccgggg agggctcggg ggaggggcgc ggcggccccc ggagcgccgg 660

cggctgtcga ggcgcggcga gccgcagcca ttgcctttta tggtaatcgt gcgagagggc 720

gcagggactt cctttgtccc aaatctgtgc ggagccgaaa tctgggaggc gccgccgcac 780

cccctctagc gggcgcgggg cgaagcggtg cggcgccggc aggaaggaaa tgggcgggga 840

gggccttcgt gcgtcgccgc gccgccgtcc ccttctccct c 881

<210> 11
<211> 881
<212> DNA
<213> artificial

<220>
<223> modified chicken beta-globin intron

<400> 11
gcgggagtcg ctgcgttgcc ttcgccccgt gccccgctcc gccgccgcct cgcgccgccc 60

gccccggctc tgactgaccg cgttactccc acaggtgagc gggcgggacg gcccttctcc 120

tccgggctgt aattagcgct tggtttaatg acggcttgtt tcttttctgt ggctgcgtga 180

aagccttgag gggctccggg agggcccttt gtgcgggggg agcggctcgg ggggtgcgtg 240

cgtgtgtgtg tgcgtgggga gcgccgcgtg cggctccgcg ctgcccggcg gctgtgagcg 300

ctgcgggcgc ggcgcggggc tttgtgcgct ccgcagtgtg cgcgagggga gcgcggccgg 360

gggcggtgcc ccgcggtgcg gggggggctg cgaggggaac aaaggctgcg tgcggggtgt 420

gtgcgtgggg gggtgagcag ggggtgtggg cgcgtcggtc gggctgcaac cccccctgca 480

cccccctccc cgagttgctg agcacggccc ggcttcgggt gcggggctcc gtacggggcg 540

tggcgcgggg ctcgccgtgc cgggcggggg gtggcggcag gtgggggtgc cgggcggggc 600

ggggccgcct cgggccgggg agggctcggg ggaggggcgc ggcggccccc ggagcgccgg 660

cggctgtcga ggcgcggcga gccgcagcca ttgccttttt tggtaatcgt gcgagagggc 720

gcagggactt cctttgtccc aaatctgtgc ggagccgaaa tctgggaggc gccgccgcac 780

cccctctagc gggcgcgggg cgaagcggtg cggcgccggc aggaaggaat tgggcgggga 840

gggccttcgt gcgtcgccgc gccgccgtcc ccttctccct c 881

<210> 12
<211> 952
<212> PRT
<213> homo sapiens

<400> 12

<210>  13
<211>  2778
<212>  DNA
<213>  artificial

<220>
<223>  hGAAco1 w/o sp

<400>  13
   ggccatatcc tgctgcacga ctttctacta gtgcccagag agctgagcgg cagctctccc      60
   gtgctggaag aaacacaccc tgcccatcag cagggcgcct ctagacctgg acctagagat     120
   gcccaggccc accccggcag acctagagct gtgcctaccc agtgtgacgt gccccccaac      180
   agcagattcg actgcgcccc tgacaaggcc atcacccagg aacagtgcga ggccagaggc     240
   tgctgctaca tccctgccaa gcagggactg cagggcgctc agatgggaca gccctggtgc     300
   ttcttcccac cctcctaccc cagctacaag ctggaaaacc tgagcagcag cgagatgggc     360
   tacaccgcca ccctgaccag aaccaccccc acattcttcc caaaggacat cctgaccctg     420
   cggctggacg tgatgatgga aaccgagaac cggctgcact tcaccatcaa ggaccccgcc     480
   aatcggagat acgaggtgcc cctggaaacc ccccacgtgc actctagagc ccccagccct     540
   ctgtacagcg tggaattcag cgaggaaccc ttcggcgtga tcgtgcggag acagctggat     600
   ggcagagtgc tgctgaacac caccgtggcc cctctgttct tcgccgacca gttcctgcag     660
   ctgagcacca gcctgcccag ccagtacatc acaggactgg ccgagcacct gagccccctg      720
   atgctgagca catcctggac ccggatcacc ctgtggaaca gggatctggc ccctacccct     780
   ggcgccaatc tgtacggcag ccaccctttc tacctggccc tggaagatgg cggatctgcc     840
   cacggagtgt ttctgctgaa ctccaacgcc atggacgtgg tgctgcagcc tagccctgcc     900
   ctgtcttgga gaagcacagg cggcatcctg gatgtgtaca tctttctggg ccccgagccc     960
   aagagcgtgg tgcagcagta tctggatgtc gtgggctacc ccttcatgcc cccttactgg    1020
   ggcctgggat tccacctgtg cagatggggc tactccagca ccgccatcac cagacaggtg 1080
   gtggaaaaca tgaccagagc ccacttccca ctggatgtgc agtggaacga cctggactac 1140
   atggacagca gacgggactt caccttcaac aaggacggct tccgggactt ccccgccatg 1200
   gtgcaggaac tgcatcaggg cggcagacgg tacatgatga tcgtggatcc cgccatcagc 1260
   tcctctggcc ctgccggctc ttacagaccc tacgacgagg gcctgcggag aggcgtgttc 1320
   atcaccaacg agacaggcca gcccctgatc ggcaaagtgt ggcctggcag cacagccttc 1380
   cccgacttca ccaatcctac cgccctggct tggtgggagg acatggtggc cgagttccac 1440
   gaccaggtgc ccttcgacgg catgtggatc gacatgaacg agcccagcaa cttcatccgg 1500
   ggcagcgagg atggctgccc caacaacgaa ctggaaaatc ccccttacgt gcccggcgtc 1560
   gtgggcggaa cactgcaggc cgctacaatc tgtgccagca gccaccagtt tctgagcacc 1620
   cactacaacc tgcacaacct gtacggcctg accgaggcca ttgccagcca ccgcgctctc 1680
   gtgaaagcca gaggcacacg gcccttcgtg atcagcagaa gcacctttgc cggccacggc 1740
   agatacgccg gacattggac tggcgacgtg tggtcctctt gggagcagct ggcctctagc 1800
   gtgcccgaga tcctgcagtt caatctgctg ggcgtgccac tcgtgggcgc cgatgtgtgt 1860
   ggcttcctgg gcaacacctc cgaggaactg tgtgtgcggt ggacacagct gggcgccttc 1920
   taccctttca tgagaaacca caacagcctg ctgagcctgc cccaggaacc ctacagcttt 1980
   agcgagcctg cacagcaggc catgcggaag gccctgacac tgagatacgc tctgctgccc 2040
   cacctgtaca ccctgtttca ccaggcccat gtggccggcg agacagtggc cagacctctg 2100
   tttctggaat tccccaagga cagcagcacc tggaccgtgg accatcagct gctgtgggga 2160
   gaggctctgc tgattacccc agtgctgcag gcaggcaagg ccgaagtgac cggctacttt 2220
   cccctgggca cttggtacga cctgcagacc gtgcctgtgg aagccctggg atctctgcct 2280
   ccacctcctg ccgctcctag agagcctgcc attcactctg agggccagtg ggtcacactg 2340
   cctgcccccc tggataccat caacgtgcac ctgagggccg gctacatcat accactgcag 2400
   ggacctggcc tgaccaccac cgagtctaga cagcagccaa tggccctggc cgtggccctg 2460
   accaaaggcg gagaagctag gggcgagctg ttctgggacg atggcgagag cctggaagtg 2520
   ctggaaagag gcgcctatac ccaagtgatc ttcctggccc ggaacaacac catcgtgaac 2580
   gagctggtgc gcgtgacctc tgaaggcgct ggactgcagc tgcagaaagt gaccgtgctg 2640
   ggagtggcca cagcccctca gcaggtgctg tctaatggcg tgcccgtgtc caacttcacc 2700
   tacagccccg acaccaaggt gctggacatc tgcgtgtcac tgctgatggg agagcagttt 2760
   ctggtgtcct ggtgctga 2778

<210> 14

<211>  2778
<212>  DNA
<213>  artificial

<220>
<223>  hGAAco2 w/o sp

<400>  14
ggacacatcc tgctgcacga cttcctgttg gtgcctagag agctgagcgg atcatcccca      60

gtgctggagg agactcatcc tgctcaccaa cagggagctt ccagaccagg accgagagac     120

gcccaagccc atcctggtag accaagagct gtgcctaccc aatgcgacgt gccacccaac     180

tcccgattcg actgcgcgcc agataaggct attacccaag agcagtgtga agccagaggt     240

tgctgctaca tcccagcgaa gcaaggattg caaggcgccc aaatgggaca accttggtgt     300

ttcttccccc cttcgtaccc atcatataaa ctcgaaaacc tgtcctcttc ggaaatgggt     360

tatactgcca ccctcaccag aactactcct actttcttcc cgaaagacat cttgaccttg     420

aggctggacg tgatgatgga gactgaaaac cggctgcatt tcactatcaa agatcctgcc     480

aatcggcgat acgaggtccc tctggaaacc cctcacgtgc actcacgggc tccttctccg     540

ctttactccg tcgaattctc tgaggaaccc ttcggagtga tcgttagacg ccagctggat     600

ggtagagtgc tgttgaacac tactgtggcc ccacttttct tcgctgacca gtttctgcaa     660

ctgtccactt ccctgccatc ccagtacatt actggactcg ccgaacacct gtcgccactg     720

atgctctcga cctcttggac tagaatcact ttgtggaaca gagacttggc ccctactccg     780

ggagcaaatc tgtacggaag ccaccctttt tacctggcgc tcgaagatgg cggatccgct     840

cacggagtgt tcctgctgaa tagcaacgca atggacgtgg tgctgcaacc ttcccctgca     900

ctcagttgga gaagtaccgg gggtattctg gacgtgtaca tcttcctcgg accagaaccc     960

aagagcgtgg tgcagcaata tctggacgtg gtcggatacc cttttatgcc tccttactgg    1020

ggactgggat tccacctttg ccgttggggc tactcatcca ccgccattac cagacaggtg    1080

gtggagaata tgaccagagc ccacttccct ctcgacgtgc agtggaacga tctggactat    1140

atggactccc ggagagattt caccttcaac aaggacgggt tccgcgattt tcccgcgatg    1200

gttcaagagc tccaccaggg tggtcgaaga tatatgatga tcgtcgaccc agccatttcg    1260

agcagcggac ccgctggatc ttatagacct tacgacgaag gccttaggag aggagtgttc    1320

atcacaaacg agactggaca gcctttgatc ggtaaagtgt ggcctggatc aaccgccttt    1380

cctgacttta ccaatcccac tgccttggct tggtgggagg acatggtggc cgaattccac    1440

gaccaagtcc cctttgatgg aatgtggatc gatatgaacg aaccaagcaa ttttatcaga    1500

ggttccgaag acggttgccc caacaacgaa ctggaaaacc ctccttatgt gcccggagtc    1560

gtgggcggaa cattacaggc cgcgactatt tgcgccagca gccaccaatt cctgtccact    1620

cactacaacc tccacaacct ttatggatta accgaagcta ttgcaagtca cagggctctg    1680

gtgaaggcta gagggactag gccctttgtg atctcccgat ccacctttgc cggacacggg 1740

agatacgccg gtcactggac tggtgacgtg tggagctcat gggaacaact ggcctcctcc 1800

gtgccggaaa tcttacagtt caaccttctg ggtgtccctc ttgtcggagc agacgtgtgt 1860

gggtttcttg gtaacacctc cgaggaactg tgtgtgcgct ggactcaact gggtgcattc 1920

tacccattca tgagaaacca caactccttg ctgtccctgc cacaagagcc ctactcgttc 1980

agcgagcctg cacaacaggc tatgcggaag gcactgaccc tgagatacgc cctgcttcca 2040

cacttataca ctctcttcca tcaagcgcat gtggcaggag aaaccgttgc aaggcctctt 2100

ttccttgaat tccccaagga ttcctcgact tggacggtgg atcatcagct gctgtgggga 2160

gaagctctgc tgattactcc agtgttgcaa gccggaaaag ctgaggtgac cggatacttt 2220

ccgctgggaa cctggtacga cctccagact gtccctgttg aagcccttgg atcactgcct 2280

ccgcctccgg cagctccacg cgaaccagct atacattccg agggacagtg ggttacatta 2340

ccagctcctc tggacacaat caacgtccac ttaagagctg gctacattat ccctctgcaa 2400

ggaccaggac tgactacgac cgagagcaga cagcagccaa tggcactggc tgtggctctg 2460

accaagggag gggaagctag aggagaactc ttctgggatg atggggagtc ccttgaagtg 2520

ctggaaagag gcgcttacac tcaagtcatt ttccttgcac ggaacaacac cattgtgaac 2580

gaattggtgc gagtgaccag cgaaggagct ggacttcaac tgcagaaggt cactgtgctc 2640

ggagtggcta ccgctcctca gcaagtgctg tcgaatggag tccccgtgtc aaactttacc 2700

tactcccctg acactaaggt gctcgacatt tgcgtgtccc tcctgatggg agagcagttc 2760

cttgtgtcct ggtgttga 2778

<210> 15
<211> 397
<212> DNA
<213> artificial

<220>
<223> hAAT promoter

<400> 15
gatcttgcta ccagtggaac agccactaag gattctgcag tgagagcaga gggccagcta 60

agtggtactc tcccagagac tgtctgactc acgccacccc ctccaccttg gacacaggac 120

gctgtggttt ctgagccagg tacaatgact cctttcggta agtgcagtgg aagctgtaca 180

ctgcccaggc aaagcgtccg ggcagcgtag gcgggcgact cagatcccag ccagtggact 240

tagcccctgt ttgctcctcc gataactggg gtgaccttgg ttaatattca ccagcagcct 300

cccccgttgc ccctctggat ccactgctta aatacggacg aggacagggc cctgtctcct 360

cagcttcagg caccaccact gacctgggac agtgaat 397

<210> 16
<211> 321
<212> DNA
<213> artificial

<220>
<223> ApoE control region

<400> 16
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg g 321


<210> 17
<211> 2808
<212> DNA
<213> artificial

<220>
<223> sp7+hGAAco1-delta-8

<400> 17
atggcctttc tgtggctgct gagctgttgg gccctgctgg gcaccacctt cggcctacta 60

gtgcccagag agctgagcgg cagctctccc gtgctggaag aaacacaccc tgcccatcag 120

cagggcgcct ctagacctgg acctagagat gcccaggccc accccggcag acctagagct 180

gtgcctaccc agtgtgacgt gccccccaac agcagattcg actgcgcccc tgacaaggcc 240

atcacccagg aacagtgcga ggccagaggc tgctgctaca tccctgccaa gcagggactg 300

cagggcgctc agatgggaca gccctggtgc ttcttcccac cctcctaccc cagctacaag 360

ctggaaaacc tgagcagcag cgagatgggc tacaccgcca ccctgaccag aaccaccccc 420

acattcttcc caaaggacat cctgaccctg cggctggacg tgatgatgga aaccgagaac 480

cggctgcact tcaccatcaa ggaccccgcc aatcggagat acgaggtgcc cctggaaacc 540

ccccacgtgc actctagagc ccccagccct ctgtacagcg tggaattcag cgaggaaccc 600

ttcggcgtga tcgtgcggag acagctggat ggcagagtgc tgctgaacac caccgtggcc 660

cctctgttct tcgccgacca gttcctgcag ctgagcacca gcctgcccag ccagtacatc 720

acaggactgg ccgagcacct gagccccctg atgctgagca catcctggac ccggatcacc 780

ctgtggaaca gggatctggc ccctacccct ggcgccaatc tgtacggcag ccaccctttc 840

tacctggccc tggaagatgg cggatctgcc cacggagtgt ttctgctgaa ctccaacgcc 900

atggacgtgg tgctgcagcc tagccctgcc ctgtcttgga gaagcacagg cggcatcctg 960

gatgtgtaca tctttctggg ccccgagccc aagagcgtgg tgcagcagta tctggatgtc 1020

gtgggctacc ccttcatgcc cccttactgg ggcctgggat tccacctgtg cagatggggc 1080

tactccagca ccgccatcac cagacaggtg gtggaaaaca tgaccagagc ccacttccca 1140

ctggatgtgc agtggaacga cctggactac atggacagca gacgggactt caccttcaac 1200

aaggacggct tccgggactt ccccgccatg gtgcaggaac tgcatcaggg cggcagacgg 1260

tacatgatga tcgtggatcc cgccatcagc tcctctggcc ctgccggctc ttacagaccc 1320

tacgacgagg gcctgcggag aggcgtgttc atcaccaacg agacaggcca gcccctgatc 1380

ggcaaagtgt ggcctggcag cacagccttc cccgacttca ccaatcctac cgccctggct 1440

tggtgggagg acatggtggc cgagttccac gaccaggtgc ccttcgacgg catgtggatc 1500

gacatgaacg agcccagcaa cttcatccgg ggcagcgagg atggctgccc caacaacgaa 1560

ctggaaaatc ccccttacgt gcccggcgtc gtgggcggaa cactgcaggc cgctacaatc 1620

tgtgccagca gccaccagtt tctgagcacc cactacaacc tgcacaacct gtacggcctg 1680

accgaggcca ttgccagcca ccgcgctctc gtgaaagcca gaggcacacg gcccttcgtg 1740

atcagcagaa gcacctttgc cggccacggc agatacgccg gacattggac tggcgacgtg 1800

tggtcctctt gggagcagct ggcctctagc gtgcccgaga tcctgcagtt caatctgctg 1860

ggcgtgccac tcgtgggcgc cgatgtgtgt ggcttcctgg gcaacacctc cgaggaactg 1920

tgtgtgcggt ggacacagct gggcgccttc taccctttca tgagaaacca caacagcctg 1980

ctgagcctgc cccaggaacc ctacagcttt agcgagcctg cacagcaggc catgcggaag 2040

gccctgacac tgagatacgc tctgctgccc cacctgtaca ccctgtttca ccaggcccat 2100

gtggccggcg agacagtggc cagacctctg tttctggaat tccccaagga cagcagcacc 2160

tggaccgtgg accatcagct gctgtgggga gaggctctgc tgattacccc agtgctgcag 2220

gcaggcaagg ccgaagtgac cggctacttt cccctgggca cttggtacga cctgcagacc 2280

gtgcctgtgg aagccctggg atctctgcct ccacctcctg ccgctcctag agagcctgcc 2340

attcactctg agggccagtg ggtcacactg cctgcccccc tggataccat caacgtgcac 2400

ctgagggccg gctacatcat accactgcag ggacctggcc tgaccaccac cgagtctaga 2460

cagcagccaa tggccctggc cgtggccctg accaaaggcg gagaagctag gggcgagctg 2520

ttctgggacg atggcgagag cctggaagtg ctggaaagag gcgcctatac ccaagtgatc 2580

ttcctggccc ggaacaacac catcgtgaac gagctggtgc gcgtgacctc tgaaggcgct 2640

ggactgcagc tgcagaaagt gaccgtgctg ggagtggcca cagcccctca gcaggtgctg 2700

tctaatggcg tgcccgtgtc caacttcacc tacagccccg acaccaaggt gctggacatc 2760

tgcgtgtcac tgctgatggg agagcagttt ctggtgtcct ggtgctga 2808

<210>  18
<211>  2829
<212>  DNA
<213>  artificial

<220>
<223>  sp6+hGAAco1-delta-8

<400>  18
atgcctccac ctagaacagg cagaggcctg ctgtggctgg gcctggtgct gtctagtgtg      60

tgtgtggccc tgggcctact agtgcccaga gagctgagcg gcagctctcc cgtgctggaa     120

gaaacacacc ctgcccatca gcagggcgcc tctagacctg gacctagaga tgcccaggcc     180

caccccggca gacctagagc tgtgcctacc cagtgtgacg tgccccccaa cagcagattc     240

gactgcgccc ctgacaaggc catcacccag gaacagtgcg aggccagagg ctgctgctac     300

atccctgcca agcagggact gcagggcgct cagatgggac agccctggtg cttcttccca     360

ccctcctacc ccagctacaa gctggaaaac ctgagcagca gcgagatggg ctacaccgcc     420

accctgacca gaaccacccc cacattcttc ccaaaggaca tcctgaccct gcggctggac     480

gtgatgatgg aaaccgagaa ccggctgcac ttcaccatca aggaccccgc caatcggaga     540

tacgaggtgc ccctggaaac cccccacgtg cactctagag cccccagccc tctgtacagc     600

gtggaattca gcgaggaacc cttcggcgtg atcgtgcgga gacagctgga tggcagagtg     660

ctgctgaaca ccaccgtggc ccctctgttc ttcgccgacc agttcctgca gctgagcacc     720

agcctgccca gccagtacat cacaggactg gccgagcacc tgagccccct gatgctgagc     780

acatcctgga cccggatcac cctgtggaac agggatctgg cccctacccc tggcgccaat     840

ctgtacggca gccacccttt ctacctggcc ctggaagatg gcggatctgc ccacggagtg     900

tttctgctga actccaacgc catggacgtg gtgctgcagc ctagccctgc cctgtcttgg     960

agaagcacag gcggcatcct ggatgtgtac atctttctgg gccccgagcc caagagcgtg    1020

gtgcagcagt atctggatgt cgtgggctac cccttcatgc ccccttactg gggcctggga    1080

ttccacctgt gcagatgggg ctactccagc accgccatca ccagacaggt ggtggaaaac    1140

atgaccagag cccacttccc actggatgtg cagtggaacg acctggacta catggacagc    1200

agacgggact tcaccttcaa caaggacggc ttccgggact tccccgccat ggtgcaggaa    1260

ctgcatcagg gcggcagacg gtacatgatg atcgtggatc ccgccatcag ctcctctggc    1320

cctgccggct cttacagacc ctacgacgag ggcctgcgga gaggcgtgtt catcaccaac    1380

gagacaggcc agcccctgat cggcaaagtg tggcctggca gcacagcctt ccccgacttc    1440

accaatccta ccgccctggc ttggtgggag gacatggtgg ccgagttcca cgaccaggtg    1500

cccttcgacg gcatgtggat cgacatgaac gagcccagca acttcatccg gggcagcgag    1560

gatggctgcc ccaacaacga actggaaaat cccccttacg tgcccggcgt cgtgggcgga    1620

acactgcagg ccgctacaat ctgtgccagc agccaccagt ttctgagcac ccactacaac 1680

ctgcacaacc tgtacggcct gaccgaggcc attgccagcc accgcgctct cgtgaaagcc 1740

agaggcacac ggcccttcgt gatcagcaga agcacctttg ccggccacgg cagatacgcc 1800

ggacattgga ctggcgacgt gtggtcctct tgggagcagc tggcctctag cgtgcccgag 1860

atcctgcagt tcaatctgct gggcgtgcca ctcgtgggcg ccgatgtgtg tggcttcctg 1920

ggcaacacct ccgaggaact gtgtgtgcgg tggacacagc tgggcgcctt ctaccctttc 1980

atgagaaacc acaacagcct gctgagcctg ccccaggaac cctacagctt tagcgagcct 2040

gcacagcagg ccatgcggaa ggccctgaca ctgagatacg ctctgctgcc ccacctgtac 2100

accctgtttc accaggccca tgtggccggc gagacagtgg ccagacctct gtttctggaa 2160

ttccccaagg acagcagcac ctggaccgtg gaccatcagc tgctgtgggg agaggctctg 2220

ctgattaccc cagtgctgca ggcaggcaag gccgaagtga ccggctactt tcccctgggc 2280

acttggtacg acctgcagac cgtgcctgtg gaagccctgg gatctctgcc tccacctcct 2340

gccgctccta gagagcctgc cattcactct gagggccagt gggtcacact gcctgccccc 2400

ctggatacca tcaacgtgca cctgagggcc ggctacatca taccactgca gggacctggc 2460

ctgaccacca ccgagtctag acagcagcca atggccctgg ccgtggccct gaccaaaggc 2520

ggagaagcta ggggcgagct gttctgggac gatggcgaga gcctggaagt gctggaaaga 2580

ggcgcctata cccaagtgat cttcctggcc cggaacaaca ccatcgtgaa cgagctggtg 2640

cgcgtgacct ctgaaggcgc tggactgcag ctgcagaaag tgaccgtgct gggagtggcc 2700

acagcccctc agcaggtgct gtctaatggc gtgcccgtgt ccaacttcac ctacagcccc 2760

gacaccaagg tgctggacat ctgcgtgtca ctgctgatgg gagagcagtt tctggtgtcc 2820

tggtgctga 2829

<210> 19
<211> 2820
<212> DNA
<213> artificial

<220>
<223> sp8+hGAAco1-delta-8

<400> 19
atggccagca gactgaccct gctgacactc cttctgctgc tgctggccgg cgatagagcc 60

agcagcctac tagtgcccag agagctgagc ggcagctctc ccgtgctgga agaaacacac 120

cctgcccatc agcagggcgc ctctagacct ggacctagag atgcccaggc ccaccccggc 180

agacctagag ctgtgcctac ccagtgtgac gtgcccccca acagcagatt cgactgcgcc 240

cctgacaagg ccatcaccca ggaacagtgc gaggccagag gctgctgcta catccctgcc 300

aagcagggac tgcagggcgc tcagatggga cagccctggt gcttcttccc accctcctac 360

cccagctaca agctggaaaa cctgagcagc agcgagatgg gctacaccgc caccctgacc 420

agaaccaccc ccacattctt cccaaaggac atcctgaccc tgcggctgga cgtgatgatg 480

gaaaccgaga accggctgca cttcaccatc aaggaccccg ccaatcggag atacgaggtg 540

cccctggaaa ccccccacgt gcactctaga gcccccagcc ctctgtacag cgtggaattc 600

agcgaggaac ccttcggcgt gatcgtgcgg agacagctgg atggcagagt gctgctgaac 660

accaccgtgg cccctctgtt cttcgccgac cagttcctgc agctgagcac cagcctgccc 720

agccagtaca tcacaggact ggccgagcac ctgagccccc tgatgctgag cacatcctgg 780

acccggatca ccctgtggaa cagggatctg gcccctaccc ctggcgccaa tctgtacggc 840

agccaccctt tctacctggc cctggaagat ggcggatctg cccacggagt gtttctgctg 900

aactccaacg ccatggacgt ggtgctgcag cctagccctg ccctgtcttg gagaagcaca 960

ggcggcatcc tggatgtgta catctttctg ggccccgagc ccaagagcgt ggtgcagcag 1020

tatctggatg tcgtgggcta ccccttcatg cccccttact ggggcctggg attccacctg 1080

tgcagatggg gctactccag caccgccatc accagacagg tggtggaaaa catgaccaga 1140

gcccacttcc cactggatgt gcagtggaac gacctggact acatggacag cagacgggac 1200

ttcaccttca acaaggacgg cttccgggac ttccccgcca tggtgcagga actgcatcag 1260

ggcggcagac ggtacatgat gatcgtggat cccgccatca gctcctctgg ccctgccggc 1320

tcttacagac cctacgacga gggcctgcgg agaggcgtgt tcatcaccaa cgagacaggc 1380

cagcccctga tcggcaaagt gtggcctggc agcacagcct tccccgactt caccaatcct 1440

accgccctgg cttggtggga ggacatggtg gccgagttcc acgaccaggt gcccttcgac 1500

ggcatgtgga tcgacatgaa cgagcccagc aacttcatcc ggggcagcga ggatggctgc 1560

cccaacaacg aactggaaaa tcccccttac gtgcccggcg tcgtgggcgg aacactgcag 1620

gccgctacaa tctgtgccag cagccaccag tttctgagca cccactacaa cctgcacaac 1680

ctgtacggcc tgaccgaggc cattgccagc caccgcgctc tcgtgaaagc cagaggcaca 1740

cggcccttcg tgatcagcag aagcaccttt gccggccacg gcagatacgc cggacattgg 1800

actggcgacg tgtggtcctc ttgggagcag ctggcctcta gcgtgcccga gatcctgcag 1860

ttcaatctgc tgggcgtgcc actcgtgggc gccgatgtgt gtggcttcct gggcaacacc 1920

tccgaggaac tgtgtgtgcg gtggacacag ctgggcgcct tctacccttt catgagaaac 1980

cacaacagcc tgctgagcct gccccaggaa ccctacagct ttagcgagcc tgcacagcag 2040

gccatgcgga aggccctgac actgagatac gctctgctgc cccacctgta caccctgttt 2100

caccaggccc atgtggccgg cgagacagtg gccagacctc tgtttctgga attccccaag 2160

gacagcagca cctggaccgt ggaccatcag ctgctgtggg gagaggctct gctgattacc 2220

ccagtgctgc aggcaggcaa ggccgaagtg accggctact ttcccctggg cacttggtac 2280

gacctgcaga ccgtgcctgt ggaagccctg ggatctctgc ctccacctcc tgccgctcct 2340

agagagcctg ccattcactc tgagggccag tgggtcacac tgcctgcccc cctggatacc 2400

atcaacgtgc acctgagggc cggctacatc ataccactgc agggacctgg cctgaccacc 2460

accgagtcta gacagcagcc aatggccctg gccgtggccc tgaccaaagg cggagaagct 2520

aggggcgagc tgttctggga cgatggcgag agcctggaag tgctggaaag aggcgcctat 2580

acccaagtga tcttcctggc ccggaacaac accatcgtga acgagctggt gcgcgtgacc 2640

tctgaaggcg ctggactgca gctgcagaaa gtgaccgtgc tgggagtggc cacagcccct 2700

cagcaggtgc tgtctaatgg cgtgcccgtg tccaacttca cctacagccc cgacaccaag 2760

gtgctggaca tctgcgtgtc actgctgatg ggagagcagt ttctggtgtc ctggtgctga 2820

<210> 20
<211> 4300
<212> DNA
<213> artificial

<220>
<223> construct: sp7+hGAAco1-delta-8

<400> 20
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg ggtacccggg gatcttgcta ccagtggaac agccactaag 360

gattctgcag tgagagcaga gggccagcta agtggtactc tcccagagac tgtctgactc 420

acgccacccc ctccaccttg gacacaggac gctgtggttt ctgagccagg tacaatgact 480

cctttcggta agtgcagtgg aagctgtaca ctgcccaggc aaagcgtccg ggcagcgtag 540

gcgggcgact cagatcccag ccagtggact tagcccctgt ttgctcctcc gataactggg 600

gtgaccttgg ttaatattca ccagcagcct cccccgttgc ccctctggat ccactgctta 660

aatacggacg aggacagggc cctgtctcct cagcttcagg caccaccact gacctgggac 720

agtgaataga tcctgagaac ttcagggtga gtctatggga cccttgatgt tttctttccc 780

cttcttttct atggttaagt tcatgtcata ggaaggggag aagtaacagg gtacacatat 840

tgaccaaatc agggtaattt tgcatttgta attttaaaaa atgctttctt cttttaatat 900

acttttttgt ttatcttatt tctaatactt tccctaatct ctttctttca gggcaataat 960

gatacaatgt atcatgcctc tttgcaccat tctaaagaat aacagtgata atttctgggt 1020
taaggcaata gcaatatttc tgcatataaa tatttctgca tataaattgt aactgatgta 1080
agaggtttca tattgctaat agcagctaca atccagctac cattctgctt ttattttctg 1140
gttgggataa ggctggatta ttctgagtcc aagctaggcc cttttgctaa tcttgttcat 1200
acctcttatc ttcctcccac agctcctggg caacctgctg gtctctctgc tggcccatca 1260
ctttggcaaa gcacgcgtgc caccatggcc tttctgtggc tgctgagctg ttgggccctg 1320
ctgggcacca ccttcggcct actagtgccc agagagctga gcggcagctc tcccgtgctg 1380
gaagaaacac accctgccca tcagcagggc gcctctagac ctggacctag agatgcccag 1440
gcccaccccg gcagacctag agctgtgcct acccagtgtg acgtgccccc caacagcaga 1500
ttcgactgcg cccctgacaa ggccatcacc caggaacagt gcgaggccag aggctgctgc 1560
tacatccctg ccaagcaggg actgcagggc gctcagatgg gacagccctg gtgcttcttc 1620
ccaccctcct accccagcta caagctggaa aacctgagca gcagcgagat gggctacacc 1680
gccaccctga ccagaaccac ccccacattc ttcccaaagg acatcctgac cctgcggctg 1740
gacgtgatga tggaaaccga gaaccggctg cacttcacca tcaaggaccc cgccaatcgg 1800
agatacgagg tgcccctgga aaccccccac gtgcactcta gagcccccag ccctctgtac 1860
agcgtggaat tcagcgagga acccttcggc gtgatcgtgc ggagacagct ggatggcaga 1920
gtgctgctga acaccaccgt ggcccctctg ttcttcgccg accagttcct gcagctgagc 1980
accagcctgc ccagccagta catcacagga ctggccgagc acctgagccc cctgatgctg 2040
agcacatcct ggacccggat caccctgtgg aacagggatc tggcccctac ccctggcgcc 2100
aatctgtacg gcagccaccc tttctacctg gccctggaag atggcggatc tgcccacgga 2160
gtgtttctgc tgaactccaa cgccatggac gtggtgctgc agcctagccc tgccctgtct 2220
tggagaagca caggcggcat cctggatgtg tacatctttc tgggccccga gcccaagagc 2280
gtggtgcagc agtatctgga tgtcgtgggc taccccttca tgccccctta ctggggcctg 2340
ggattccacc tgtgcagatg gggctactcc agcaccgcca tcaccagaca ggtggtggaa 2400
aacatgacca gagcccactt cccactggat gtgcagtgga acgacctgga ctacatggac 2460
agcagacggg acttcacctt caacaaggac ggcttccggg acttccccgc catggtgcag 2520
gaactgcatc agggcggcag acggtacatg atgatcgtgg atcccgccat cagctcctct 2580
ggccctgccg gctcttacag accctacgac gagggcctgc ggagaggcgt gttcatcacc 2640
aacgagacag gccagcccct gatcggcaaa gtgtggcctg gcagcacagc cttccccgac 2700
ttcaccaatc ctaccgccct ggcttggtgg gaggacatgg tggccgagtt ccacgaccag 2760
gtgcccttcg acggcatgtg gatcgacatg aacgagccca gcaacttcat ccggggcagc 2820
gaggatggct gccccaacaa cgaactggaa aatccccctt acgtgcccgg cgtcgtgggc 2880

ggaacactgc aggccgctac aatctgtgcc agcagccacc agtttctgag cacccactac 2940

aacctgcaca acctgtacgg cctgaccgag gccattgcca gccaccgcgc tctcgtgaaa 3000

gccagaggca cacggccctt cgtgatcagc agaagcacct ttgccggcca cggcagatac 3060

gccggacatt ggactggcga cgtgtggtcc tcttgggagc agctggcctc tagcgtgccc 3120

gagatcctgc agttcaatct gctgggcgtg ccactcgtgg gcgccgatgt gtgtggcttc 3180

ctgggcaaca cctccgagga actgtgtgtg cggtggacac agctgggcgc cttctaccct 3240

ttcatgagaa accacaacag cctgctgagc ctgccccagg aaccctacag ctttagcgag 3300

cctgcacagc aggccatgcg gaaggccctg acactgagat acgctctgct gccccacctg 3360

tacaccctgt ttcaccaggc ccatgtggcc ggcgagacag tggccagacc tctgtttctg 3420

gaattcccca aggacagcag cacctggacc gtggaccatc agctgctgtg gggagaggct 3480

ctgctgatta ccccagtgct gcaggcaggc aaggccgaag tgaccggcta ctttcccctg 3540

ggcacttggt acgacctgca gaccgtgcct gtggaagccc tgggatctct gcctccacct 3600

cctgccgctc ctagagagcc tgccattcac tctgagggcc agtgggtcac actgcctgcc 3660

cccctggata ccatcaacgt gcacctgagg gccggctaca tcataccact gcagggacct 3720

ggcctgacca ccaccgagtc tagacagcag ccaatggccc tggccgtggc cctgaccaaa 3780

ggcggagaag ctaggggcga gctgttctgg gacgatggcg agagcctgga agtgctggaa 3840

agaggcgcct atacccaagt gatcttcctg gcccggaaca acaccatcgt gaacgagctg 3900

gtgcgcgtga cctctgaagg cgctggactg cagctgcaga aagtgaccgt gctgggagtg 3960

gccacagccc ctcagcaggt gctgtctaat ggcgtgcccg tgtccaactt cacctacagc 4020

cccgacacca aggtgctgga catctgcgtg tcactgctga tgggagagca gtttctggtg 4080

tcctggtgct gactcgagag atctaccggt gaattcaccg cgggtttaaa ctgtgccttc 4140

tagttgccag ccatctgttg tttgcccctc ccccgtgcct tccttgaccc tggaaggtgc 4200

cactcccact gtcctttcct aataaaatga ggaaattgca tcgcattgtc tgagtaggtg 4260

tcattctatt ctggggggtg gggtgggggc tagctctaga 4300

<210> 21
<211> 4321
<212> DNA
<213> artificial

<220>
<223> construct: sp6+hGAAco1-delta-8

<400> 21
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg ggtacccggg gatcttgcta ccagtggaac agccactaag 360

gattctgcag tgagagcaga gggccagcta agtggtactc tcccagagac tgtctgactc 420

acgccacccc ctccaccttg gacacaggac gctgtggttt ctgagccagg tacaatgact 480

cctttcggta agtgcagtgg aagctgtaca ctgcccaggc aaagcgtccg ggcagcgtag 540

gcgggcgact cagatcccag ccagtggact tagcccctgt ttgctcctcc gataactggg 600

gtgaccttgg ttaatattca ccagcagcct cccccgttgc ccctctggat ccactgctta 660

aatacggacg aggacagggc cctgtctcct cagcttcagg caccaccact gacctgggac 720

agtgaataga tcctgagaac ttcagggtga gtctatggga cccttgatgt tttctttccc 780

cttcttttct atggttaagt tcatgtcata ggaaggggag aagtaacagg gtacacatat 840

tgaccaaatc agggtaattt tgcatttgta attttaaaaa atgctttctt cttttaatat 900

acttttttgt ttatcttatt tctaatactt tccctaatct ctttctttca gggcaataat 960

gatacaatgt atcatgcctc tttgcaccat tctaaagaat aacagtgata atttctgggt 1020

taaggcaata gcaatatttc tgcatataaa tatttctgca tataaattgt aactgatgta 1080

agaggtttca tattgctaat agcagctaca atccagctac cattctgctt ttattttctg 1140

gttgggataa ggctggatta ttctgagtcc aagctaggcc cttttgctaa tcttgttcat 1200

acctcttatc ttcctcccac agctcctggg caacctgctg gtctctctgc tggcccatca 1260

ctttggcaaa gcacgcgtgc caccatgcct ccacctagaa caggcagagg cctgctgtgg 1320

ctgggcctgg tgctgtctag tgtgtgtgtg gccctgggcc tactagtgcc cagagagctg 1380

agcggcagct ctcccgtgct ggaagaaaca caccctgccc atcagcaggg cgcctctaga 1440

cctggaccta gagatgccca ggcccacccc ggcagaccta gagctgtgcc tacccagtgt 1500

gacgtgcccc ccaacagcag attcgactgc gcccctgaca aggccatcac ccaggaacag 1560

tgcgaggcca gaggctgctg ctacatccct gccaagcagg gactgcaggg cgctcagatg 1620

ggacagccct ggtgcttctt cccaccctcc taccccagct acaagctgga aaacctgagc 1680

agcagcgaga tgggctacac cgccaccctg accagaacca cccccacatt cttcccaaag 1740

gacatcctga ccctgcggct ggacgtgatg atggaaaccg agaaccggct gcacttcacc 1800

atcaaggacc ccgccaatcg gagatacgag gtgcccctgg aaacccccca cgtgcactct 1860

agagccccca gccctctgta cagcgtggaa ttcagcgagg aacccttcgg cgtgatcgtg 1920

cggagacagc tggatggcag agtgctgctg aacaccaccg tggcccctct gttcttcgcc 1980

gaccagttcc tgcagctgag caccagcctg cccagccagt acatcacagg actggccgag 2040

cacctgagcc ccctgatgct gagcacatcc tggacccgga tcaccctgtg gaacagggat 2100

ctggccccta cccctggcgc caatctgtac ggcagccacc ctttctacct ggccctggaa 2160

gatggcggat ctgcccacgg agtgtttctg ctgaactcca acgccatgga cgtggtgctg 2220

cagcctagcc ctgccctgtc ttggagaagc acaggcggca tcctggatgt gtacatcttt 2280

ctgggccccg agcccaagag cgtggtgcag cagtatctgg atgtcgtggg ctaccccttc 2340

atgccccctt actggggcct gggattccac ctgtgcagat ggggctactc cagcaccgcc 2400

atcaccagac aggtggtgga aaacatgacc agagcccact tcccactgga tgtgcagtgg 2460

aacgacctgg actacatgga cagcagacgg gacttcacct tcaacaagga cggcttccgg 2520

gacttccccg ccatggtgca ggaactgcat cagggcggca gacggtacat gatgatcgtg 2580

gatcccgcca tcagctcctc tggccctgcc ggctcttaca gaccctacga cgagggcctg 2640

cggagaggcg tgttcatcac caacgagaca ggccagcccc tgatcggcaa agtgtggcct 2700

ggcagcacag ccttccccga cttcaccaat cctaccgccc tggcttggtg ggaggacatg 2760

gtggccgagt tccacgacca ggtgcccttc gacggcatgt ggatcgacat gaacgagccc 2820

agcaacttca tccggggcag cgaggatggc tgccccaaca acgaactgga aaatccccct 2880

tacgtgcccg gcgtcgtggg cggaacactg caggccgcta caatctgtgc cagcagccac 2940

cagtttctga gcacccacta caacctgcac aacctgtacg gcctgaccga ggccattgcc 3000

agccaccgcg ctctcgtgaa agccagaggc acacggccct tcgtgatcag cagaagcacc 3060

tttgccggcc acggcagata cgccggacat tggactggcg acgtgtggtc ctcttgggag 3120

cagctggcct ctagcgtgcc cgagatcctg cagttcaatc tgctgggcgt gccactcgtg 3180

ggcgccgatg tgtgtggctt cctgggcaac acctccgagg aactgtgtgt gcggtggaca 3240

cagctgggcg ccttctaccc tttcatgaga aaccacaaca gcctgctgag cctgccccag 3300

gaaccctaca gctttagcga gcctgcacag caggccatgc ggaaggccct gacactgaga 3360

tacgctctgc tgccccacct gtacaccctg tttcaccagg cccatgtggc cggcgagaca 3420

gtggccagac ctctgtttct ggaattcccc aaggacagca gcacctggac cgtggaccat 3480

cagctgctgt ggggagaggc tctgctgatt accccagtgc tgcaggcagg caaggccgaa 3540

gtgaccggct actttcccct gggcacttgg tacgacctgc agaccgtgcc tgtggaagcc 3600

ctgggatctc tgcctccacc tcctgccgct cctagagagc ctgccattca ctctgagggc 3660

cagtgggtca cactgcctgc ccccctggat accatcaacg tgcacctgag ggccggctac 3720

atcataccac tgcagggacc tggcctgacc accaccgagt ctagacagca gccaatggcc 3780

ctggccgtgg ccctgaccaa aggcggagaa gctaggggcg agctgttctg ggacgatggc 3840

gagagcctgg aagtgctgga aagaggcgcc tatacccaag tgatcttcct ggcccggaac 3900

aacaccatcg tgaacgagct ggtgcgcgtg acctctgaag gcgctggact gcagctgcag 3960

aaagtgaccg tgctgggagt ggccacagcc cctcagcagg tgctgtctaa tggcgtgccc 4020

gtgtccaact tcacctacag ccccgacacc aaggtgctgg acatctgcgt gtcactgctg 4080

atgggagagc agtttctggt gtcctggtgc tgactcgaga gatctaccgg tgaattcacc 4140

gcgggtttaa actgtgcctt ctagttgcca gccatctgtt gtttgcccct cccccgtgcc 4200

ttccttgacc ctggaaggtg ccactcccac tgtcctttcc taataaaatg aggaaattgc 4260

atcgcattgt ctgagtaggt gtcattctat tctggggggt ggggtggggg ctagctctag 4320

a 4321

<210> 22
<211> 4312
<212> DNA
<213> artificial

<220>
<223> sp8+hGAAco1-delta-8

<400> 22
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg ggtacccggg gatcttgcta ccagtggaac agccactaag 360

gattctgcag tgagagcaga gggccagcta agtggtactc tcccagagac tgtctgactc 420

acgccacccc ctccaccttg gacacaggac gctgtggttt ctgagccagg tacaatgact 480

cctttcggta agtgcagtgg aagctgtaca ctgcccaggc aaagcgtccg ggcagcgtag 540

gcgggcgact cagatcccag ccagtggact tagcccctgt ttgctcctcc gataactggg 600

gtgaccttgg ttaatattca ccagcagcct cccccgttgc ccctctggat ccactgctta 660

aatacggacg aggacagggc cctgtctcct cagcttcagg caccaccact gacctgggac 720

agtgaataga tcctgagaac ttcagggtga gtctatggga cccttgatgt tttctttccc 780

cttcttttct atggttaagt tcatgtcata ggaaggggag aagtaacagg gtacacatat 840

tgaccaaatc agggtaattt tgcatttgta attttaaaaa atgctttctt cttttaatat 900

acttttttgt ttatcttatt tctaatactt tccctaatct ctttctttca gggcaataat 960

gatacaatgt atcatgcctc tttgcaccat tctaaagaat aacagtgata atttctgggt 1020

taaggcaata gcaatatttc tgcatataaa tatttctgca tataaattgt aactgatgta 1080

agaggtttca tattgctaat agcagctaca atccagctac cattctgctt ttattttctg 1140

gttgggataa ggctggatta ttctgagtcc aagctaggcc cttttgctaa tcttgttcat 1200
acctcttatc ttcctcccac agctcctggg caacctgctg gtctctctgc tggcccatca 1260
ctttggcaaa gcacgcgtgc caccatggcc agcagactga ccctgctgac actccttctg 1320
ctgctgctgg ccggcgatag agccagcagc ctactagtgc ccagagagct gagcggcagc 1380
tctcccgtgc tggaagaaac acaccctgcc catcagcagg gcgcctctag acctggacct 1440
agagatgccc aggcccaccc cggcagacct agagctgtgc ctacccagtg tgacgtgccc 1500
cccaacagca gattcgactg cgcccctgac aaggccatca cccaggaaca gtgcgaggcc 1560
agaggctgct gctacatccc tgccaagcag ggactgcagg gcgctcagat gggacagccc 1620
tggtgcttct tcccaccctc ctaccccagc tacaagctgg aaaacctgag cagcagcgag 1680
atgggctaca ccgccaccct gaccagaacc acccccacat tcttcccaaa ggacatcctg 1740
accctgcggc tggacgtgat gatggaaacc gagaaccggc tgcacttcac catcaaggac 1800
cccgccaatc ggagatacga ggtgcccctg gaaacccccc acgtgcactc tagagccccc 1860
agccctctgt acagcgtgga attcagcgag gaacccttcg gcgtgatcgt gcggagacag 1920
ctggatggca gagtgctgct gaacaccacc gtggcccctc tgttcttcgc cgaccagttc 1980
ctgcagctga gcaccagcct gcccagccag tacatcacag gactggccga gcacctgagc 2040
cccctgatgc tgagcacatc ctggacccgg atcaccctgt ggaacaggga tctggcccct 2100
acccctggcg ccaatctgta cggcagccac cctttctacc tggccctgga agatggcgga 2160
tctgcccacg gagtgtttct gctgaactcc aacgccatgg acgtggtgct gcagcctagc 2220
cctgccctgt cttggagaag cacaggcggc atcctggatg tgtacatctt tctgggcccc 2280
gagcccaaga gcgtggtgca gcagtatctg gatgtcgtgg gctacccctt catgcccccc 2340
tactggggcc tgggattcca cctgtgcaga tggggctact ccagcaccgc catcaccaga 2400
caggtggtgg aaaacatgac cagagcccac ttcccactgg atgtgcagtg gaacgacctg 2460
gactacatgg acagcagacg ggacttcacc ttcaacaagg acggcttccg ggacttcccc 2520
gccatggtgc aggaactgca tcagggcggc agacggtaca tgatgatcgt ggatcccgcc 2580
atcagctcct ctggccctgc cggctcttac agaccctacg acgagggcct gcggagaggc 2640
gtgttcatca ccaacgagac aggccagccc ctgatcggca aagtgtggcc tggcagcaca 2700
gccttccccg acttcaccaa tcctaccgcc ctggcttggt gggaggacat ggtggccgag 2760
ttccacgacc aggtgccctt cgacggcatg tggatcgaca tgaacgagcc cagcaacttc 2820
atccggggca gcgaggatgg ctgccccaac aacgaactgg aaaatccccc ttacgtgccc 2880
ggcgtcgtgg gcggaacact gcaggccgct acaatctgtg ccagcagcca ccagtttctg 2940
agcacccact acaacctgca caacctgtac ggcctgaccg aggccattgc cagccaccgc 3000

gctctcgtga aagccagagg cacacggccc ttcgtgatca gcagaagcac ctttgccggc 3060

cacggcagat acgccggaca ttggactggc gacgtgtggt cctcttggga gcagctggcc 3120

tctagcgtgc ccgagatcct gcagttcaat ctgctgggcg tgccactcgt gggcgccgat 3180

gtgtgtggct tcctgggcaa cacctccgag gaactgtgtg tgcggtggac acagctgggc 3240

gccttctacc ctttcatgag aaaccacaac agcctgctga gcctgcccca ggaaccctac 3300

agctttagcg agcctgcaca gcaggccatg cggaaggccc tgacactgag atacgctctg 3360

ctgccccacc tgtacaccct gtttcaccag gcccatgtgg ccggcgagac agtggccaga 3420

cctctgtttc tggaattccc caaggacagc agcacctgga ccgtggacca tcagctgctg 3480

tggggagagg ctctgctgat taccccagtg ctgcaggcag gcaaggccga agtgaccggc 3540

tactttcccc tgggcacttg gtacgacctg cagaccgtgc ctgtggaagc cctgggatct 3600

ctgcctccac ctcctgccgc tcctagagag cctgccattc actctgaggg ccagtgggtc 3660

acactgcctg cccccctgga taccatcaac gtgcacctga gggccggcta catcatacca 3720

ctgcagggac ctggcctgac caccaccgag tctagacagc agccaatggc cctggccgtg 3780

gccctgacca aaggcggaga agctaggggc gagctgttct gggacgatgg cgagagcctg 3840

gaagtgctgg aaagaggcgc ctatacccaa gtgatcttcc tggcccggaa caacaccatc 3900

gtgaacgagc tggtgcgcgt gacctctgaa ggcgctggac tgcagctgca gaaagtgacc 3960

gtgctgggag tggccacagc ccctcagcag gtgctgtcta atggcgtgcc cgtgtccaac 4020

ttcacctaca gccccgacac caaggtgctg gacatctgcg tgtcactgct gatgggagag 4080

cagtttctgg tgtcctggtg ctgactcgag agatctaccg gtgaattcac cgcgggttta 4140

aactgtgcct tctagttgcc agccatctgt tgtttgcccc tcccccgtgc cttccttgac 4200

cctggaaggt gccactccca ctgtcctttc ctaataaaat gaggaaattg catcgcattg 4260

tctgagtagg tgtcattcta ttctgggggg tggggtgggg gctagctcta ga 4312

<210> 23
<211> 16
<212> PRT
<213> artificial

<220>
<223> sp3

<400> 23

Met Leu Leu Leu Ser Ala Leu Leu Leu Gly Leu Ala Phe Gly Tyr Ser
1               5                   10                  15

<210> 24
<211> 16
<212> PRT
<213> artificial

<220>
<223>  sp4

<400>  24

Met Leu Leu Ser Phe Ala Leu Leu Leu Gly Leu Ala Leu Gly Tyr Ser
1               5                   10                  15


<210>  25
<211>  16
<212>  PRT
<213>  artificial

<220>
<223>  sp5

<400>  25

Met Leu Leu Glu His Ala Leu Leu Leu Gly Leu Ala His Gly Tyr Ser
1               5                   10                  15


<210>  26
<211>  54
<212>  DNA
<213>  artificial

<220>
<223>  sp7

<400>  26
atggcctttc tgtggctgct gagctgttgg gccctgctgg gcaccacctt cggc          54


<210>  27
<211>  75
<212>  DNA
<213>  artificial

<220>
<223>  sp6

<400>  27
atgcctccac ctagaacagg cagaggcctg ctgtggctgg gcctggtgct gtctagtgtg      60

tgtgtggccc tgggc                                                       75


<210>  28
<211>  66
<212>  DNA
<213>  artificial

<220>
<223>  sp8

<400>  28
atggccagca gactgaccct gctgacactc cttctgctgc tgctggccgg cgatagagcc      60

agcagc                                                                 66

<210>  29
<211>  917
<212>  PRT
<213>  artificial

<220>
<223>  hGAA-delta-8

<400>  29

Leu Leu Val Pro Arg Glu Leu Ser Gly Ser Ser Pro Val Leu Glu Glu
1               5                   10                  15

Thr His Pro Ala His Gln Gln Gly Ala Ser Arg Pro Gly Pro Arg Asp
            20                  25                  30

Ala Gln Ala His Pro Gly Arg Pro Arg Ala Val Pro Thr Gln Cys Asp
        35                  40                  45

Val Pro Pro Asn Ser Arg Phe Asp Cys Ala Pro Asp Lys Ala Ile Thr
    50                  55                  60

Gln Glu Gln Cys Glu Ala Arg Gly Cys Cys Tyr Ile Pro Ala Lys Gln
65                  70                  75                  80

Gly Leu Gln Gly Ala Gln Met Gly Gln Pro Trp Cys Phe Phe Pro Pro
                85                  90                  95

Ser Tyr Pro Ser Tyr Lys Leu Glu Asn Leu Ser Ser Ser Glu Met Gly
            100                 105                 110

Tyr Thr Ala Thr Leu Thr Arg Thr Thr Pro Thr Phe Phe Pro Lys Asp
        115                 120                 125

Ile Leu Thr Leu Arg Leu Asp Val Met Met Glu Thr Glu Asn Arg Leu
    130                 135                 140

His Phe Thr Ile Lys Asp Pro Ala Asn Arg Arg Tyr Glu Val Pro Leu
145                 150                 155                 160

Glu Thr Pro His Val His Ser Arg Ala Pro Ser Pro Leu Tyr Ser Val
                165                 170                 175

Glu Phe Ser Glu Glu Pro Phe Gly Val Ile Val Arg Arg Gln Leu Asp
            180                 185                 190

Gly Arg Val Leu Leu Asn Thr Thr Val Ala Pro Leu Phe Phe Ala Asp
        195                 200                 205

Gln Phe Leu Gln Leu Ser Thr Ser Leu Pro Ser Gln Tyr Ile Thr Gly
    210                 215                 220

Leu Ala Glu His Leu Ser Pro Leu Met Leu Ser Thr Ser Trp Thr Arg
225                 230                 235                 240

Ile Thr Leu Trp Asn Arg Asp Leu Ala Pro Thr Pro Gly Ala Asn Leu
                245                 250                 255

Tyr Gly Ser His Pro Phe Tyr Leu Ala Leu Glu Asp Gly Gly Ser Ala
            260                 265                 270

His Gly Val Phe Leu Leu Asn Ser Asn Ala Met Asp Val Val Leu Gln
        275                 280                 285

Pro Ser Pro Ala Leu Ser Trp Arg Ser Thr Gly Gly Ile Leu Asp Val
    290                 295                 300

Tyr Ile Phe Leu Gly Pro Glu Pro Lys Ser Val Val Gln Gln Tyr Leu
305                 310                 315                 320

Asp Val Val Gly Tyr Pro Phe Met Pro Pro Tyr Trp Gly Leu Gly Phe
                325                 330                 335

His Leu Cys Arg Trp Gly Tyr Ser Ser Thr Ala Ile Thr Arg Gln Val
            340                 345                 350

Val Glu Asn Met Thr Arg Ala His Phe Pro Leu Asp Val Gln Trp Asn
        355                 360                 365

Asp Leu Asp Tyr Met Asp Ser Arg Arg Asp Phe Thr Phe Asn Lys Asp
    370                 375                 380

Gly Phe Arg Asp Phe Pro Ala Met Val Gln Glu Leu His Gln Gly Gly
385                 390                 395                 400

Arg Arg Tyr Met Met Ile Val Asp Pro Ala Ile Ser Ser Ser Gly Pro
                405                 410                 415

Ala Gly Ser Tyr Arg Pro Tyr Asp Glu Gly Leu Arg Arg Gly Val Phe
            420                 425                 430

Ile Thr Asn Glu Thr Gly Gln Pro Leu Ile Gly Lys Val Trp Pro Gly
        435                 440                 445

Ser Thr Ala Phe Pro Asp Phe Thr Asn Pro Thr Ala Leu Ala Trp Trp
    450                 455                 460

Glu Asp Met Val Ala Glu Phe His Asp Gln Val Pro Phe Asp Gly Met
465                 470                 475                 480

Trp Ile Asp Met Asn Glu Pro Ser Asn Phe Ile Arg Gly Ser Glu Asp
                485                 490                 495

Gly Cys Pro Asn Asn Glu Leu Glu Asn Pro Pro Tyr Val Pro Gly Val
            500                 505                 510

Val Gly Gly Thr Leu Gln Ala Ala Thr Ile Cys Ala Ser Ser His Gln
        515                 520                 525

Phe Leu Ser Thr His Tyr Asn Leu His Asn Leu Tyr Gly Leu Thr Glu
    530                 535                 540

Ala Ile Ala Ser His Arg Ala Leu Val Lys Ala Arg Gly Thr Arg Pro
545                 550                 555                 560

Phe Val Ile Ser Arg Ser Thr Phe Ala Gly His Gly Arg Tyr Ala Gly
                565                 570                 575

His Trp Thr Gly Asp Val Trp Ser Ser Trp Glu Gln Leu Ala Ser Ser
            580                 585                 590

Val Pro Glu Ile Leu Gln Phe Asn Leu Leu Gly Val Pro Leu Val Gly
        595                 600                 605

Ala Asp Val Cys Gly Phe Leu Gly Asn Thr Ser Glu Glu Leu Cys Val
    610                 615                 620

Arg Trp Thr Gln Leu Gly Ala Phe Tyr Pro Phe Met Arg Asn His Asn
625                 630                 635                 640

Ser Leu Leu Ser Leu Pro Gln Glu Pro Tyr Ser Phe Ser Glu Pro Ala
                645                 650                 655

Gln Gln Ala Met Arg Lys Ala Leu Thr Leu Arg Tyr Ala Leu Leu Pro
            660                 665                 670

His Leu Tyr Thr Leu Phe His Gln Ala His Val Ala Gly Glu Thr Val
        675                 680                 685

Ala Arg Pro Leu Phe Leu Glu Phe Pro Lys Asp Ser Ser Thr Trp Thr
    690                 695                 700

Val Asp His Gln Leu Leu Trp Gly Glu Ala Leu Leu Ile Thr Pro Val
705                 710                 715                 720

Leu Gln Ala Gly Lys Ala Glu Val Thr Gly Tyr Phe Pro Leu Gly Thr
                725                 730                 735

Trp Tyr Asp Leu Gln Thr Val Pro Val Glu Ala Leu Gly Ser Leu Pro
            740                 745                 750

Pro Pro Pro Ala Ala Pro Arg Glu Pro Ala Ile His Ser Glu Gly Gln
        755                 760                 765

Trp Val Thr Leu Pro Ala Pro Leu Asp Thr Ile Asn Val His Leu Arg
    770                 775                 780

Ala Gly Tyr Ile Ile Pro Leu Gln Gly Pro Gly Leu Thr Thr Thr Glu
785                 790                 795                 800

Ser Arg Gln Gln Pro Met Ala Leu Ala Val Ala Leu Thr Lys Gly Gly
                805                 810                 815

Glu Ala Arg Gly Glu Leu Phe Trp Asp Asp Gly Glu Ser Leu Glu Val
            820                 825                 830

Leu Glu Arg Gly Ala Tyr Thr Gln Val Ile Phe Leu Ala Arg Asn Asn
        835                 840                 845

Thr Ile Val Asn Glu Leu Val Arg Val Thr Ser Glu Gly Ala Gly Leu
    850                 855                 860

Gln Leu Gln Lys Val Thr Val Leu Gly Val Ala Thr Ala Pro Gln Gln
865                 870                 875                 880

Val Leu Ser Asn Gly Val Pro Val Ser Asn Phe Thr Tyr Ser Pro Asp
                885                 890                 895

Thr Lys Val Leu Asp Ile Cys Val Ser Leu Leu Met Gly Glu Gln Phe
            900                 905                 910

Leu Val Ser Trp Cys
        915

<210>  30
<211>  883
<212>  PRT
<213>  artificial

<220>
<223>  hGAA-delta-42

<400>  30

Ala His Pro Gly Arg Pro Arg Ala Val Pro Thr Gln Cys Asp Val Pro
1               5                   10                  15

Pro Asn Ser Arg Phe Asp Cys Ala Pro Asp Lys Ala Ile Thr Gln Glu
            20                  25                  30

Gln Cys Glu Ala Arg Gly Cys Cys Tyr Ile Pro Ala Lys Gln Gly Leu
        35                  40                  45

Gln Gly Ala Gln Met Gly Gln Pro Trp Cys Phe Phe Pro Pro Ser Tyr
    50                  55                  60

Pro Ser Tyr Lys Leu Glu Asn Leu Ser Ser Ser Glu Met Gly Tyr Thr
65                  70                  75                  80

Ala Thr Leu Thr Arg Thr Thr Pro Thr Phe Phe Pro Lys Asp Ile Leu
                85                  90                  95

Thr Leu Arg Leu Asp Val Met Met Glu Thr Glu Asn Arg Leu His Phe
            100                 105                 110

Thr Ile Lys Asp Pro Ala Asn Arg Arg Tyr Glu Val Pro Leu Glu Thr
        115                 120                 125

Pro His Val His Ser Arg Ala Pro Ser Pro Leu Tyr Ser Val Glu Phe
    130                 135                 140

Ser Glu Glu Pro Phe Gly Val Ile Val Arg Arg Gln Leu Asp Gly Arg
145                 150                 155                 160

Val Leu Leu Asn Thr Thr Val Ala Pro Leu Phe Phe Ala Asp Gln Phe
                165                 170                 175

Leu Gln Leu Ser Thr Ser Leu Pro Ser Gln Tyr Ile Thr Gly Leu Ala
            180                 185                 190

Glu His Leu Ser Pro Leu Met Leu Ser Thr Ser Trp Thr Arg Ile Thr
        195                 200                 205

Leu Trp Asn Arg Asp Leu Ala Pro Thr Pro Gly Ala Asn Leu Tyr Gly
    210                 215                 220

Ser His Pro Phe Tyr Leu Ala Leu Glu Asp Gly Gly Ser Ala His Gly
225                 230                 235                 240

Val Phe Leu Leu Asn Ser Asn Ala Met Asp Val Val Leu Gln Pro Ser
                245                 250                 255

Pro Ala Leu Ser Trp Arg Ser Thr Gly Gly Ile Leu Asp Val Tyr Ile
            260                 265                 270

Phe Leu Gly Pro Glu Pro Lys Ser Val Val Gln Gln Tyr Leu Asp Val
        275                 280                 285

Val Gly Tyr Pro Phe Met Pro Pro Tyr Trp Gly Leu Gly Phe His Leu
    290                 295                 300

Cys Arg Trp Gly Tyr Ser Ser Thr Ala Ile Thr Arg Gln Val Val Glu
305                 310                 315                 320

Asn Met Thr Arg Ala His Phe Pro Leu Asp Val Gln Trp Asn Asp Leu
                325                 330                 335

Asp Tyr Met Asp Ser Arg Arg Asp Phe Thr Phe Asn Lys Asp Gly Phe
            340                 345                 350

Arg Asp Phe Pro Ala Met Val Gln Glu Leu His Gln Gly Gly Arg Arg
        355                 360                 365

Tyr Met Met Ile Val Asp Pro Ala Ile Ser Ser Ser Gly Pro Ala Gly
    370                 375                 380

Ser Tyr Arg Pro Tyr Asp Glu Gly Leu Arg Arg Gly Val Phe Ile Thr
385                 390                 395                 400

Asn Glu Thr Gly Gln Pro Leu Ile Gly Lys Val Trp Pro Gly Ser Thr
                405                 410                 415

Ala Phe Pro Asp Phe Thr Asn Pro Thr Ala Leu Ala Trp Trp Glu Asp
            420                 425                 430

Met Val Ala Glu Phe His Asp Gln Val Pro Phe Asp Gly Met Trp Ile
        435                 440                 445

Asp Met Asn Glu Pro Ser Asn Phe Ile Arg Gly Ser Glu Asp Gly Cys
    450                 455                 460

Pro Asn Asn Glu Leu Glu Asn Pro Pro Tyr Val Pro Gly Val Val Gly
465                 470                 475                 480

Gly Thr Leu Gln Ala Ala Thr Ile Cys Ala Ser Ser His Gln Phe Leu
                485                 490                 495

Ser Thr His Tyr Asn Leu His Asn Leu Tyr Gly Leu Thr Glu Ala Ile

            500                 505                 510

Ala Ser His Arg Ala Leu Val Lys Ala Arg Gly Thr Arg Pro Phe Val
        515                 520                 525

Ile Ser Arg Ser Thr Phe Ala Gly His Gly Arg Tyr Ala Gly His Trp
    530                 535                 540

Thr Gly Asp Val Trp Ser Ser Trp Glu Gln Leu Ala Ser Ser Val Pro
545                 550                 555                 560

Glu Ile Leu Gln Phe Asn Leu Leu Gly Val Pro Leu Val Gly Ala Asp
                565                 570                 575

Val Cys Gly Phe Leu Gly Asn Thr Ser Glu Glu Leu Cys Val Arg Trp
            580                 585                 590

Thr Gln Leu Gly Ala Phe Tyr Pro Phe Met Arg Asn His Asn Ser Leu
        595                 600                 605

Leu Ser Leu Pro Gln Glu Pro Tyr Ser Phe Ser Glu Pro Ala Gln Gln
    610                 615                 620

Ala Met Arg Lys Ala Leu Thr Leu Arg Tyr Ala Leu Leu Pro His Leu
625                 630                 635                 640

Tyr Thr Leu Phe His Gln Ala His Val Ala Gly Glu Thr Val Ala Arg
                645                 650                 655

Pro Leu Phe Leu Glu Phe Pro Lys Asp Ser Ser Thr Trp Thr Val Asp
            660                 665                 670

His Gln Leu Leu Trp Gly Glu Ala Leu Leu Ile Thr Pro Val Leu Gln
        675                 680                 685

Ala Gly Lys Ala Glu Val Thr Gly Tyr Phe Pro Leu Gly Thr Trp Tyr
    690                 695                 700

Asp Leu Gln Thr Val Pro Val Glu Ala Leu Gly Ser Leu Pro Pro Pro
705                 710                 715                 720

Pro Ala Ala Pro Arg Glu Pro Ala Ile His Ser Glu Gly Gln Trp Val
                725                 730                 735

Thr Leu Pro Ala Pro Leu Asp Thr Ile Asn Val His Leu Arg Ala Gly
            740                 745                 750

Tyr Ile Ile Pro Leu Gln Gly Pro Gly Leu Thr Thr Thr Glu Ser Arg
        755                 760                 765

Gln Gln Pro Met Ala Leu Ala Val Ala Leu Thr Lys Gly Gly Glu Ala
    770                 775                 780

Arg Gly Glu Leu Phe Trp Asp Asp Gly Glu Ser Leu Glu Val Leu Glu
785                 790                 795                 800

Arg Gly Ala Tyr Thr Gln Val Ile Phe Leu Ala Arg Asn Asn Thr Ile
                805                 810                 815

Val Asn Glu Leu Val Arg Val Thr Ser Glu Gly Ala Gly Leu Gln Leu
            820                 825                 830

Gln Lys Val Thr Val Leu Gly Val Ala Thr Ala Pro Gln Gln Val Leu
        835                 840                 845

Ser Asn Gly Val Pro Val Ser Asn Phe Thr Tyr Ser Pro Asp Thr Lys
    850                 855                 860

Val Leu Asp Ile Cys Val Ser Leu Leu Met Gly Glu Gln Phe Leu Val
865                 870                 875                 880

Ser Trp Cys

<210> 31
<211> 2778
<212> DNA
<213> hGAAwt w/o sp

<400> 31
gggcacatcc tactccatga tttcctgctg gttccccgag agctgagtgg ctcctcccca 60

gtcctggagg agactcaccc agctcaccag cagggagcca gcagaccagg gccccgggat 120

gcccaggcac accccgggcg gccgcgagca gtgcccacac agtgcgacgt cccccccaac 180

agccgcttcg attgcgcccc tgacaaggcc atcacccagg aacagtgcga ggcccgcggc 240

tgttgctaca tccctgcaaa gcaggggctg cagggagccc agatggggca gccctggtgc 300

ttcttcccac ccagctaccc cagctacaag ctggagaacc tgagctcctc tgaaatgggc 360

tacacggcca ccctgacccg taccaccccc accttcttcc ccaaggacat cctgaccctg 420

cggctggacg tgatgatgga gactgagaac cgcctccact tcacgatcaa agatccagct 480

aacaggcgct acgaggtgcc cttggagacc ccgcatgtcc acagccgggc accgtcccca 540

ctctacagcg tggagttctc cgaggagccc ttcggggtga tcgtgcgccg gcagctggac 600

ggccgcgtgc tgctgaacac gacggtggcg cccctgttct ttgcggacca gttccttcag 660

ctgtccacct cgctgccctc gcagtatatc acaggcctcg ccgagcacct cagtcccctg 720

atgctcagca ccagctggac caggatcacc ctgtggaacc gggaccttgc gcccacgccc 780

ggtgcgaacc tctacgggtc tcaccctttc tacctggcgc tggaggacgg cgggtcggca 840

cacggggtgt tcctgctaaa cagcaatgcc atggatgtgg tcctgcagcc gagccctgcc 900

cttagctgga ggtcgacagg tgggatcctg gatgtctaca tcttcctggg cccagagccc 960

aagagcgtgg tgcagcagta cctggacgtt gtgggatacc cgttcatgcc gccatactgg 1020

ggcctgggct tccacctgtg ccgctggggc tactcctcca ccgctatcac ccgccaggtg 1080

gtggagaaca tgaccagggc ccacttcccc ctggacgtcc agtggaacga cctggactac 1140

atggactccc ggagggactt cacgttcaac aaggatggct tccgggactt cccggccatg 1200

gtgcaggagc tgcaccaggg cggccggcgc tacatgatga tcgtggatcc tgccatcagc 1260

agctcgggcc ctgccgggag ctacaggccc tacgacgagg gtctgcggag gggggttttc 1320

atcaccaacg agaccggcca gccgctgatt gggaaggtat ggcccgggtc cactgccttc 1380

cccgacttca ccaaccccac agccctggcc tggtgggagg acatggtggc tgagttccat 1440

gaccaggtgc ccttcgacgg catgtggatt gacatgaacg agccttccaa cttcatcagg 1500

ggctctgagg acggctgccc caacaatgag ctggagaacc caccctacgt gcctggggtg 1560

gttgggggga ccctccaggc ggccaccatc tgtgcctcca gccaccagtt tctctccaca 1620

cactacaacc tgcacaacct ctacggcctg accgaagcca tcgcctccca cagggcgctg 1680

gtgaaggctc gggggacacg cccatttgtg atctcccgct cgacctttgc tggccacggc 1740

cgatacgccg gccactggac gggggacgtg tggagctcct gggagcagct cgcctcctcc 1800

gtgccagaaa tcctgcagtt taacctgctg ggggtgcctc tggtcggggc cgacgtctgc 1860

ggcttcctgg gcaacacctc agaggagctg tgtgtgcgct ggacccagct gggggccttc 1920

taccccttca tgcggaacca caacagcctg ctcagtctgc cccaggagcc gtacagcttc 1980

agcgagccgg cccagcaggc catgaggaag gccctcaccc tgcgctacgc actcctcccc 2040

cacctctaca cactgttcca ccaggcccac gtcgcggggg agaccgtggc ccggcccctc 2100

ttcctggagt tccccaagga ctctagcacc tggactgtgg accaccagct cctgtggggg 2160

gaggccctgc tcatcacccc agtgctccag gccgggaagg ccgaagtgac tggctacttc 2220

cccttgggca catggtacga cctgcagacg gtgccagtag aggcccttgg cagcctccca 2280

cccccacctg cagctccccg tgagccagcc atccacagcg aggggcagtg ggtgacgctg 2340

ccggcccccc tggacaccat caacgtccac ctccgggctg ggtacatcat ccccctgcag 2400

ggccctggcc tcacaaccac agagtcccgc cagcagccca tggccctggc tgtggccctg 2460

accaagggtg gggaggcccg aggggagctg ttctgggacg atggagagag cctggaagtg 2520

ctggagcgag gggcctacac acaggtcatc ttcctggcca ggaataacac gatcgtgaat 2580

gagctggtac gtgtgaccag tgagggagct ggcctgcagc tgcagaaggt gactgtcctg 2640

ggcgtggcca cggcgcccca gcaggtcctc tccaacggtg tccctgtctc caacttcacc 2700

tacagccccg acaccaaggt cctggacatc tgtgtctcgc tgttgatggg agagcagttt 2760

ctcgtcagct ggtgttag 2778

<210> 32
<211> 2754
<212> DNA
<213> artificial

<220>
<223> hGAAco1-delta-8 w/o sp

<400> 32
ctactagtgc ccagagagct gagcggcagc tctcccgtgc tggaagaaac acaccctgcc 60

catcagcagg gcgcctctag acctggacct agagatgccc aggcccaccc cggcagacct 120

agagctgtgc ctacccagtg tgacgtgccc cccaacagca gattcgactg cgcccctgac 180

aaggccatca cccaggaaca gtgcgaggcc agaggctgct gctacatccc tgccaagcag 240

ggactgcagg gcgctcagat gggacagccc tggtgcttct tcccaccctc ctaccccagc 300

tacaagctgg aaaacctgag cagcagcgag atgggctaca ccgccaccct gaccagaacc 360

acccccacat tcttcccaaa ggacatcctg accctgcggc tggacgtgat gatggaaacc 420

gagaaccggc tgcacttcac catcaaggac cccgccaatc ggagatacga ggtgcccctg 480

gaaacccccc acgtgcactc tagagccccc agccctctgt acagcgtgga attcagcgag 540

gaacccttcg gcgtgatcgt gcggagacag ctggatggca gagtgctgct gaacaccacc 600

gtggcccctc tgttcttcgc cgaccagttc ctgcagctga gcaccagcct gcccagccag 660

tacatcacag gactggccga gcacctgagc cccctgatgc tgagcacatc ctggacccgg 720

atcaccctgt ggaacaggga tctggcccct acccctggcg ccaatctgta cggcagccac 780

cctttctacc tggccctgga agatggcgga tctgcccacg gagtgtttct gctgaactcc 840

aacgccatgg acgtggtgct gcagcctagc cctgccctgt cttggagaag cacaggcggc 900

atcctggatg tgtacatctt tctgggcccc gagcccaaga gcgtggtgca gcagtatctg 960

gatgtcgtgg gctacccctt catgccccct tactggggcc tgggattcca cctgtgcaga 1020

tggggctact ccagcaccgc catcaccaga caggtggtgg aaaacatgac cagagcccac 1080

ttcccactgg atgtgcagtg gaacgacctg gactacatgg acagcagacg ggacttcacc 1140

ttcaacaagg acggcttccg ggacttcccc gccatggtgc aggaactgca tcagggcggc 1200

agacggtaca tgatgatcgt ggatcccgcc atcagctcct ctggccctgc cggctcttac 1260

agaccctacg acgagggcct gcggagaggc gtgttcatca ccaacgagac aggccagccc 1320

ctgatcggca aagtgtggcc tggcagcaca gccttccccg acttcaccaa tcctaccgcc 1380

ctggcttggt gggaggacat ggtggccgag ttccacgacc aggtgccctt cgacggcatg 1440

tggatcgaca tgaacgagcc cagcaacttc atccggggca gcgaggatgg ctgccccaac 1500

aacgaactgg aaaatccccc ttacgtgccc ggcgtcgtgg gcggaacact gcaggccgct 1560

acaatctgtg ccagcagcca ccagtttctg agcacccact acaacctgca caacctgtac 1620

ggcctgaccg aggccattgc cagccaccgc gctctcgtga aagccagagg cacacggccc 1680

ttcgtgatca gcagaagcac ctttgccggc cacggcagat acgccggaca ttggactggc 1740

gacgtgtggt cctcttggga gcagctggcc tctagcgtgc ccgagatcct gcagttcaat 1800

ctgctgggcg tgccactcgt gggcgccgat gtgtgtggct tcctgggcaa cacctccgag 1860

gaactgtgtg tgcggtggac acagctgggc gccttctacc ctttcatgag aaaccacaac 1920

agcctgctga gcctgcccca ggaaccctac agctttagcg agcctgcaca gcaggccatg 1980

cggaaggccc tgacactgag atacgctctg ctgccccacc tgtacaccct gtttcaccag 2040

gcccatgtgg ccggcgagac agtggccaga cctctgtttc tggaattccc caaggacagc 2100

agcacctgga ccgtggacca tcagctgctg tggggagagg ctctgctgat taccccagtg 2160

ctgcaggcag gcaaggccga agtgaccggc tactttcccc tgggcacttg gtacgacctg 2220

cagaccgtgc ctgtggaagc cctgggatct ctgcctccac ctcctgccgc tcctagagag 2280

cctgccattc actctgaggg ccagtgggtc acactgcctg cccccctgga taccatcaac 2340

gtgcacctga gggccggcta catcatacca ctgcagggac ctggcctgac caccaccgag 2400

tctagacagc agccaatggc cctggccgtg gccctgacca aaggcggaga agctaggggc 2460

gagctgttct gggacgatgg cgagagcctg gaagtgctgg aaagaggcgc ctatacccaa 2520

gtgatcttcc tggcccggaa caacaccatc gtgaacgagc tggtgcgcgt gacctctgaa 2580

ggcgctggac tgcagctgca gaaagtgacc gtgctgggag tggccacagc ccctcagcag 2640

gtgctgtcta atggcgtgcc cgtgtccaac ttcacctaca gccccgacac caaggtgctg 2700

gacatctgcg tgtcactgct gatgggagag cagtttctgg tgtcctggtg ctga 2754

<210> 33
<211> 2652
<212> DNA
<213> artificial

<220>
<223> hGAAco1-delta-42 w/o sp

<400> 33
gcccaccccg gcagacctag agctgtgcct acccagtgtg acgtgccccc caacagcaga 60

ttcgactgcg cccctgacaa ggccatcacc caggaacagt gcgaggccag aggctgctgc 120

tacatccctg ccaagcaggg actgcagggc gctcagatgg gacagccctg gtgcttcttc 180

ccaccctcct accccagcta caagctggaa aacctgagca gcagcgagat gggctacacc 240

gccaccctga ccagaaccac ccccacattc ttcccaaagg acatcctgac cctgcggctg 300

gacgtgatga tggaaaccga gaaccggctg cacttcacca tcaaggaccc cgccaatcgg 360

agatacgagg tgcccctgga aaccccccac gtgcactcta gagcccccag ccctctgtac 420

agcgtggaat tcagcgagga acccttcggc gtgatcgtgc ggagacagct ggatggcaga 480

gtgctgctga acaccaccgt ggcccctctg ttcttcgccg accagttcct gcagctgagc 540

accagcctgc ccagccagta catcacagga ctggccgagc acctgagccc cctgatgctg 600

agcacatcct ggacccggat caccctgtgg aacagggatc tggcccctac ccctggcgcc 660

aatctgtacg gcagccaccc tttctacctg gccctggaag atggcggatc tgcccacgga 720

gtgtttctgc tgaactccaa cgccatggac gtggtgctgc agcctagccc tgccctgtct 780

tggagaagca caggcggcat cctggatgtg tacatctttc tgggccccga gcccaagagc 840

gtggtgcagc agtatctgga tgtcgtgggc taccccttca tgccccctta ctggggcctg 900

ggattccacc tgtgcagatg gggctactcc agcaccgcca tcaccagaca ggtggtggaa 960

aacatgacca gagcccactt cccactggat gtgcagtgga acgacctgga ctacatggac 1020

agcagacggg acttcacctt caacaaggac ggcttccggg acttccccgc catggtgcag 1080

gaactgcatc agggcggcag acggtacatg atgatcgtgg atcccgccat cagctcctct 1140

ggccctgccg gctcttacag accctacgac gagggcctgc ggagaggcgt gttcatcacc 1200

aacgagacag gccagcccct gatcggcaaa gtgtggcctg gcagcacagc cttccccgac 1260

ttcaccaatc ctaccgccct ggcttggtgg gaggacatgg tggccgagtt ccacgaccag 1320

gtgcccttcg acggcatgtg gatcgacatg aacgagccca gcaacttcat ccggggcagc 1380

gaggatggct gccccaacaa cgaactggaa aatccccctt acgtgcccgg cgtcgtgggc 1440

ggaacactgc aggccgctac aatctgtgcc agcagccacc agtttctgag cacccactac 1500

aacctgcaca acctgtacgg cctgaccgag gccattgcca gccaccgcgc tctcgtgaaa 1560

gccagaggca cacggccctt cgtgatcagc agaagcacct ttgccggcca cggcagatac 1620

gccggacatt ggactggcga cgtgtggtcc tcttgggagc agctggcctc tagcgtgccc 1680

gagatcctgc agttcaatct gctgggcgtg ccactcgtgg gcgccgatgt gtgtggcttc 1740

ctgggcaaca cctccgagga actgtgtgtg cggtggacac agctgggcgc cttctaccct 1800

ttcatgagaa accacaacag cctgctgagc ctgccccagg aaccctacag ctttagcgag 1860

cctgcacagc aggccatgcg gaaggccctg acactgagat acgctctgct gccccacctg 1920

tacaccctgt ttcaccaggc ccatgtggcc ggcgagacag tggccagacc tctgtttctg 1980

gaattcccca aggacagcag cacctggacc gtggaccatc agctgctgtg gggagaggct 2040

ctgctgatta ccccagtgct gcaggcaggc aaggccgaag tgaccggcta ctttcccctg 2100

ggcacttggt acgacctgca gaccgtgcct gtggaagccc tgggatctct gcctccacct 2160

cctgccgctc ctagagagcc tgccattcac tctgagggcc agtgggtcac actgcctgcc 2220

cccctggata ccatcaacgt gcacctgagg gccggctaca tcataccact gcagggacct 2280

ggcctgacca ccaccgagtc tagacagcag ccaatggccc tggccgtggc cctgaccaaa 2340

ggcggagaag ctaggggcga gctgttctgg gacgatggcg agagcctgga agtgctggaa 2400

agaggcgcct atacccaagt gatcttcctg gcccggaaca acaccatcgt gaacgagctg 2460

gtgcgcgtga cctctgaagg cgctggactg cagctgcaga aagtgaccgt gctgggagtg 2520

gccacagccc ctcagcaggt gctgtctaat ggcgtgcccg tgtccaactt cacctacagc 2580

cccgacacca aggtgctgga catctgcgtg tcactgctga tgggagagca gtttctggtg 2640

tcctggtgct ga 2652

<210> 34
<211> 2754
<212> DNA
<213> artificial

<220>
<223> hGAAco2-delta-8

<400> 34
ctgttggtgc ctagagagct gagcggatca tccccagtgc tggaggagac tcatcctgct 60

caccaacagg gagcttccag accaggaccg agagacgccc aagcccatcc tggtagacca 120

agagctgtgc ctacccaatg cgacgtgcca cccaactccc gattcgactg cgcgccagat 180

aaggctatta cccaagagca gtgtgaagcc agaggttgct gctacatccc agcgaagcaa 240

ggattgcaag gcgcccaaat gggacaacct tggtgtttct tccccccttc gtacccatca 300

tataaactcg aaaacctgtc ctcttcggaa atgggttata ctgccaccct caccagaact 360

actcctactt tcttcccgaa agacatcttg accttgaggc tggacgtgat gatggagact 420

gaaaaccggc tgcatttcac tatcaaagat cctgccaatc ggcgatacga ggtccctctg 480

gaaacccctc acgtgcactc acgggctcct tctccgcttt actccgtcga attctctgag 540

gaacccttcg gagtgatcgt tagacgccag ctggatggta gagtgctgtt gaacactact 600

gtggccccac ttttcttcgc tgaccagttt ctgcaactgt ccacttccct gccatcccag 660

tacattactg gactcgccga acacctgtcg ccactgatgc tctcgacctc ttggactaga 720

atcactttgt ggaacagaga cttggcccct actccgggag caaatctgta cggaagccac 780

cctttttacc tggcgctcga agatggcgga tccgctcacg gagtgttcct gctgaatagc 840

aacgcaatgg acgtggtgct gcaaccttcc cctgcactca gttggagaag taccgggggt 900

attctggacg tgtacatctt cctcggacca gaacccaaga gcgtggtgca gcaatatctg 960

gacgtggtcg gatacccttt tatgcctcct tactggggac tgggattcca cctttgccgt 1020

tggggctact catccaccgc cattaccaga caggtggtgg agaatatgac cagagcccac 1080

ttccctctcg acgtgcagtg gaacgatctg gactatatgg actcccggag agatttcacc 1140

ttcaacaagg acgggttccg cgattttccc gcgatggttc aagagctcca ccagggtggt 1200

cgaagatata tgatgatcgt cgacccagcc atttcgagca gcggacccgc tggatcttat 1260

agaccttacg acgaaggcct taggagagga gtgttcatca caaacgagac tggacagcct 1320

ttgatcggta aagtgtggcc tggatcaacc gcctttcctg actttaccaa tcccactgcc 1380

ttggcttggt gggaggacat ggtggccgaa ttccacgacc aagtcccctt tgatggaatg 1440

tggatcgata tgaacgaacc aagcaatttt atcagaggtt ccgaagacgg ttgccccaac 1500

aacgaactgg aaaaccctcc ttatgtgccc ggagtcgtgg gcggaacatt acaggccgcg 1560

actatttgcg ccagcagcca ccaattcctg tccactcact acaacctcca caacctttat 1620

ggattaaccg aagctattgc aagtcacagg gctctggtga aggctagagg gactaggccc 1680

tttgtgatct cccgatccac ctttgccgga cacgggagat acgccggtca ctggactggt 1740

gacgtgtgga gctcatggga acaactggcc tcctccgtgc cggaaatctt acagttcaac 1800

cttctgggtg tccctcttgt cggagcagac gtgtgtgggt ttcttggtaa cacctccgag 1860

gaactgtgtg tgcgctggac tcaactgggt gcattctacc cattcatgag aaaccacaac 1920

tccttgctgt ccctgccaca agagccctac tcgttcagcg agcctgcaca acaggctatg 1980

cggaaggcac tgaccctgag atacgccctg cttccacact tatacactct cttccatcaa 2040

gcgcatgtgg caggagaaac cgttgcaagg cctcttttcc ttgaattccc caaggattcc 2100

tcgacttgga cggtggatca tcagctgctg tggggagaag ctctgctgat tactccagtg 2160

ttgcaagccg gaaaagctga ggtgaccgga tactttccgc tgggaacctg gtacgacctc 2220

cagactgtcc ctgttgaagc ccttggatca ctgcctccgc ctccggcagc tccacgcgaa 2280

ccagctatac attccgaggg acagtgggtt acattaccag ctcctctgga cacaatcaac 2340

gtccacttaa gagctggcta cattatccct ctgcaaggac caggactgac tacgaccgag 2400

agcagacagc agccaatggc actggctgtg gctctgacca agggagggga agctagagga 2460

gaactcttct gggatgatgg ggagtccctt gaagtgctgg aaagaggcgc ttacactcaa 2520

gtcattttcc ttgcacggaa caacaccatt gtgaacgaat tggtgcgagt gaccagcgaa 2580

ggagctggac ttcaactgca gaaggtcact gtgctcggag tggctaccgc tcctcagcaa 2640

gtgctgtcga atggagtccc cgtgtcaaac tttacctact cccctgacac taaggtgctc 2700

gacatttgcg tgtccctcct gatgggagag cagttccttg tgtcctggtg ttga 2754

<210> 35
<211> 2652
<212> DNA
<213> artificial

<220>
<223> hGAAco2-delta-42

<400> 35
   gcccatcctg gtagaccaag agctgtgcct acccaatgcg acgtgccacc caactcccga 60
   ttcgactgcg cgccagataa ggctattacc caagagcagt gtgaagccag aggttgctgc 120
   tacatcccag cgaagcaagg attgcaaggc gcccaaatgg gacaaccttg gtgtttcttc 180
   cccccttcgt acccatcata taaactcgaa aacctgtcct cttcggaaat gggttatact 240
   gccaccctca ccagaactac tcctactttc ttcccgaaag acatcttgac cttgaggctg 300
   gacgtgatga tggagactga aaaccggctg catttcacta tcaaagatcc tgccaatcgg 360
   cgatacgagg tccctctgga aacccctcac gtgcactcac gggctccttc tccgctttac 420
   tccgtcgaat tctctgagga acccttcgga gtgatcgtta gacgccagct ggatggtaga 480
   gtgctgttga acactactgt ggccccactt ttcttcgctg accagtttct gcaactgtcc 540
   acttccctgc catcccagta cattactgga ctcgccgaac acctgtcgcc actgatgctc 600
   tcgacctctt ggactagaat cactttgtgg aacagagact tggcccctac tccgggagca 660
   aatctgtacg gaagccaccc tttttacctg gcgctcgaag atggcggatc cgctcacgga 720
   gtgttcctgc tgaatagcaa cgcaatggac gtggtgctgc aaccttcccc tgcactcagt 780
   tggagaagta ccgggggtat tctggacgtg tacatcttcc tcggaccaga acccaagagc 840
   gtggtgcagc aatatctgga cgtggtcgga taccctttta tgcctcctta ctggggactg 900
   ggattccacc tttgccgttg gggctactca tccaccgcca ttaccagaca ggtggtggag 960
   aatatgacca gagcccactt ccctctcgac gtgcagtgga acgatctgga ctatatggac 1020
   tcccggagag atttcacctt caacaaggac gggttccgcg attttcccgc gatggttcaa 1080
   gagctccacc agggtggtcg aagatatatg atgatcgtcg acccagccat ttcgagcagc 1140
   ggacccgctg gatcttatag accttacgac gaaggcctta ggagaggagt gttcatcaca 1200
   aacgagactg gacagccttt gatcggtaaa gtgtggcctg gatcaaccgc ctttcctgac 1260
   tttaccaatc ccactgcctt ggcttggtgg gaggacatgg tggccgaatt ccacgaccaa 1320
   gtcccctttg atggaatgtg gatcgatatg aacgaaccaa gcaattttat cagaggttcc 1380
   gaagacggtt gccccaacaa cgaactggaa aaccctcctt atgtgcccgg agtcgtgggc 1440
   ggaacattac aggccgcgac tatttgcgcc agcagccacc aattcctgtc cactcactac 1500
   aacctccaca acctttatgg attaaccgaa gctattgcaa gtcacagggc tctggtgaag 1560
   gctagaggga ctaggccctt tgtgatctcc cgatccacct ttgccggaca cgggagatac 1620
   gccggtcact ggactggtga cgtgtggagc tcatgggaac aactggcctc ctccgtgccg 1680
   gaaatcttac agttcaacct tctgggtgtc cctcttgtcg gagcagacgt gtgtgggttt 1740
   cttggtaaca cctccgagga actgtgtgtg cgctggactc aactgggtgc attctaccca 1800
   ttcatgagaa accacaactc cttgctgtcc ctgccacaag agccctactc gttcagcgag 1860
   cctgcacaac aggctatgcg gaaggcactg accctgagat acgccctgct tccacactta 1920
   tacactctct tccatcaagc gcatgtggca ggagaaaccg ttgcaaggcc tcttttcctt 1980
   gaattcccca aggattcctc gacttggacg gtggatcatc agctgctgtg gggagaagct 2040
   ctgctgatta ctccagtgtt gcaagccgga aaagctgagg tgaccggata ctttccgctg 2100
   ggaacctggt acgacctcca gactgtccct gttgaagccc ttggatcact gcctccgcct 2160
   ccggcagctc cacgcgaacc agctatacat tccgagggac agtgggttac attaccagct 2220
   cctctggaca caatcaacgt ccacttaaga gctggctaca ttatccctct gcaaggacca 2280
   ggactgacta cgaccgagag cagacagcag ccaatggcac tggctgtggc tctgaccaag 2340
   ggaggggaag ctagaggaga actcttctgg gatgatgggg agtcccttga agtgctggaa 2400
   agaggcgctt acactcaagt cattttcctt gcacggaaca acaccattgt gaacgaattg 2460
   gtgcgagtga ccagcgaagg agctggactt caactgcaga aggtcactgt gctcggagtg 2520
   gctaccgctc ctcagcaagt gctgtcgaat ggagtccccg tgtcaaactt tacctactcc 2580
   cctgacacta aggtgctcga catttgcgtg tccctcctga tgggagagca gttccttgtg 2640
   tcctggtgtt ga 2652

<210> 36
<211> 925
<212> PRT
<213> artificial

<220>
<223> variant hGAAwt w/o sp

<400> 36

Gly His Ile Leu Leu His Asp Phe Leu Leu Val Pro Arg Glu Leu Ser
1               5                   10                  15

Gly Ser Ser Pro Val Leu Glu Glu Thr His Pro Ala His Gln Gln Gly
            20                  25                  30

Ala Ser Arg Pro Gly Pro Arg Asp Ala Gln Ala His Pro Gly Arg Pro
        35                  40                  45

Arg Ala Val Pro Thr Gln Cys Asp Val Pro Pro Asn Ser Arg Phe Asp
    50                  55                  60

Cys Ala Pro Asp Lys Ala Ile Thr Gln Glu Gln Cys Glu Ala Arg Gly
65                  70                  75                  80

Cys Cys Tyr Ile Pro Ala Lys Gln Gly Leu Gln Gly Ala Gln Met Gly
                85                  90                  95

Gln Pro Trp Cys Phe Phe Pro Pro Ser Tyr Pro Ser Tyr Lys Leu Glu
            100                 105                 110

Asn Leu Ser Ser Ser Glu Met Gly Tyr Thr Ala Thr Leu Thr Arg Thr
        115                 120                 125

Thr Pro Thr Phe Phe Pro Lys Asp Ile Leu Thr Leu Arg Leu Asp Val
    130                 135                 140

Met Met Glu Thr Glu Asn Arg Leu His Phe Thr Ile Lys Asp Pro Ala
145                 150                 155                 160

Asn Arg Arg Tyr Glu Val Pro Leu Glu Thr Pro Arg Val His Ser Arg
                165                 170                 175

Ala Pro Ser Pro Leu Tyr Ser Val Glu Phe Ser Glu Glu Pro Phe Gly
            180                 185                 190

Val Ile Val His Arg Gln Leu Asp Gly Arg Val Leu Leu Asn Thr Thr
        195                 200                 205

Val Ala Pro Leu Phe Phe Ala Asp Gln Phe Leu Gln Leu Ser Thr Ser
    210                 215                 220

Leu Pro Ser Gln Tyr Ile Thr Gly Leu Ala Glu His Leu Ser Pro Leu
225                 230                 235                 240

Met Leu Ser Thr Ser Trp Thr Arg Ile Thr Leu Trp Asn Arg Asp Leu
                245                 250                 255

Ala Pro Thr Pro Gly Ala Asn Leu Tyr Gly Ser His Pro Phe Tyr Leu
            260                 265                 270

Ala Leu Glu Asp Gly Gly Ser Ala His Gly Val Phe Leu Leu Asn Ser
        275                 280                 285

Asn Ala Met Asp Val Val Leu Gln Pro Ser Pro Ala Leu Ser Trp Arg
    290                 295                 300

Ser Thr Gly Gly Ile Leu Asp Val Tyr Ile Phe Leu Gly Pro Glu Pro
305                 310                 315                 320

Lys Ser Val Val Gln Gln Tyr Leu Asp Val Val Gly Tyr Pro Phe Met
                325                 330                 335

Pro Pro Tyr Trp Gly Leu Gly Phe His Leu Cys Arg Trp Gly Tyr Ser
            340                 345                 350

Ser Thr Ala Ile Thr Arg Gln Val Val Glu Asn Met Thr Arg Ala His
        355                 360                 365

Phe Pro Leu Asp Val Gln Trp Asn Asp Leu Asp Tyr Met Asp Ser Arg
    370                 375                 380

Arg Asp Phe Thr Phe Asn Lys Asp Gly Phe Arg Asp Phe Pro Ala Met
385                 390                 395                 400

Val Gln Glu Leu His Gln Gly Gly Arg Arg Tyr Met Met Ile Val Asp
                405                 410                 415

Pro Ala Ile Ser Ser Ser Gly Pro Ala Gly Ser Tyr Arg Pro Tyr Asp
            420                 425                 430

Glu Gly Leu Arg Arg Gly Val Phe Ile Thr Asn Glu Thr Gly Gln Pro
        435                 440                 445

Leu Ile Gly Lys Val Trp Pro Gly Ser Thr Ala Phe Pro Asp Phe Thr
    450                 455                 460

Asn Pro Thr Ala Leu Ala Trp Trp Glu Asp Met Val Ala Glu Phe His
465                 470                 475                 480

Asp Gln Val Pro Phe Asp Gly Met Trp Ile Asp Met Asn Glu Pro Ser
                485                 490                 495

Asn Phe Ile Arg Gly Ser Glu Asp Gly Cys Pro Asn Asn Glu Leu Glu
            500                 505                 510

Asn Pro Pro Tyr Val Pro Gly Val Val Gly Gly Thr Leu Gln Ala Ala
        515                 520                 525

Thr Ile Cys Ala Ser Ser His Gln Phe Leu Ser Thr His Tyr Asn Leu
    530                 535                 540

His Asn Leu Tyr Gly Leu Thr Glu Ala Ile Ala Ser His Arg Ala Leu
545                 550                 555                 560

Val Lys Ala Arg Gly Thr Arg Pro Phe Val Ile Ser Arg Ser Thr Phe
                565                 570                 575

Ala Gly His Gly Arg Tyr Ala Gly His Trp Thr Gly Asp Val Trp Ser
            580                 585                 590

Ser Trp Glu Gln Leu Ala Ser Ser Val Pro Glu Ile Leu Gln Phe Asn
        595                 600                 605

Leu Leu Gly Val Pro Leu Val Gly Ala Asp Val Cys Gly Phe Leu Gly
    610                 615                 620

Asn Thr Ser Glu Glu Leu Cys Val Arg Trp Thr Gln Leu Gly Ala Phe
625                 630                 635                 640

Tyr Pro Phe Met Arg Asn His Asn Ser Leu Leu Ser Leu Pro Gln Glu
                645                 650                 655

Pro Tyr Ser Phe Ser Glu Pro Ala Gln Gln Ala Met Arg Lys Ala Leu
            660                 665                 670

Thr Leu Arg Tyr Ala Leu Leu Pro His Leu Tyr Thr Leu Phe His Gln
        675                 680                 685

Ala His Val Ala Gly Glu Thr Val Ala Arg Pro Leu Phe Leu Glu Phe
    690                 695                 700

Pro Lys Asp Ser Ser Thr Trp Thr Val Asp His Gln Leu Leu Trp Gly
705                 710                 715                 720

Glu Ala Leu Leu Ile Thr Pro Val Leu Gln Ala Gly Lys Ala Glu Val
                725                 730                 735

Thr Gly Tyr Phe Pro Leu Gly Thr Trp Tyr Asp Leu Gln Thr Val Pro
            740                 745                 750

Ile Glu Ala Leu Gly Ser Leu Pro Pro Pro Pro Ala Ala Pro Arg Glu
        755                 760                 765

Pro Ala Ile His Ser Glu Gly Gln Trp Val Thr Leu Pro Ala Pro Leu
    770                 775                 780

Asp Thr Ile Asn Val His Leu Arg Ala Gly Tyr Ile Ile Pro Leu Gln
785                 790                 795                 800

Gly Pro Gly Leu Thr Thr Thr Glu Ser Arg Gln Gln Pro Met Ala Leu
                805                 810                 815

Ala Val Ala Leu Thr Lys Gly Gly Glu Ala Arg Gly Glu Leu Phe Trp

820                 825                 830

Asp Asp Gly Glu Ser Leu Glu Val Leu Glu Arg Gly Ala Tyr Thr Gln
        835                 840                 845

Val Ile Phe Leu Ala Arg Asn Asn Thr Ile Val Asn Glu Leu Val Arg
    850                 855                 860

Val Thr Ser Glu Gly Ala Gly Leu Gln Leu Gln Lys Val Thr Val Leu
865                 870                 875                 880

Gly Val Ala Thr Ala Pro Gln Gln Val Leu Ser Asn Gly Val Pro Val
                885                 890                 895

Ser Asn Phe Thr Tyr Ser Pro Asp Thr Lys Val Leu Asp Ile Cys Val
            900                 905                 910

Ser Leu Leu Met Gly Glu Gln Phe Leu Val Ser Trp Cys
        915                 920                 925

<210>  37
<211>  952
<212>  PRT
<213>  homo sapiens

<400>  37

Met Gly Val Arg His Pro Pro Cys Ser His Arg Leu Leu Ala Val Cys
1               5                   10                  15

Ala Leu Val Ser Leu Ala Thr Ala Ala Leu Leu Gly His Ile Leu Leu
            20                  25                  30

His Asp Phe Leu Leu Val Pro Arg Glu Leu Ser Gly Ser Ser Pro Val
        35                  40                  45

Leu Glu Glu Thr His Pro Ala His Gln Gln Gly Ala Ser Arg Pro Gly
    50                  55                  60

Pro Arg Asp Ala Gln Ala His Pro Gly Arg Pro Arg Ala Val Pro Thr
65                  70                  75                  80

Gln Cys Asp Val Pro Pro Asn Ser Arg Phe Asp Cys Ala Pro Asp Lys
                85                  90                  95

Ala Ile Thr Gln Glu Gln Cys Glu Ala Arg Gly Cys Cys Tyr Ile Pro
            100                 105                 110

Ala Lys Gln Gly Leu Gln Gly Ala Gln Met Gly Gln Pro Trp Cys Phe

115                 120                 125

Phe Pro Pro Ser Tyr Pro Ser Tyr Lys Leu Glu Asn Leu Ser Ser Ser
    130                 135                 140

Glu Met Gly Tyr Thr Ala Thr Leu Thr Arg Thr Thr Pro Thr Phe Phe
145                 150                 155                 160

Pro Lys Asp Ile Leu Thr Leu Arg Leu Asp Val Met Met Glu Thr Glu
                165                 170                 175

Asn Arg Leu His Phe Thr Ile Lys Asp Pro Ala Asn Arg Arg Tyr Glu
            180                 185                 190

Val Pro Leu Glu Thr Pro Arg Val His Ser Arg Ala Pro Ser Pro Leu
        195                 200                 205

Tyr Ser Val Glu Phe Ser Glu Glu Pro Phe Gly Val Ile Val His Arg
    210                 215                 220

Gln Leu Asp Gly Arg Val Leu Leu Asn Thr Thr Val Ala Pro Leu Phe
225                 230                 235                 240

Phe Ala Asp Gln Phe Leu Gln Leu Ser Thr Ser Leu Pro Ser Gln Tyr
                245                 250                 255

Ile Thr Gly Leu Ala Glu His Leu Ser Pro Leu Met Leu Ser Thr Ser
            260                 265                 270

Trp Thr Arg Ile Thr Leu Trp Asn Arg Asp Leu Ala Pro Thr Pro Gly
        275                 280                 285

Ala Asn Leu Tyr Gly Ser His Pro Phe Tyr Leu Ala Leu Glu Asp Gly
    290                 295                 300

Gly Ser Ala His Gly Val Phe Leu Leu Asn Ser Asn Ala Met Asp Val
305                 310                 315                 320

Val Leu Gln Pro Ser Pro Ala Leu Ser Trp Arg Ser Thr Gly Gly Ile
                325                 330                 335

Leu Asp Val Tyr Ile Phe Leu Gly Pro Glu Pro Lys Ser Val Val Gln
            340                 345                 350

Gln Tyr Leu Asp Val Val Gly Tyr Pro Phe Met Pro Pro Tyr Trp Gly
        355                 360                 365

Leu Gly Phe His Leu Cys Arg Trp Gly Tyr Ser Ser Thr Ala Ile Thr
    370                 375                 380

Arg Gln Val Val Glu Asn Met Thr Arg Ala His Phe Pro Leu Asp Val
385                 390                 395                 400

Gln Trp Asn Asp Leu Asp Tyr Met Asp Ser Arg Arg Asp Phe Thr Phe
                405                 410                 415

Asn Lys Asp Gly Phe Arg Asp Phe Pro Ala Met Val Gln Glu Leu His
            420                 425                 430

Gln Gly Gly Arg Arg Tyr Met Met Ile Val Asp Pro Ala Ile Ser Ser
        435                 440                 445

Ser Gly Pro Ala Gly Ser Tyr Arg Pro Tyr Asp Glu Gly Leu Arg Arg
    450                 455                 460

Gly Val Phe Ile Thr Asn Glu Thr Gly Gln Pro Leu Ile Gly Lys Val
465                 470                 475                 480

Trp Pro Gly Ser Thr Ala Phe Pro Asp Phe Thr Asn Pro Thr Ala Leu
                485                 490                 495

Ala Trp Trp Glu Asp Met Val Ala Glu Phe His Asp Gln Val Pro Phe
            500                 505                 510

Asp Gly Met Trp Ile Asp Met Asn Glu Pro Ser Asn Phe Ile Arg Gly
        515                 520                 525

Ser Glu Asp Gly Cys Pro Asn Asn Glu Leu Glu Asn Pro Pro Tyr Val
    530                 535                 540

Pro Gly Val Val Gly Gly Thr Leu Gln Ala Ala Thr Ile Cys Ala Ser
545                 550                 555                 560

Ser His Gln Phe Leu Ser Thr His Tyr Asn Leu His Asn Leu Tyr Gly
                565                 570                 575

Leu Thr Glu Ala Ile Ala Ser His Arg Ala Leu Val Lys Ala Arg Gly
            580                 585                 590

Thr Arg Pro Phe Val Ile Ser Arg Ser Thr Phe Ala Gly His Gly Arg
        595                 600                 605

Tyr Ala Gly His Trp Thr Gly Asp Val Trp Ser Ser Trp Glu Gln Leu
    610                 615                 620

Ala Ser Ser Val Pro Glu Ile Leu Gln Phe Asn Leu Leu Gly Val Pro
625                 630                 635                 640

Leu Val Gly Ala Asp Val Cys Gly Phe Leu Gly Asn Thr Ser Glu Glu
                645                 650                 655

Leu Cys Val Arg Trp Thr Gln Leu Gly Ala Phe Tyr Pro Phe Met Arg
            660                 665                 670

Asn His Asn Ser Leu Leu Ser Leu Pro Gln Glu Pro Tyr Ser Phe Ser
        675                 680                 685

Glu Pro Ala Gln Gln Ala Met Arg Lys Ala Leu Thr Leu Arg Tyr Ala
    690                 695                 700

Leu Leu Pro His Leu Tyr Thr Leu Phe His Gln Ala His Val Ala Gly
705                 710                 715                 720

Glu Thr Val Ala Arg Pro Leu Phe Leu Glu Phe Pro Lys Asp Ser Ser
                725                 730                 735

Thr Trp Thr Val Asp His Gln Leu Leu Trp Gly Glu Ala Leu Leu Ile
            740                 745                 750

Thr Pro Val Leu Gln Ala Gly Lys Ala Glu Val Thr Gly Tyr Phe Pro
        755                 760                 765

Leu Gly Thr Trp Tyr Asp Leu Gln Thr Val Pro Ile Glu Ala Leu Gly
    770                 775                 780

Ser Leu Pro Pro Pro Pro Ala Ala Pro Arg Glu Pro Ala Ile His Ser
785                 790                 795                 800

Glu Gly Gln Trp Val Thr Leu Pro Ala Pro Leu Asp Thr Ile Asn Val
                805                 810                 815

His Leu Arg Ala Gly Tyr Ile Ile Pro Leu Gln Gly Pro Gly Leu Thr
            820                 825                 830

Thr Thr Glu Ser Arg Gln Gln Pro Met Ala Leu Ala Val Ala Leu Thr
        835                 840                 845

Lys Gly Gly Glu Ala Arg Gly Glu Leu Phe Trp Asp Asp Gly Glu Ser
    850                 855                 860

Leu Glu Val Leu Glu Arg Gly Ala Tyr Thr Gln Val Ile Phe Leu Ala
865                 870                 875                 880

Arg Asn Asn Thr Ile Val Asn Glu Leu Val Arg Val Thr Ser Glu Gly
                885                 890                 895

Ala Gly Leu Gln Leu Gln Lys Val Thr Val Leu Gly Val Ala Thr Ala
            900                 905                 910

Pro Gln Gln Val Leu Ser Asn Gly Val Pro Val Ser Asn Phe Thr Tyr
        915                 920                 925

Ser Pro Asp Thr Lys Val Leu Asp Ile Cys Val Ser Leu Leu Met Gly
    930                 935                 940

Glu Gln Phe Leu Val Ser Trp Cys
945                 950

<210>  38
<211>  952
<212>  PRT
<213>  homo sapiens

<400>  38

<210> 39
<211> 957
<212> PRT
<213> homo sapiens

<400> 39

<210>  40
<211>  952
<212>  PRT
<213>  homo sapiens

<400>  40